# EUROPEAN PATENT SPECIFICATION

(11) **EP 4 028 046 B1**
(45) Date of publication and mention of the grant of the patent: **14.02.2024**
(21) Application number: 20775561.2
(22) Date of filing: 10.09.2020
(51) Int. Cl.: A61K 38/36, A61K 38/37, A61P 9/10

(54) **METHODS OF TREATMENT RELATED TO COMPLEXES OF VON WILLEBRAND FACTOR AND COMPLEMENT C1Q**
VERFAHREN ZUR BEHANDLUNG IM ZUSAMMENHANG MIT KOMPLEXEN DES VON-WILLEBRAND-FAKTORS UND KOMPLEMENT C1Q
MÉTHODES DE TRAITEMENT LIÉS À DES COMPLEXES DU FACTEUR DE VON WILLEBRAND ET DU COMPLÉMENT C1Q

(30) Priority: 11.09.2019 US 201962899036 P
(43) Date of publication of application: 20.07.2022
(73) Proprietor: Takeda Pharmaceutical Company Limited, Osaka-shi, Osaka 541-0045 (JP)
(72) Inventor: DONAT, Claudia, Osaka-shi, Osaka, 541-0045 (JP); THANEI, Sophia, Osaka-shi, Osaka, 541-0045 (JP); TRENDELENBURG, Marten, Osaka-shi, Osaka, 541-0045 (JP); TURECEK, Peter, Osaka-shi, Osaka, 541-0045 (JP)
(74) Representative: Hoffmann Eitle
(86) International application number: PCT/US2020/050185
(87) International publication number: WO 2021/050718

(56) References cited:
- CLAUDIA DONAT ET AL: "Binding of von Willebrand Factor to Complement C1q Decreases the Phagocytosis of Cholesterol Crystals and Subsequent IL-1 Secretion in Macrophages", FRONTIERS IN IMMUNOLOGY, vol. 10, 21 November 2019 (2019-11-21), XP055749830, CH ISSN: 1664-3224, DOI: 10.3389/fimmu.2019.02712
- DATABASE BIOSIS [Online] BIOSCIENCES INFORMATION SERVICE, PHILADELPHIA, PA, US; October 2019 (2019-10), DONAT CLAUDIA ET AL: "VON WILLEBRAND FACTOR MODULATES THE IMMUNOLOGICAL EFFECTS OF COMPLEMENT C1Q ON HUMAN MONOCYTE-DERIVED MACROPHAGES", XP002801111, Database accession no. PREV201900970096
- FELICE GRAGNANO ET AL: "The Role of von Willebrand Factor in Vascular Inflammation: From Pathogenesis to Targeted Therapy", MEDIATORS OF INFLAMMATION., vol. 2017, 1 January 2017 (2017-01-01), pages 1-13, XP055749854, GB ISSN: 0962-9351, DOI: 10.1155/2017/5620314
- PRAKASH DODDAPATTAR ET AL: "Endothelial Cell-Derived Von Willebrand Factor, But Not Platelet-Derived, Promotes Atherosclerosis in Apolipoprotein E-Deficient Mice", ARTERIOSCLEROSIS, THROMBOSIS, AND VASCULAR BIOLOGY, vol. 38, no. 3, 1 March 2018 (2018-03-01), pages 520-528, XP055749870, ISSN: 1079-5642, DOI: 10.1161/ATVBAHA.117.309918
- DONAT CLAUDIA ET AL: "Binding of von Willebrand factor to complement C1q on a cholesterol crystal surface", MOLECULAR IMMUNOLOGY, vol. 89, 2017, page 137, XP085162903, ISSN: 0161-5890, DOI: 10.1016/J.MOLIMM.2017.06.077
- MELINDA D. WU ET AL: "Platelets and von Willebrand factor in atherogenesis", BLOOD, vol. 129, no. 11, 16 March 2017 (2017-03-16), pages 1415-1419, XP055750147, US ISSN: 0006-4971, DOI: 10.1182/blood-2016-07-692673

## Description

### CROSS REFERENCES TO APPLICATIONS

This application claims priority to U.S. Provisional Patent Application Serial No. 62/899,036, filed September 11, 2019 .

### BACKGROUND OF THE INVENTION

Atherosclerosis, which is considered to be a pathological remodeling of the arteries, is a major cause of morbidity and mortality in developed countries and is the underlying basis of myocardial infarction, stroke and peripheral artery disease. It can be considered as an unusual form of chronic inflammation occurring within the artery wall. Atherosclerosis is characterized by patchy intimal plaques (atheromas) that encroach on the lumen of medium-sized and large arteries; the plaques contain lipids, inflammatory cells, smooth muscle cells, and connective tissue. It can affect large and medium-sized arteries, including the coronary, carotid, and cerebral arteries, the aorta and its branches, and major arteries of the extremities.

Symptoms develop when growth or rupture of the plaque reduces or obstructs blood flow; and the symptoms may vary depending on which artery is affected. Atherosclerotic plaques may be stable or unstable. Stable plaques regress, remain static, or grow slowly, sometimes over several decades, until they may cause stenosis or occlusion.

Unstable plaques are vulnerable to spontaneous erosion, fissure, or rupture, causing acute thrombosis, occlusion, and infarction long before they cause hemodynamically significant stenosis. Most clinical events result from unstable plaques, which do not appear severe on angiography. Rupture of an unstable plaque leads to platelet aggregation and the formation of an arterial blood clot (thrombus) attached to the vessel wall. Thrombus material breaks away from the wall and can be transported to a distant site (embolism), where it may lead to blockage of smaller arteries. The clinical consequences of arterial thrombosis are heart attacks, strokes and renal disease. Indeed, the majority (about 60%) of arterial thrombosis is associated with ruptured atherosclerotic plaques.

The earliest visible lesion of atherosclerosis is the fatty streak, which is an accumulation of lipid-laden foam cells in the intimal layer of an artery. The hallmark of atherosclerosis is atherosclerotic plaque, which is an evolution of the fatty streak and has three major components: lipids (*e.g*., cholesterol and triglycerides); inflammatory cells (*e.g.,* monocytes, macrophages, dendritic cells, and derivative thereof) and smooth muscle cells; and a connective tissue matrix.

Macrophages are intrinsically involved with the progression of atherosclerosis. Macrophages and macrophage derived foam cells are a major constituent of atherosclerotic plaques, a higher ratio of macrophages being associated with unstable plaques. Additional descriptions of the role of inflammation and macrophages in atherosclerosis can be found, for example, in Moore and Tabas, Cell, 2011, 145, 341-355 and Libby, Nature, 2002, 420(6217), 868-74.

At the onset of atherosclerosis, macrophage-derived foam cells accumulate in the subendothelial space of a blood vessels (arteries) and are key to the progression of an atherosclerotic lesion (plaque) with a fibrous cap. The foam cells increase expression of key atherogenic and inflammatory cytokines and chemokines, thereby inducing an inflammatory response. As the atherosclerotic lesion progresses into a vulnerable plaque, a necrotic core comprising apoptotic foam cells, cell debris, and cholesterol crystals forms and the fibrous cap thins and weakens. The necrotic core contributes to inflammation, thrombosis, proteolytic breakdown, and physical stress on the fibrous cap. Eventually, the accumulation of apoptotic cells and defective phagocytic clearance (efferocytosis) in the necrotic core and the instability of the thinning fibrous cap results in the rupture of the atherosclerotic plaque and the formation of a blood clot (thrombus). In some cases, the blood clot can lead to myocardial infarction (heart attack), unstable angina, sudden cardiac death, or stroke (Virmani et al., J. Interv. Cardiol, 2002, 15, 439-446).

Complement C1q, an initiation molecule of the classical complement pathway, exerts versatile immunomodulatory functions independent of complement activation. Non-classical functions of C1q include the clearance of apoptotic cells and cholesterol crystals (CC) as well as the modulation of cytokine secretion by immune cells, such as macrophages.

In advanced stages of atherosclerosis, complement activation - in particular when involving the terminal pathway - seems to lead to an inflammatory response, contributing to disease pathology. In contrast, in early atherosclerotic lesions in the absence of, or prior to, expression of other complement components, observational and experimental studies suggest that C1q and early downstream components of the classical pathway play a protective role (Patzelt et al., Front Physiol, 2015, 6, 49). Many have reviewed the early atherogenic role of platelets as well as von Willebrand factor (VWF) as mediators of platelet-endothelial interaction (Wu, et al., Blood, 129 (2017) 1415-1419).

Von Willebrand factor has been shown to bind to surface-bound C1q *in vitro* (Kölm et al., Molecular Immunology, 56 (2013) 240-316; Abstract CC 4). Recently, C1q has been shown to mediate the binding of von Willebrand factor (VWF), an initiation molecule of primary hemostasis (Kölm et al., J Immunol, 197 (2016) 3669-3679). C1q-bound von Willebrand factor has also been found to induce thrombocytes rolling *in vitro.* (Kölm, et al., Molecular Immunology 61 (2014) 210-292; Abstract 002.; and Kölm, et al., J Immunol, 197 (2016) 3669-3679). C1q was also found to bind to cholesterol crystals (CC) with subsequent binding of vWF (Donat et al., Molecular Immunology 89 (2017) 130-139 137; Abstract 045). The *in vivo* relevance of C1q-mediated binding of vWF by studying C1q-deficient mice with regard to alterations in hemostasis has also been examined (Donat et al., Frontiers in Immunology, 11 (2020) 1-10; Article 1522). However, the role of the interaction of C1q-VWF on atherosclerosis progression remains unclear.

VWF is a glycoprotein circulating in plasma as a series of multimers ranging in size from about 500 to 20,000 kD. The full length of cDNA of VWF has been cloned; the propolypeptide corresponds to amino acid residues 23 to 764 of the full length prepro-VWF (Eikenboom et al., Haemophilia, 1 (1995), 77-90). Multimeric forms of VWF are composed of 250 kD polypeptide subunits linked together by disulfide bonds. VWF mediates the initial platelet adhesion to the sub-endothelium of the damaged vessel wall, with the larger multimers exhibiting enhanced hemostatic activity. Multimerized VWF binds to the platelet surface glycoprotein Gp1bα, through an interaction in the A1 domain of VWF, facilitating platelet adhesion. Other sites on VWF mediate binding to the blood vessel wall. Thus, VWF forms a bridge between the platelet and the vessel wall that is essential to platelet adhesion and primary hemostasis under conditions of high shear stress.

Normally, endothelial cells secrete large polymeric forms of VWF and those forms of VWF that have a lower molecular weight arise from proteolytic cleavage. The multimers of exceptionally large molecular masses are stored in the Weibel-Pallade bodies of the endothelial cells and liberated upon stimulation by agonists such as thrombin and histamine.

Elevated VWF levels are strongly associated with an increased risk of ischemic cardiovascular events (Spiel et al., Circulation, 2008, 117(11), 1449-1459). It also has been shown that VWF deficiency or blockage has a protective effect against atherosclerosis (Methia, et al., Blood, 98 (2001) 1424-1428).

There remains a need in the art for novel treatments of atherosclerosis and associated conditions.

### BRIEF SUMMARY OF THE INVENTION

Provided herein is a method of treating atherosclerosis in a subject comprising administering to a subject a therapeutically effective amount of a composition comprising recombinant Von Willebrand Factor (VWF) such that VWF binds complement C1q to form C1q VWF complexes and the C1q-VWF complex binds cholesterol crystals (CC), thereby forming a CC-C1q-VWF complex and inhibiting atherosclerotic plaque progression.

Herein, the references to the methods of treatment by therapy or surgery or in vivo diagnosis methods in this description are to be interpreted as references to compounds, pharmaceutical compositions and medicaments of the present invention for use in those methods.

In one aspect, provided is the above method wherein plaque is decreased in a subject comprising administering to a subject a therapeutically effective amount of a composition comprising Von Willebrand Factor (VWF) such that vWF binds complement C1q to form C1q-VWF complexes, and the C1q-VWF complex binds cholesterol crystals (CC), thereby forming a CC-C1q-VWF complex and decreasing inflammation of atherosclerotic plaque.

In another aspect, provided herein is the above method wherein an immune response of mactrophages in a blood vessel of a subject is modulated comprising administarting to a subject a therapeutically effective amount of a composition comprising Von Willebrand Factor (VWF) such that VWF binds complement C1q to form C1q-VWF complexes, thereby reducing macrophage cell formation, increasing the expression level of one or more phagocytosis-mediating receptors and/or costimulatory receptors on the surface of the macrophages, decreasing phagocytosis by the macrophages, and/or decreasing pro-inflammatory cytokine secretion by the macrophages.

The subject of any of the methods has atherosclerosis.

The VWF is recombinant vWF (rVWF). In some embodiments, the rVWF comprises a polypeptide selected from the group consisting of: (a) the amino acid sequence of SEQ ID NO:3;
and (c) a polypeptide encoded by the polynucleotide sequence of SEQ ID NO: 1 .

In some embodiments, the composition of rVWF comprises a high molecular weight vWF multimer comprising at least 20% VWF decamers or high order multimers. In certain embodiments, the composition of rVWF comprises a high molecular weight vWF multimer comprising at least 40% VWF decamers or high order multimers. In some embodiments, the composition of rVWF comprises a high molecular weight vWF multimer comprising at least 60% VWF decamers or high order multimers.

In some embodiments, the rVWF is matured in vitro by treatment with Furin.

In some embodiments, the rVWF has a higher specific activity compared to plasma-derived VWF.

In some embodiments, the C1q-VWF complex of any one of the methods herein binds cholesterol crystals (CC), thereby forming a CC-C1q-VWF complex.

In some embodiments, one or more phagocytosis-mediating receptors and/or costimulatory receptors are selected from the group consisting of CD14, CD86, leukocyte-associated immunoglobulin-like receptor 1 (LAIR1), lipoprotein receptor-related protein 1 (LRP-1), tyrosine protein kinase Mer (MerTk), MHC II, programmed death ligand 1 (PD-L1), scavenger receptor A 1 (SR-A1), and a combination thereof.

In some embodiments, the pro-inflammatory cytokine is selected from the group consisting of IL-1α, IL-1β, IL-6, IL-10, IL-18, IL-1R, TNF1α, and a combination thereof.

In some embodiments, any one of the methods herein further comprises determining the level of C1q-VWF complex in a sample taken from the subject after administrating the compositions comprising VWF. In certain instances, the step of determining the level of C1q-VWF complex comprises detecting the C1q-VWF complex on the surface of a cell in the sample.

The present invention provides an *in vitro* method of identifying a candidate agent for modulating phagocytosis of macrophages comprising: (a) incubating rVWF, complement C1q, and cholesterol crystals to form a complex comprising rVWF, complement C1q, and cholesterol crystals (CC-C1q-VWF complex); (b) treating a population of macrophages with the CC-C1q-VWF complex; (c) treating the population of macrophages with a candidate agent; (d) measuring the level of phagocytosis in the population of macrophages; and (e) comparing the level of phagocytosis to the level of phagocytosis of a population of macrophages that have not been treated with the candidate agent.

In some embodiments, the step of measuring of the level of phagocytosis comprises detecting the percentage of CD11c-positive cells in the population of macrophages.

In one aspect, provided herein is an *in vitro* method of identifying a candidate agent for modulating expression of phagocytosis-mediating receptors and costimulatory receptors by macrophages comprising: (a) incubating rVWF, complement C1q, and cholesterol crystals to form a complex comprising rVWF, complement C1q, and cholesterol crystals (CC-C1q-VWF complex); (b) treating a population of macrophages with the CC-C1q-VWF complex; (c) treating the population of macrophages with a candidate agent; (d) measuring the level of expression of phagocytosis-mediating receptors and costimulatory receptors in the population of macrophages; and (e) comparing the level of expression to the level of expression of phagocytosis-mediating receptors and costimulatory receptors in a population of macrophages that have not been treated with the candidate agent.

In some embodiments, the phagocytosis-mediating receptors and costimulatory receptors are selected from the group consisting of CD14, CD86, leukocyte-associated immunoglobulin-like receptor 1 (LAIR1), lipoprotein receptor-related protein 1 (LRP-1), tyrosine protein kinase Mer (MerTk), MHC II, programmed death ligand 1 (PD-L1), scavenger receptor A 1 (SR-A1), and a combination thereof.

In one aspect, provided herein is an *in vitro* method of identifying a candidate agent for modulating pro-inflammatory cytokine secretion by macrophages comprising: (a) incubating rVWF, complement C1q, and cholesterol crystals to form a complex comprising rVWF, complement C1q, and cholesterol crystals (CC-C1q-VWF complex); (b) treating a population of macrophages with the CC-C1q-VWF complex; (c) treating the population of macrophages with a candidate agent; (d) measuring the level of pro-inflammatory cytokine secretion in the population of macrophages; and (e) comparing the level of pro-inflammatory cytokine secretion to the level of pro-inflammatory cytokine secretion in a population of macrophages that have not been treated with the candidate agent.

In some embodiments, the pro-inflammatory cytokine is selected from the group consisting of IL-1α, IL-1β, IL-6, IL-10, IL-18, IL-1R, TNF1α, and a combination thereof.

In some embodiments, the rVWF comprises a polypeptide selected from the group consisting of: (a) the amino acid sequence of SEQ ID NO:3; (b) a biologically active analog, fragment, or variant of (a); (c) a polypeptide encoded by the polynucleotide sequence of SEQ ID NO: 1; and (d) a biologically active analog, fragment, or variant of (c).

In some embodiments, the composition of rVWF comprises a high molecular weight vWF multimer comprising at least 20% VWF decamers or high order multimers. In certain embodiments, the composition of rVWF comprises a high molecular weight vWF multimer comprising at least 40% VWF decamers or high order multimers. In some embodiments, the composition of rVWF comprises a high molecular weight vWF multimer comprising at least 60% VWF decamers or high order multimers.

In some embodiments, the rVWF is matured in vitro by treatment with Furin.

In some embodiments, the rVWF has a higher specific activity compared to plasma-derived VWF.

In some embodiments, the macrophages of the *in vitro* method are human monocyte-derived macrophages.

Other objects, advantages and embodiments of the invention will be apparent from the detailed description following.

### BRIEF DESCRIPTION OF THE DRAWINGS

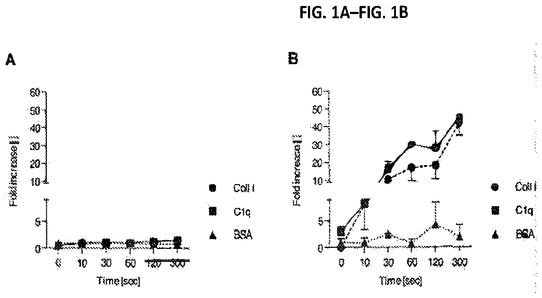

FIG. 1A and FIG. 1B: Time-dependent binding of vWF to Clq under (FIG. 1A) static and (FIG. 1B) dynamic conditions. Recombinant (r)vWF was added to surfaces coated with Clq and compared to collagen I (Coll I) and BSA as positive and negative controls, respectively. The binding of rvWF is expressed as fold-increase compared to binding to BSA at the start (0 sec).

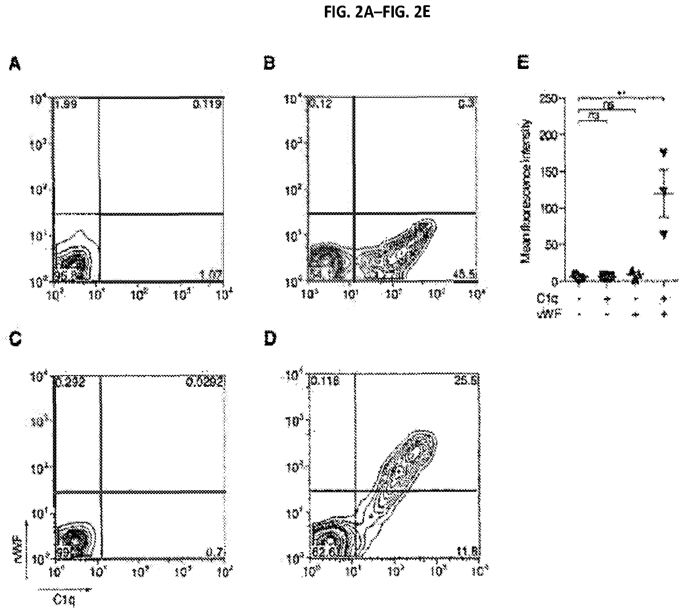

FIG. 2A-FIG. 2E: VWF-Clq interaction on apoptotic Jurkat cell-derived microparticles (MPs). Representative FACS diagrams showing (FIG. 2A) untreated MPs, (FIG. 2B) MPs incubated with Clq alone, (FIG. 2C) MPs incubated with rvWF alone and (FIG. 2D) MPs incubated with both Clq and vWF. (FIG. 2E) Pooled FACS data are shown as mean fluorescence intensity with standard deviation (n = 3, One Way ANOVA test, ns not significant, ** p < 0.01).

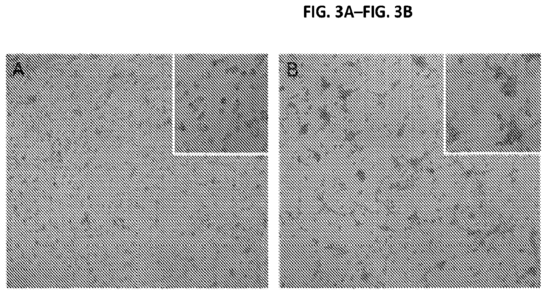

FIG. 3A and FIG. 3B: Morphological differences between human monocyte-derived macrophages having been exposed to Clq alone (elongated phenotype forming dendrites (FIG. 3A)), or Clq-vWF complexes (formation of aggregates (FIG. 3B)).

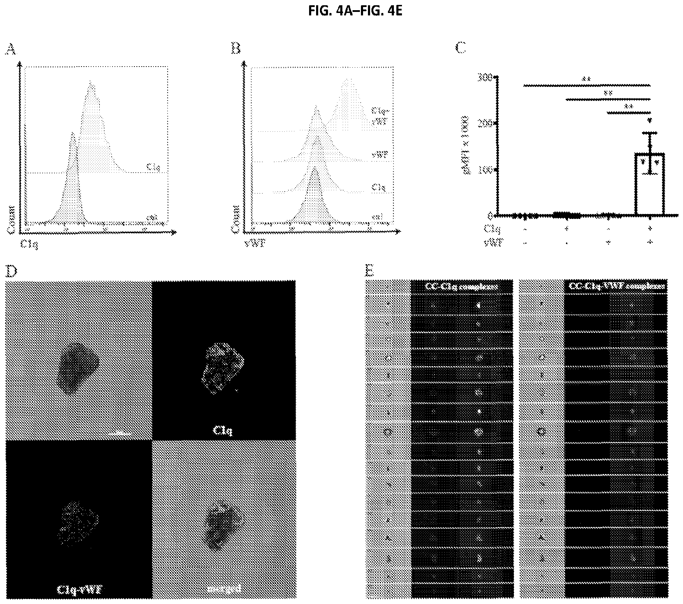

FIG. 4A-FIG. 4E: vWF binds to CC in a C1q-dependent manner. Binding of C1q and vWF to immobilized C1q on the surface of CC was determined by flow cytometry (FIG. 4A, FIG. 4B, FIG. 4C), confocal microscopy (FIG. 4D) and ImageStreamX (FIG. 4E). Representative flow cytometry diagrams show the binding of C1q (FIG. 4A) or C1q-dependent binding of vWF (FIG. 4B) on the surface of CC. Controls (ctrl) represent the presence of secondary antibodies only. Flow cytometry data are shown as mean gMFI±SD (*n* = 5, RM one-way ANOVA test and Tukey posttest, ** *p*<0.005) (FIG. 4C). Confocal microscopy depicting a representative CC in brightfield, with C1q (red) or vWF (blue) in the presence of C1q bound to its surface. The merged staining patterns visualize the C1q-vWF interaction (FIG. 4D). One of three independent experiments is shown. Scale bar = 50 µm. Fluorescent staining of CC captured by ImageStreamX for C1q (yellow) or C1q-vWF (red) (FIG. 4E). One of two independent experiments is shown. Scale bar = 10 µm.

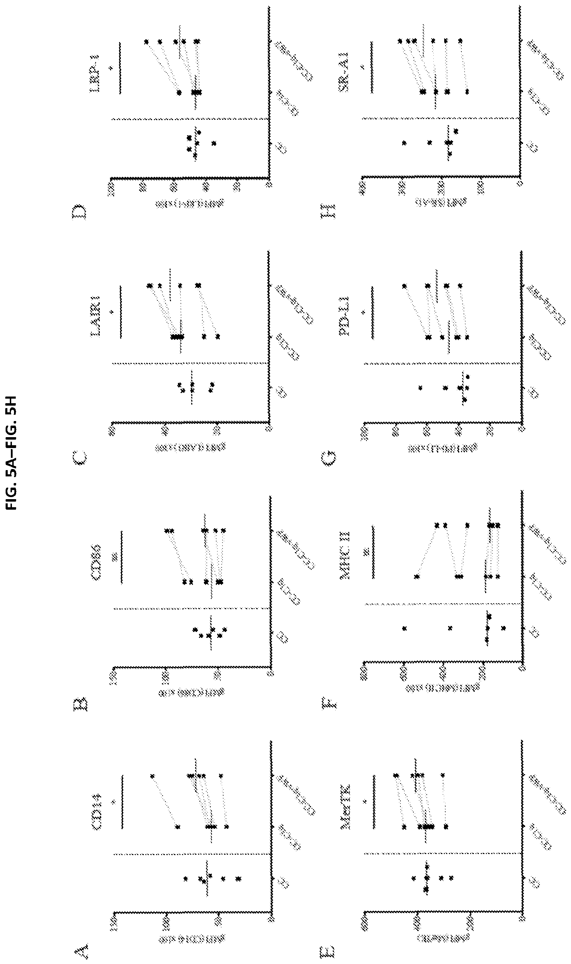

FIG. 5A-FIG. 5H: CC-C1q-vWF complexes upregulate the surface receptor expression of HMDMs. LPS-stimulated HMDMs were treated with CC, CC-C1q or CC-C1q-vWF complexes for 18 hr and analyzed by flow cytometry for surface marker expression of CD14 (FIG. 5A), CD86 (FIG. 5B), LAIR1 (FIG. 5C), LRP-1 (FIG. 5D), MerTK (FIG. 5E), MHC II (FIG. 5F), PD-L1 (FIG. 5G) and SR-A1 (FIG. 5H). Pooled flow cytometry data are shown as gMFI with horizontal lines denoting median. Data points represent independent experiments analyzing six different healthy donors used to obtain HMDMs (Wilcoxon matched pairs signed rank test, * *p*<0.05; ns = not significant).

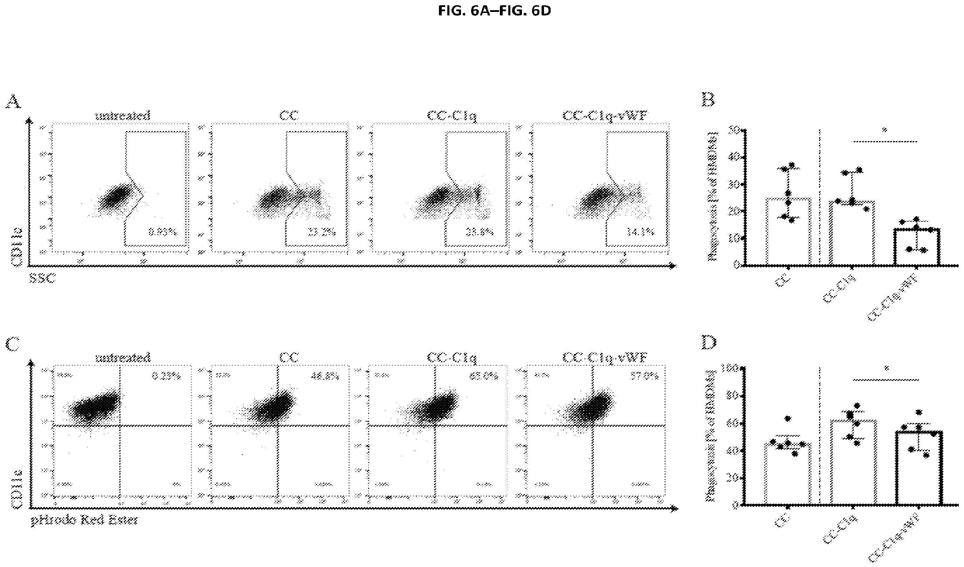

FIG. 6A-FIG. 6D: Phagocytosis of CC-C1q-vWF complexes by HMDMs is hampered. HMDMs were kept untreated or treated with CC, CC-C1q or CC-C1q-vWF complexes for 18 hr and analyzed by flow cytometry for the degree of phagocytosis of CC complexes (FIG. 6A, FIG. 6B). Phagocytosis was determined by the percentage of CD11c+ cells with a shift into the SSC^{high} gate. FACS dot plots show one out of six independent experiments (FIG. 6A). Quantification of phagocytosis for pooled data is shown in (FIG. 6B). HMDMs were treated with CC, CC-C1q or CC-C1q-vWF pHrodo-dyed complexes for 30 min and analyzed by flow cytometry for the degree of phagocytosis of CC complexes (FIG. 6C, FIG. 6D). Phagocytosis was determined by the percentage of CD11c+ cells with a shift into pHrodo Red Ester+ gate. FACS dot plots show one out of six independent experiments (FIG. 6C). Quantification of phagocytosis for pooled data is shown in (FIG. 6D).
   Horizontal lines denote median while error bars indicate interquartile range. Data points represent independent experiments analyzing six different healthy donors used to obtain HMDMs (Wilcoxon matched pairs signed rank test, * *p*<0.05).

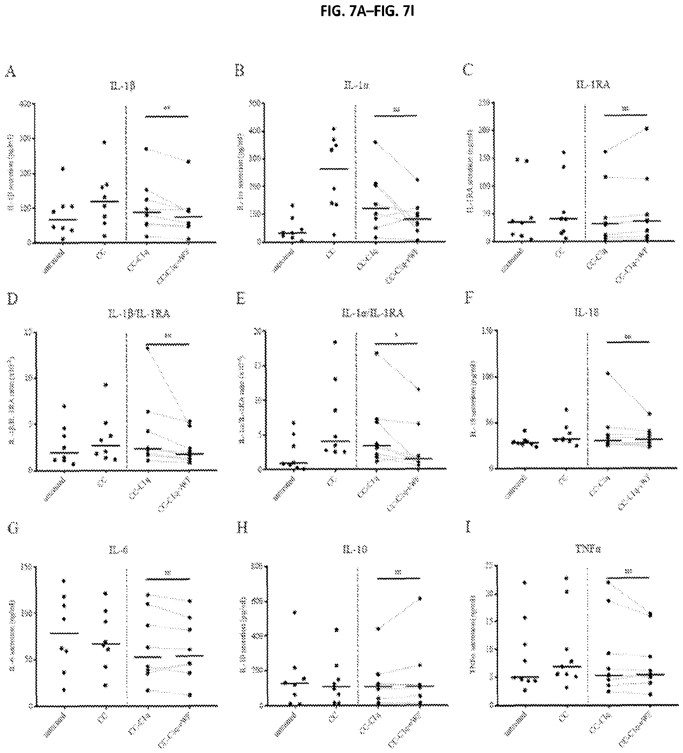

FIG. 7A-FIG. 7I: CC-C1q-vWF complexes diminish LPS-induced IL-1 secretion of HMDMs. LPS-induced HMDMs were kept untreated or treated with CC, CC-C1q or CC-C1q-vWF complexes for 18 hr. Supernatants were analyzed by ELISA for IL-1a (FIG. 7A), IL-1β (FIG. 7B), IL-1RA (FIG. 7C), IL-18 (FIG. 7F), IL-6 (FIG. 7G), IL-10 (FIG. 7H) and TNFa (FIG. 7I) levels. Data show median concentrations or median ratio of pooled cytokine levels. Data points represent independent experiments analyzing eight different healthy donors used to obtain HMDMs (Wilcoxon matched pairs signed rank test, * *p*<0.05; ** *p*<0.005; ns = not significant).

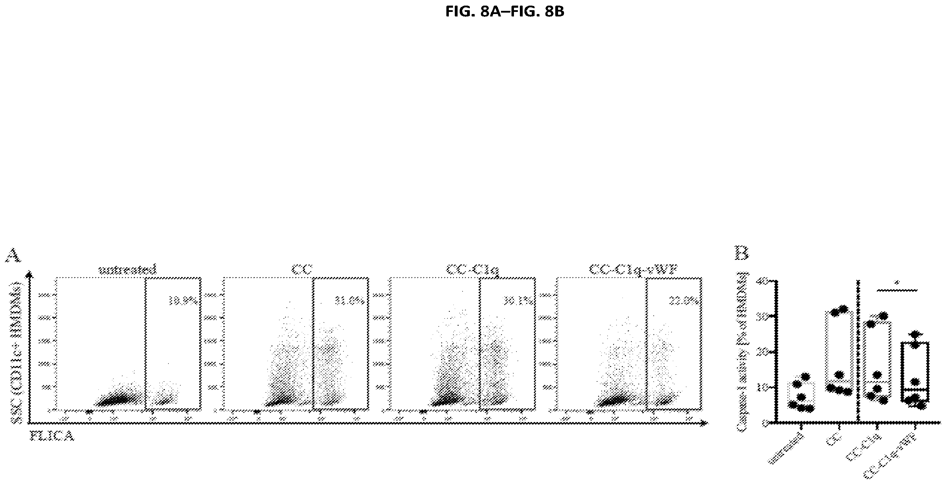

FIG. 8A and FIG. 8B: CC-C1q-vWF complexes exhibit a reduced caspase-1 activity in HMDMs. HMDMs were kept untreated or treated with CC, CC-C1q or CC-C1q-vWF complexes for 18 hr and analyzed for caspase-1 activity by flow cytometry. Activity of caspase-1 was determined by the percentage of CD11c+ cells in the FLICA+ gate. FACS dot plots show one out of six independent experiments (FIG. 8A). Quantification of caspase-1 activity for pooled data is shown in (FIG. 8B). Horizontal lines denote median while error bars indicate interquartile range. Data points represent independent experiments analyzing six different healthy donors used to obtain HMDMs (Wilcoxon matched pairs signed rank test, * *p*<0.05).

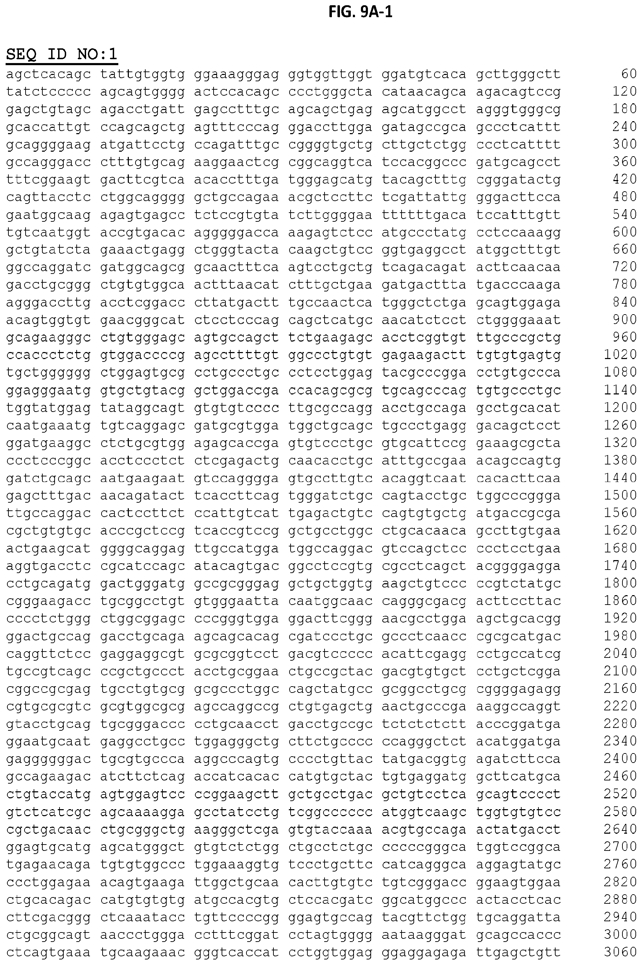

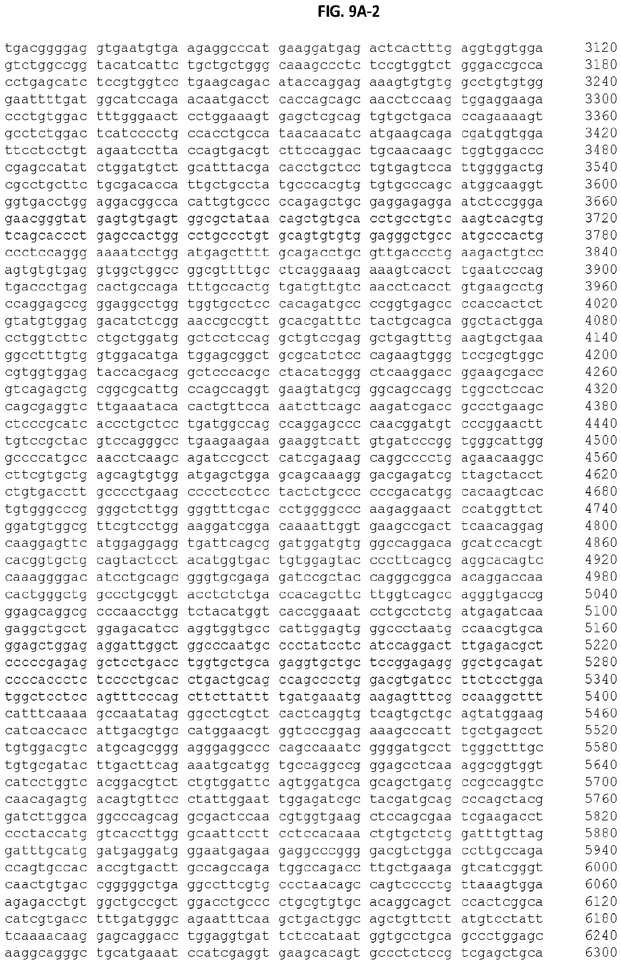

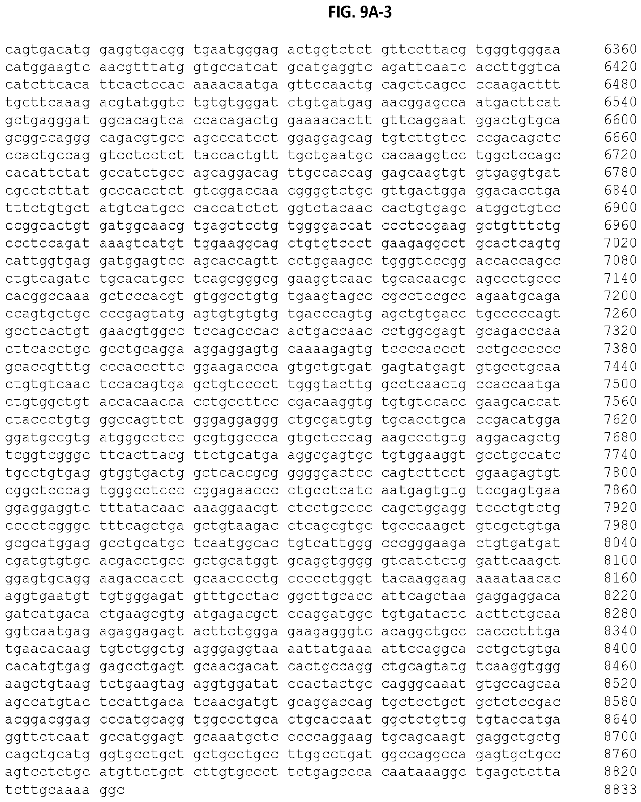

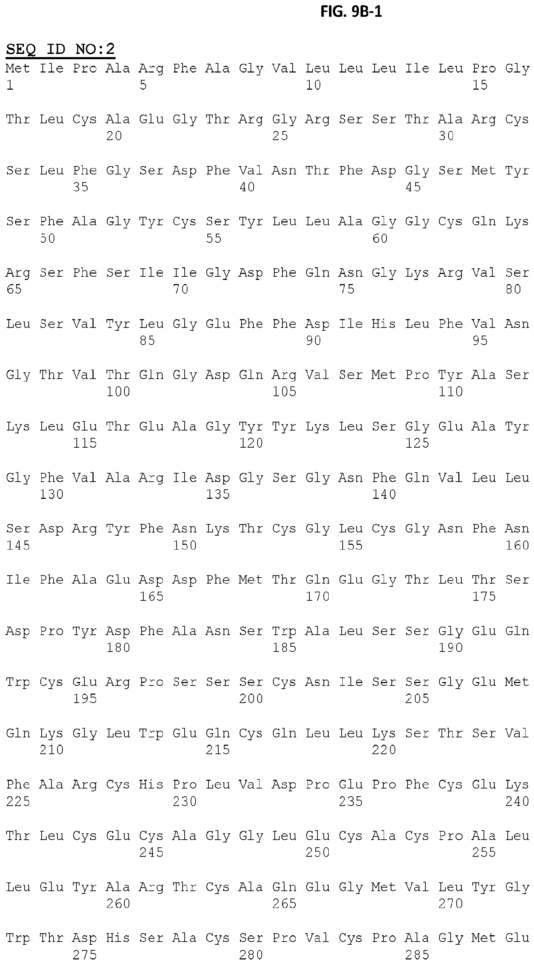

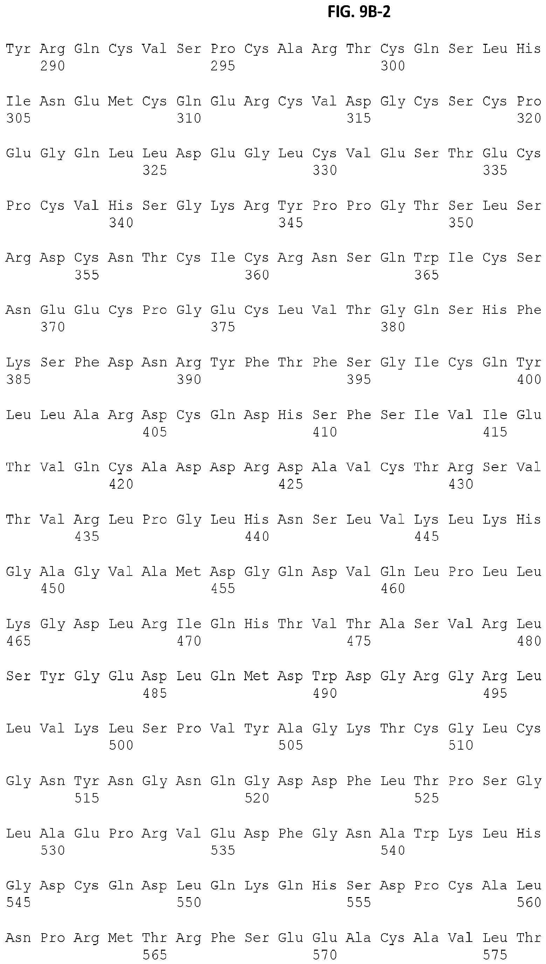

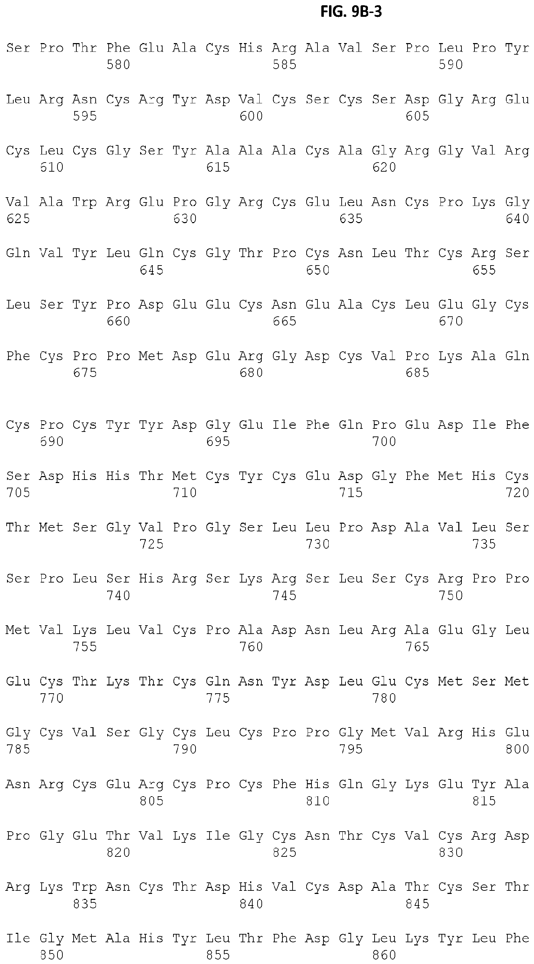

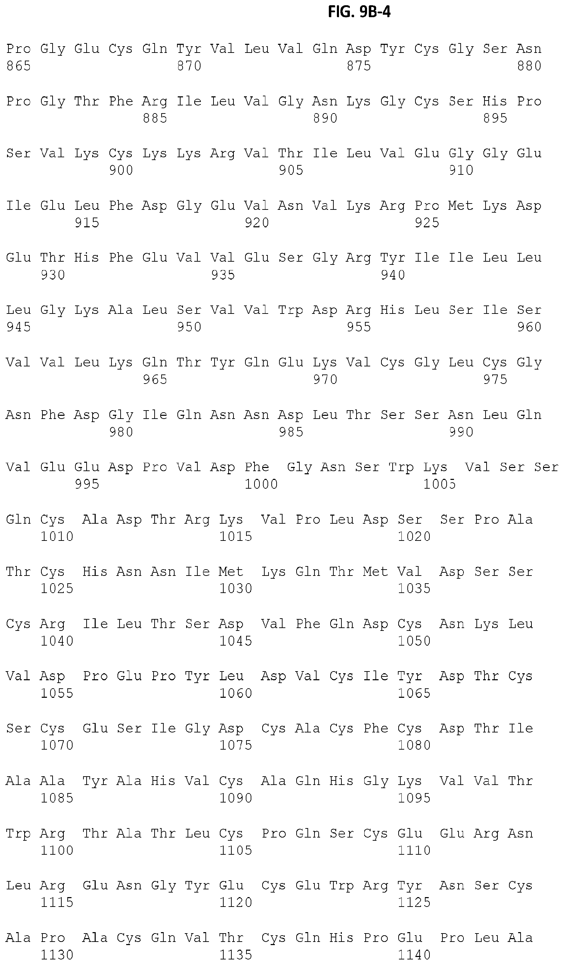

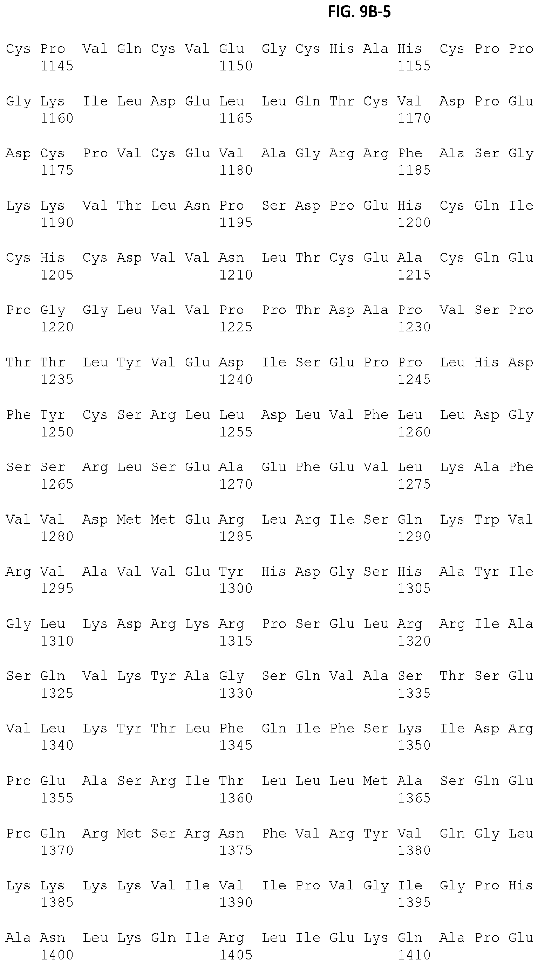

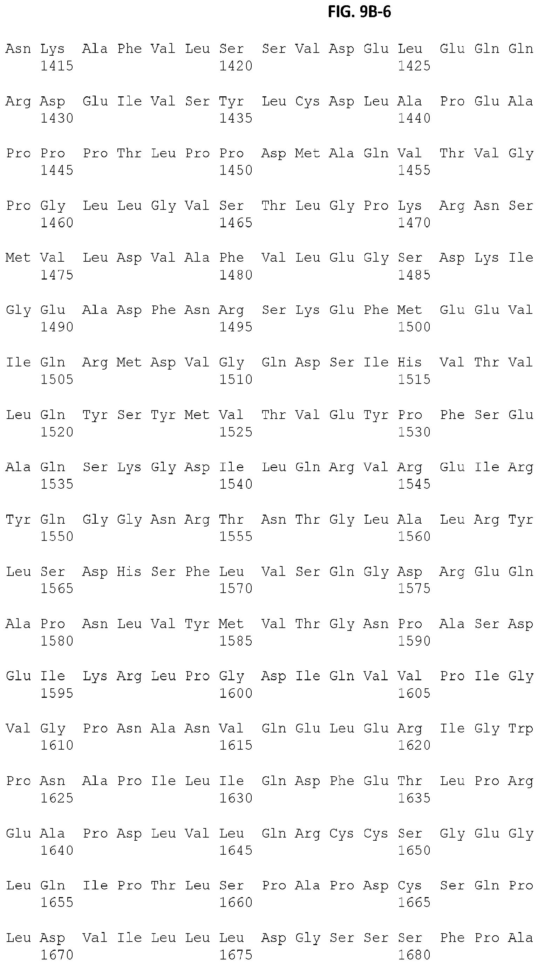

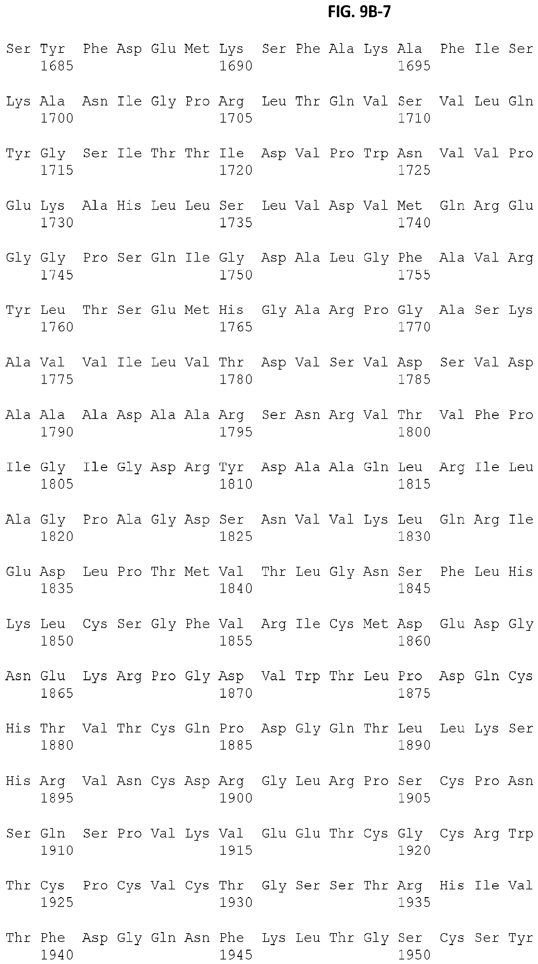

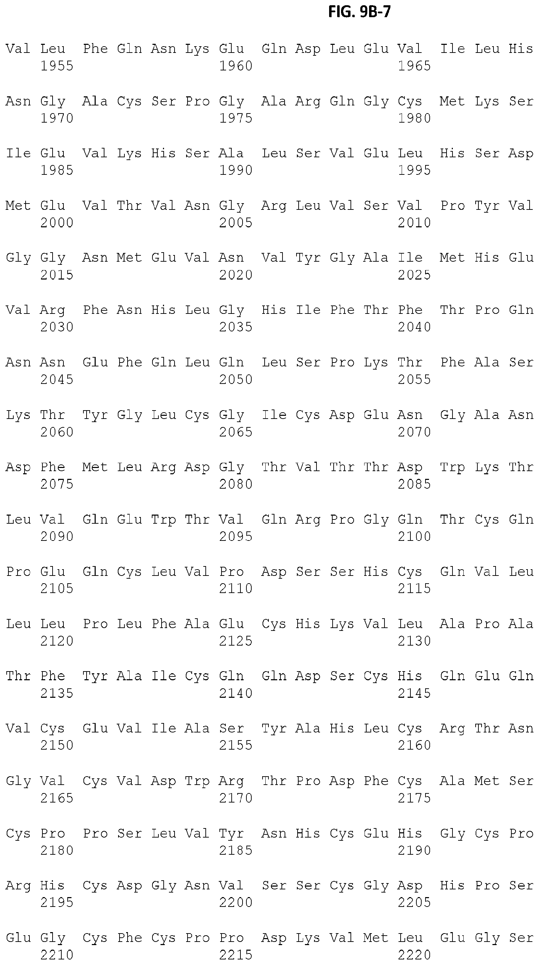

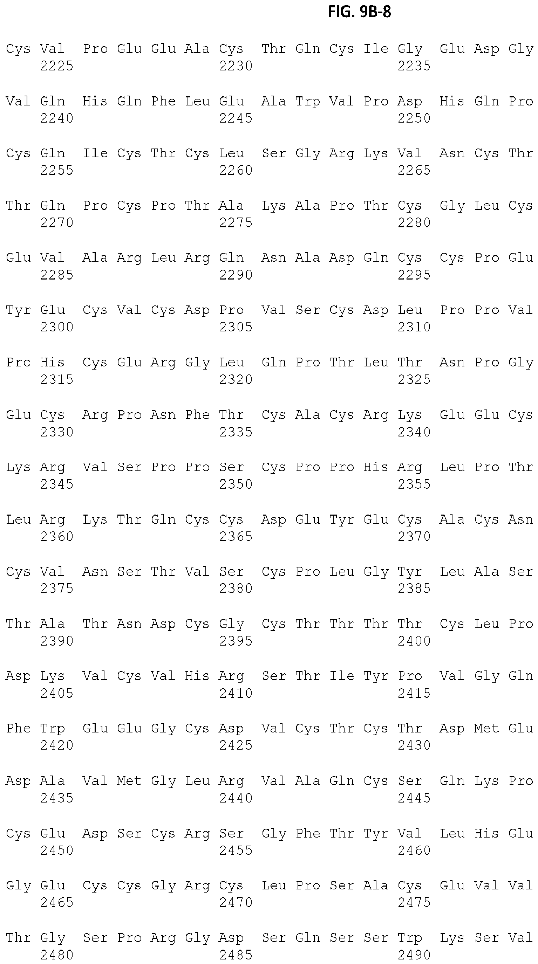

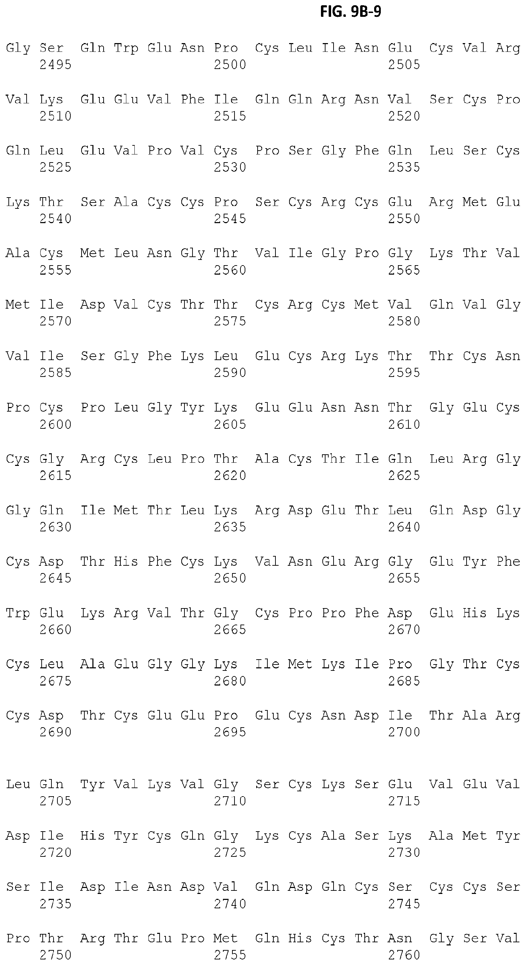

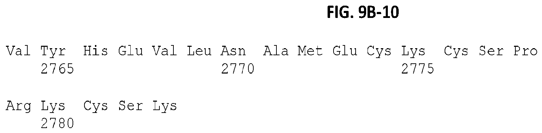

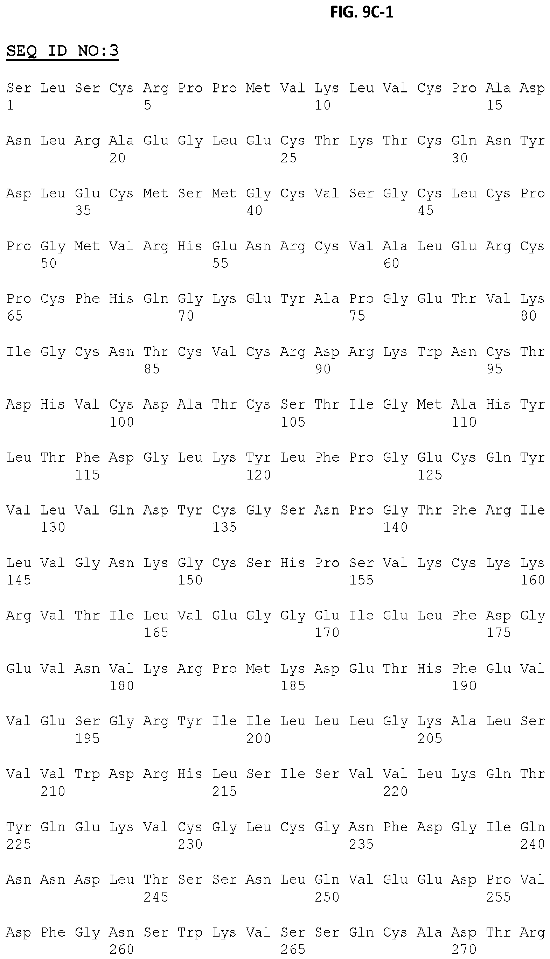

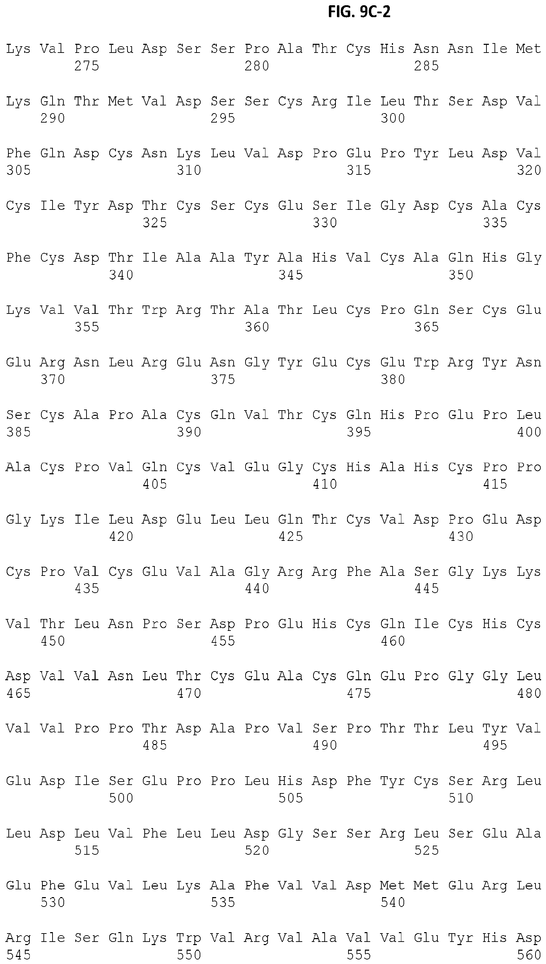

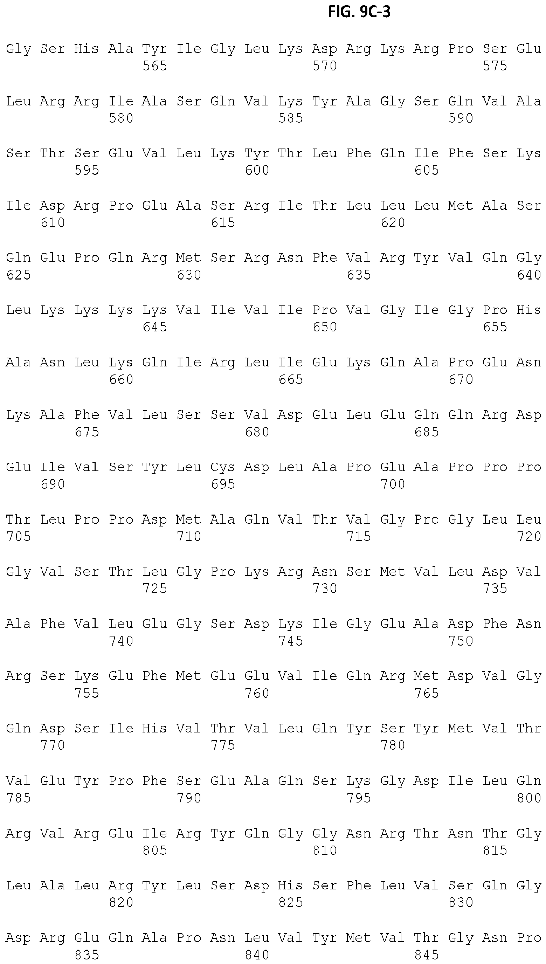

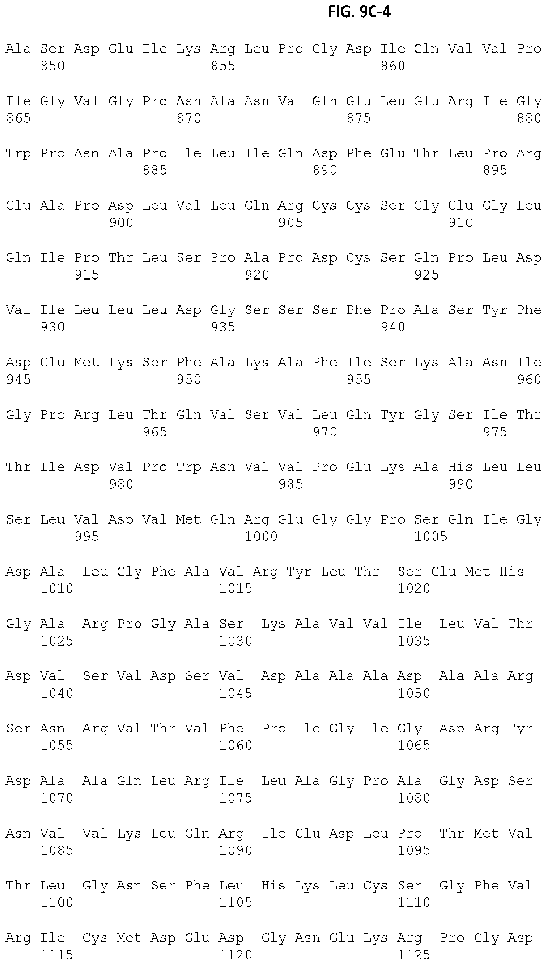

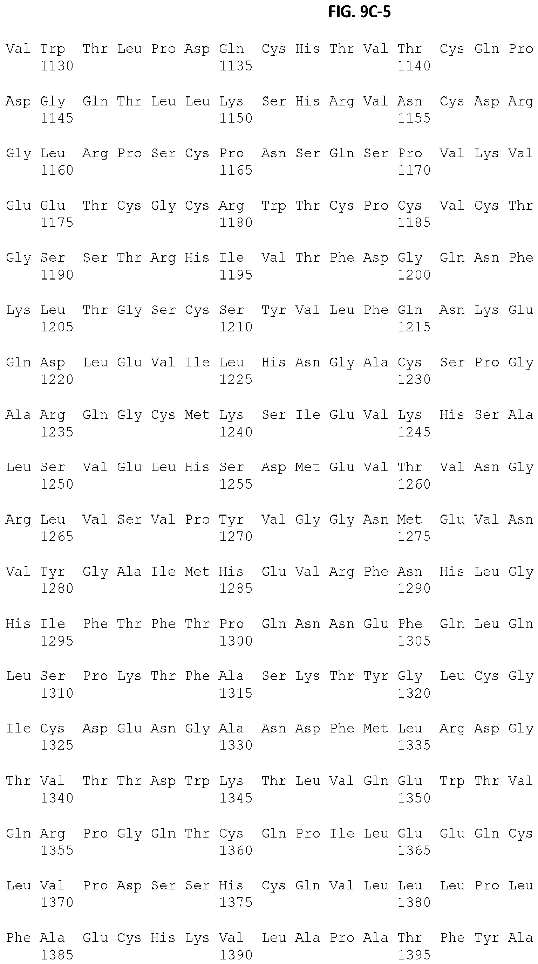

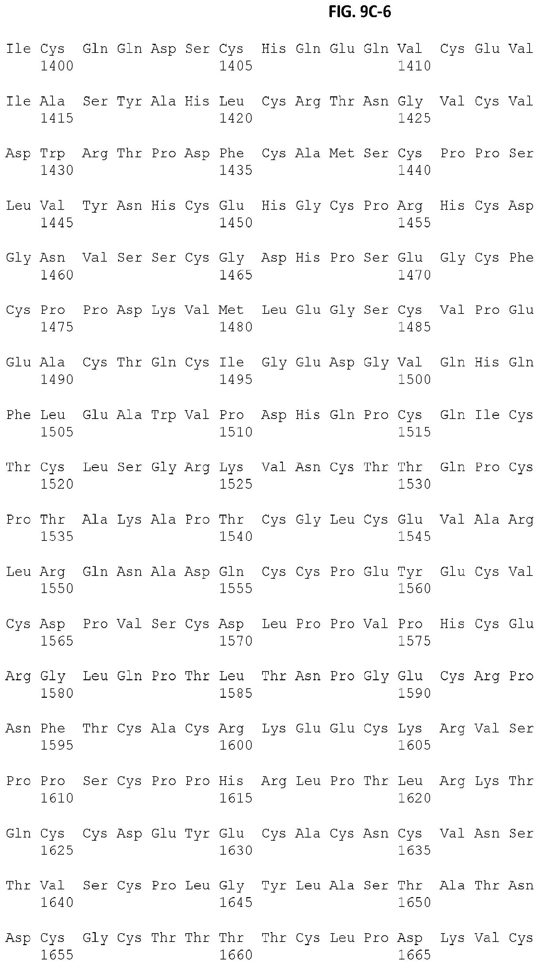

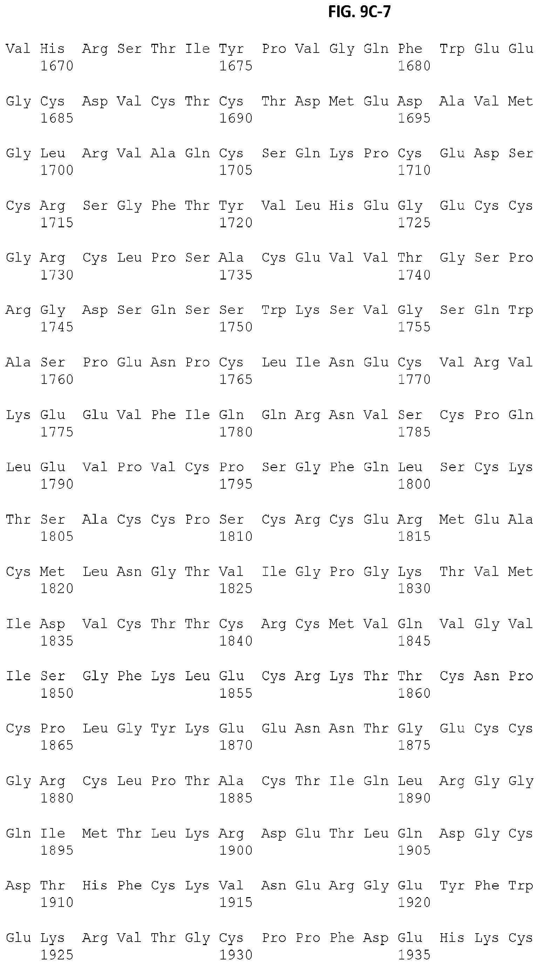

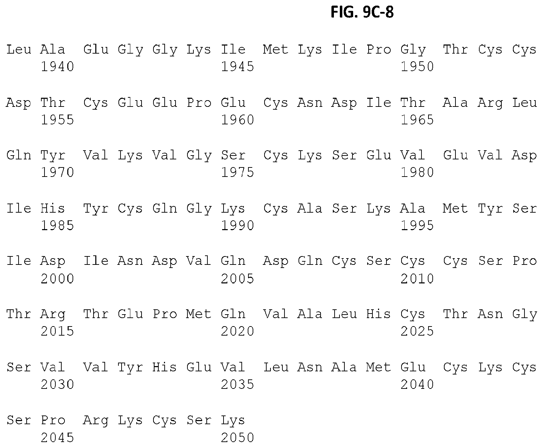

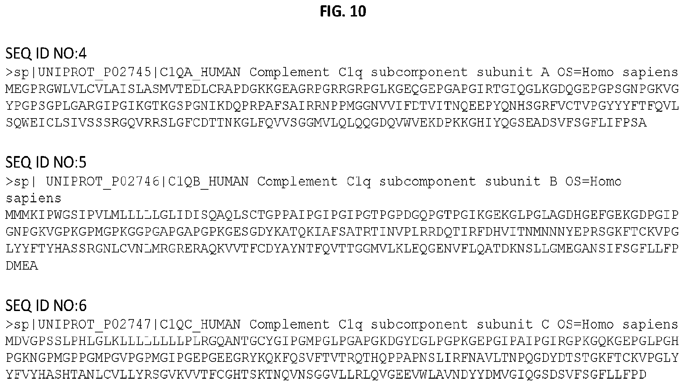

FIG. 9A-1 - FIG. 9A-3, FIG. 9B-1 - FIG. 9B-10, and FIG. 9C-1 - FIG. 9C-8 show VWF nucleic acid and amino acid sequences. FIG. 9A-1 - FIG. 9A-3 provides SEQ ID NO:1. FIG. 9B-1 - FIG. 9B-10 provides SEQ ID NO:2. FIG. 9C-1 - FIG. 9C-8 provides SEQ ID NO:3
FIG. 10 shows amino acid sequences of human complement factor C1q subunit A (SEQ ID NO:4), subunit B (SEQ ID NO:5), and subunit C (SEQ ID NO:6).

### DETAILED DESCRIPTION OF THE INVENTION

### Introduction

The present invention provides methods for treating atherosclerosis in a subject comprising administering recombinant VWF such that the VWF binds complement C1q to form C1q-VWF complexes. In some aspects, the methods reduce inflammation in an atherosclerotic plaque. In other aspects, the methods modulate immune responses of macrophages during atherosclerosis progression.

Also provided are *in vitro* methods for identifying a candidate agent for modulating phagocytosis of macrophages. In some aspects, the *in vitro* methods are used for identifying a candidate agent that modulates expression of phagocytosis-mediating receptors and costimulatory receptors by macrophages. In certain aspects, the *in vitro* methods are for identifying a candidate agent that modulates secretion of pro-inflammatory cytokines by macrophages.

### Definitions

Before the invention is further described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

As used herein. "rVWF" refers to recombinant VWF.

As used herein, "rFVIII" refers to recombinant FVIII.

The term "recombinant" when used with reference, *e.g.*, to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, for example, recombinant cells express genes that are not found within the native (non-recombinant) form of the cell or express native genes that are otherwise abnormally expressed, under expressed or not expressed at all.

As used herein, "recombinant VWF" includes VWF obtained via recombinant DNA technology. In certain embodiments, VWF proteins of the invention can comprise a construct, for example, prepared as in WO 1986/06096 published on Oct. 23, 1986 and U.S. patent application Ser. No. 07/559,509, filed on Jul. 23, 1990 .

The VWF in the present invention can include all potential forms, including the monomeric and multimeric forms. It should also be understood that the present invention encompasses different forms of VWF to be used in combination. For example, the VWF of the present invention may include different multimers, different derivatives and both biologically active derivatives and derivatives not biologically active.

In the context of the present invention, the recombinant VWF embraces any member of the VWF family from, for example, a mammal such as a primate, human, monkey, rabbit, pig, rodent, mouse, rat, hamster, gerbil, canine, feline, and biologically active derivatives thereof. Mutant and variant VWF proteins having activity are also embraced, as are functional fragments and fusion proteins of the VWF proteins. Furthermore, the VWF of the invention may further comprise tags that facilitate purification, detection, or both. The VWF described herein may further be modified with a therapeutic moiety or a moiety suitable imaging in vitro or in vivo.

As used herein, "plasma-derived VWF (pdVWF)" includes all forms of the protein found in blood including the mature VWF obtained from a mammal having the property of in vivo-stabilizing, *e.g.* binding, of at least one FVIII molecule.

The term "highly multimeric VWF" or "high molecular weight VWF" refers to VWF comprising at least 10 subunits, or 12, 14, or 16 subunits, to about 20, 22, 24 or 26 subunits or more. The term "subunit" refers to a monomer of VWF. As is known in the art, it is generally dimers of VWF that polymerize to form the larger order multimers (see, Turecek et al., Semin. Thromb. Hemost. 2010, 36(5): 510-521 .

As used herein, the term "factor VIII" or "FVIII" refers to any form of factor VIII molecule with the typical characteristics of blood coagulation factor VIII, whether endogenous to a patient, derived from blood plasma, or produced through the use of recombinant DNA techniques, and including all modified forms of factor VIII. Factor VIII (FVIII) exists naturally and in therapeutic preparations as a heterogeneous distribution of polypeptides arising from a single gene product (*see*, *e.g.*, Andersson et al., Proc. Natl. Acad. Sci. USA, 83:2979-2983 (1986)). Commercially available examples of therapeutic preparations containing Factor VIII include those sold under the trade names of HEMOFIL M^{®}, ADVATE^{®}, and RECOMBINATE^{®} (available from Baxter Healthcare Corporation, Deerfield, Ill., U.S.A.).

The terms "isolated," "purified," or "biologically pure" refer to material that is substantially or essentially free from components that normally accompany it as found in its native state. Purity and homogeneity are typically determined using analytical chemistry techniques such as polyacrylamide gel electrophoresis or high performance liquid chromatography. VWF is the predominant species present in a preparation is substantially purified. The term "purified" in some embodiments denotes that a nucleic acid or protein gives rise to essentially one band in an electrophoretic gel. In other embodiments, it means that the nucleic acid or protein is at least 50% pure, more preferably at least 60%, 65%, 70%, 75%, 80%, 85%, 90%, 95%, 96%, 97%, 98%, 99% or more pure. "Purify" or "purification" in other embodiments means removing at least one contaminant from the composition to be purified. In this sense, purification does not require that the purified compound be homogenous, *e.g.*, 100% pure.

As used herein, "plasma FVIII activity" and "in vivo FVIII activity" are used interchangeably. The in vivo FVIII activity measured using standard assays may be endogenous FVIII activity, the activity of a therapeutically administered FVIII (recombinant or plasma derived), or both endogenous and administered FVIII activity. Similarly, "plasma FVIII" refers to endogenous FVIII or administered recombinant or plasma derived FVIII.

As used herein, the term "half-life" refers to the period of time it takes for the amount of a substance undergoing decay (or clearance from a sample or from a patient) to decrease by half.

As used herein "von Willebrand Disease" refers to the group of diseases caused by a deficiency of von Willebrand factor. Von Willebrand factor helps blood platelets clump together and stick to the blood vessel wall, which is necessary for normal blood clotting. As described in further detail herein, there are several types of Von Willebrand disease including type 1, 2A, 2B, 2M and 3.

The term "monocyte" is an immature phagocyte circulating in the blood, and refers to a cell serving as an antigen-presenting immune cell, having a function of capturing and decomposing a pathogen and a foreign substance by phagocytosis.

The term "macrophage" refers to a cell positive for CD14 serving as a marker of the macrophage. Furthermore, the macrophages can be classified into an active macrophage including the M1 (inflammatory type) macrophage or the M2 (anti-inflammatory type) macrophage, and a resting macrophage according to the differentiated type thereof. As used herein, the term "macrophage" includes macrophage-derived foam cells.

The term "foam cell" refers to a lipid-containing macrophage that localizes to fatty deposits on the walls of blood vessels. Foam cells formation can be induced by low density lipoproteins (LDL) such as oxidized LDL (OxLDL) or minimally modified LDL (mmLDL), and is associated with an imbalance of cholesterol influx, esterification and efflux. Formation of foam cells is key in the progression of atherosclerosis.

As used herein "complement factor C1q," "complement factor C1q," and "C1q" refers to both wild type sequences and naturally occurring variant sequences thereof from any organism having a classical complement activation system, including any mammalian organism such as human, mouse, rat, rabbit, monkey, dog, cat, cow, horse, camel, sheep, goat, or pig. Human complement factor C1q is the recognition component of the classical complement activation pathway.

Human C1q is composed of 18 polypeptide chains comprising three types of polypeptide chains: A, B, and C chains. Human C1q includes 6 human C1q, chain A polypeptides, 6 human C1q, chain B polypeptides, and 6 human C1q, chain C polypeptides.

An exemplary amino acid sequence of human C1q, chain A (complement C1q subcomponent subunit A) can be found as UniProt No. P02745 (SEQ ID NO:4) or any polymorphic variant thereof, and its mature form of 223 amino acids spanning positions 23-245 of SEQ ID NO:4. The amino acid sequence of human C1q, chain A can be found as NCBI Ref. Sequence Nos. NP_057075.1, NP_001334394.1, and NP_001334395.1, and a corresponding nucleic acid sequence can be found as NCBI Ref. Sequence Nos. NM_015991.4, NM_001347465.2, and NM_001347466.1, respectively. In some embodiments, the amino acid sequence of human C1q, chain A refers to a mature form of any of the sequences provided herein.

An exemplary amino acid sequence of human C1q, chain B (complement C1q subcomponent subunit B) can be found as UniProt No. P02746 (SEQ ID NO:5) or any polymorphic variant thereof, and its mature form of 226 amino acids spanning positions 28-253 of SEQ ID NO:5. The amino acid sequence of human C1q, chain B can be found as NCBI Ref. Sequence No. NP_000482.3, and XP_011540361.1, and a corresponding nucleic acid sequence can be found as NCBI Ref. Sequence Nos. NM_000491.3 and XM_011542059.2, respectively. In some embodiments, the amino acid sequence of human C1q, chain B refers to a mature form of any of the sequences provided herein.

An exemplary amino acid sequence of human C1q, chain C can be found as UniProt No. P02747 (SEQ ID NO:6) or any polymorphic variant thereof, and its mature form of 217 amino acids spanning positions 29-245 of SEQ ID NO:6. The amino acid sequence of human C1q, chain C can be found as NCBI Ref. Sequence No. NP_001107573.1, NP_001334548.1, NP_758957.2, and a corresponding nucleic acid sequence can be found as NCBI Ref. Sequence No. NM_001114101.3, NM_001347619.1, and NM_172369.4, respectively. In some embodiments, the amino acid sequence of human C1q, chain C refers to a mature form of any of the sequences provided herein.

The best-known ligands for C1q are the Fc regions of aggregated immunoglobulin IgG and IgM molecules in immune complexes. Such binding triggers activation of the classical pathway, one of the three mechanisms for the activation of complement system. The result of activation of the complement system is the generation of C3 and C5 convertases that generate pro-inflammatory C3a and C5a anaphylatoxins and catalyse formation of pore-like membrane attack complex that inserts into cells membranes and cause damages by lytic or sublytic mechanisms.

C1q is implicated in the biology of antigen presenting cells such as monocytes, macrophages and dendritic cells which express or secrete this molecule (Lu, et al., Cell Mol Immunol 5 (2008) 9-21). In vitro treatment of monocytes by C1q regulates cell differentiation and confers a tolerogenic phenotype to monocyte-derived dendritic cells (Castellano et al., Eur. J. Immunol., 2007, 37, 2803-2811; Fraser et al,. J. Immunol., 2009, 183, 6175-6185). In contrast, C1q deficiency impairs the recognition and clearance of apoptotic cells which leads to the development of autoimmunity (*e.g*. systemic lupus erythematosus (SLE), glomerulonephritis) (Botto, et al., Nat. Genet., 1998, 19, 56-59; Nauta, et al., 2002, Eur. Immunol., 32, 1726-1736).

The term "cholesterol crystal" or "CC" refers to crystals of unesterified cholesterol. Macrophages latch onto extracellular aggregated LDL and digest regions of the aggregated LDL that are adjacent or nearby to the macrophages, resulting in the release of significant amounts of unesterified cholesterol. This unesterified cholesterol is a source for the formation of extracellular cholesterol crystals. *See*, *e.g.*, Guarino, et al., Biochemistry. 2004, 43:1685-93; Haka, et al., J Cell Sci. 2016, 129:1072-82; Duewell, et al., Nature. 2010, 464:1357-61; and Rajamaki, et al., PLoS One. 2010, 5:e11765.

Macrophage foam cells produce free cholesterol crystals that extend from intracellular membrane sites with various morphologies, such as but not limited to, plates, needles and helices. Highly organized cholesterol structures, characterized by a unit cell periodicity of 34.0Å, appear to serve as nucleating sites for the formation of extracellular crystals.

As used herein, "administering" (and all grammatical equivalents) includes intravenous administration, intramuscular administration, subcutaneous administration, oral administration, administration as a suppository, topical contact, intraperitoneal, intralesional, or intranasal administration, or the implantation of a slow-release device, *e.g.*, a mini-osmotic pump, to a subject. Administration is by any route including parenteral, and transmucosal (*e.g.*, oral, nasal, vaginal, rectal, or transdermal). Parenteral administration includes, *e.g.*, intravenous, intramuscular, intra-arteriole, intradermal, subcutaneous, intraperitoneal, intraventricular, and intracranial. Other modes of delivery include, but are not limited to, the use of liposomal formulations, intravenous infusion, transdermal patches, *etc.*

The terms "therapeutically effective amount or dose" or "therapeutically sufficient amount or dose" or "effective or sufficient amount or dose" refer to a dose that produces therapeutic effects for which it is administered. For example, a therapeutically effective amount of a drug useful for treating atherosclerosis can be the amount that is capable of preventing or relieving one or more symptoms associated with atherosclerosis. The exact dose will depend on the purpose of the treatment, and will be ascertainable by one skilled in the art using known techniques (*see*, *e.g.*, Lieberman, Pharmaceutical Dosage Forms (vols. 1-3, 1992); Lloyd, The Art, Science and Technology of Pharmaceutical Compounding (1999); Pickar, Dosage Calculations (1999); and Remington: The Science and Practice of Pharmacy, 20th Edition, 2003, Gennaro, Ed., Lippincott, Williams & Wilkins).

As used herein, the term "preventing" includes providing prophylaxis with respect to occurrence or recurrence of a particular disease, disorder, or condition in an individual. An individual may be predisposed to, susceptible to a particular disease, disorder, or condition, or at risk of developing such a disease, disorder, or condition, but has not yet been diagnosed with the disease, disorder, or condition.

As used herein, the term "treating" includes providing a clinical intervention designed to alter the natural course of the individual being treated during the course of clinical pathology. Desirable effects of treatment include decreasing the rate of progression, ameliorating or palliating the pathological state, and remission or improved prognosis of a particular disease, disorder, or condition. An individual is successfully "treated", for example, if one or more symptoms associated with a particular disease, disorder, or condition are mitigated or eliminated.

As used herein, the terms "patient," "individual," and "subject" are used interchangeably and refer to a mammal (preferably human) that has a disease or has the potential of contracting a disease.

As used herein, the term "about" denotes an approximate range of plus or minus 10% from a specified value. For instance, the language "about 20%" encompasses a range of 18-22%.

As used herein, the term "half-life" refers to the period of time it takes for the amount of a substance undergoing decay (or clearance from a sample or from a patient) to decrease by half.

### I. Complexes Containing Complement C1q and VWF

This invention is based, in part, on the discovery of complexes comprising VWF and complement C1q that decrease the phagocytosis of cholesterol crystals (CC). The binding of C1q and VWF also modulates the immune response of macrophages including macrophage derived foam cells. In some embodiments, C1q and VWF binding upregulates expression of phagocytosis-mediating receptors and costimulatory receptors by macrophages including macrophage derived foam cells. In some embodiments, C1q and VWF binding increases suppression of pro-inflammatory cytokine secretion by macrophages including macrophage derived foam cells.

Provided herein are methods of treatment comprising administering a therapeutically effective amount of a composition comprising VWF such that the VWF binds complement C1q such as endogenous complement C1q in the subject. In some embodiments, recombinant VWF is administered to a subject and forms a complex with endogenous C1q that is beneficial to treating atherosclerosis. In some embodiments, the complex comprising C1q and VWF reduces inflammation of an atherosclerotic plaque. In certain embodiments, the complex comprising C1q and VWF modulates an immune response by macrophages in a blood vessel in a subject. In some cases, the subject has or is at risk of having atherosclerosis. In some embodiments, the complex comprises C1q, CC, and VWF.

In some embodiments, complexes comprising C1q and VWF mediate phagocytosis of CC by macrophages in a subject with atherosclerosis. In some embodiments, the complex comprises C1q, CC, and VWF wherein C1q is bound to both VWF and the surface of cholesterol crystals.

Methods for detecting complexes comprising C1q and VWF include, but are not limited to, ELISAs, capture ELISAs, Western blots, immunoassays, and the like.

In some embodiments of a capture ELISA, a sample comprising such a complex is added to 96-well microplates that have been coated with either an anti-VWF antibody or an anti-C1q antibody prior to blocking. After an incubation period, the plates are washed and binding of VWF and/or C1q is detected. In some cases, C1q of the complex is detected using an anti-human C1q antibody such as a monoclonal mouse anti-human C1q antibody. Following an incubation period and a washing step, a detectable moiety-conjugated secondary goat anti-mouse antibody is added and a signal from the detectable moiety detected and measured. In certain cases, VWF of the complex is detected using an anti-human VWF antibody such as polyclonal rabbit anti-human VWF antibody. Following an incubation period and a washing step, a detectable moiety-conjugated secondary goat anti-rabbit antibody is added and a signal from the detectable moiety detected and measured.

In some embodiments, complexes comprising C1q and VWF are useful for reducing, retarding, suppressing, inhibiting, or preventing foam cell formation. In some embodiments, complexes comprising C1q and VWF are beneficial to reducing, retarding, suppressing, inhibiting, or preventing inflammation associated with atherosclerosis. In certain embodiments, complexes comprising C1q and VWF possess atheroprotective properties. In some embodiments, the complex comprises C1q, CC, and VWF.

In some embodiments, complexes comprising C1q and VWF inhibit atherosclerotic plaque (also referred to as "atherosclerotic lesion") progression. In some instances, such complexes inhibit formation of a fatty streak comprising an intima in a blood vessel of the subject. In certain instances, such complexes inhibit formation of a fibrous cap lesion. In some instances, such complexes inhibit formation of an established atherosclerotic lesion. In some embodiments, complexes comprising C1q and VWF reduce recruitment or infiltration of one or more cells comprising dendritic cells, macrophages, smooth muscle cells, and T cells into the lesion. In certain instances, such complexes inhibit or retard the formation of foam cells. In some embodiments, such complexes inhibit the formation of a vulnerable atherosclerotic plaque. In particular embodiments, such complexes inhibit the formation of a ruptured atherosclerotic plaque.

In some embodiments, complexes comprising C1q and VWF reduce the activation of inflammatory responses by macrophages in an atherosclerotic plaque. In some embodiments, complexes comprising C1q and VWF mediate the surface expression of receptors such as but limited to efferocytosis receptors, scavenger receptors, and costimulatory receptors on macrophages in an atherosclerotic plaque.

In some instances, the complex increases the expression level of one or more phagocytosis-mediating receptors and costimulatory receptors selected from the group consisting of CD14, CD86, leukocyte-associated immunoglobulin-like receptor 1 (LAIR1), lipoprotein receptor-related protein 1 (LRP-1), tyrosine protein kinase Mer (MerTk), MHC II, programmed death ligand 1 (PD-L1), scavenger receptor A 1 (SR-A1), and a combination thereof. In other embodiments, the complex increases the expression level of one or more phagocytosis-mediating receptors or costimulatory receptors selected from the group consisting of CD14, CD86, leukocyte-associated immunoglobulin-like receptor 1 (LAIR1; also referred to as CD305), lipoprotein receptor-related protein 1 (LRP-1; also referred to as CD91), tyrosine protein kinase Mer (MerTK), MHC II, programmed death ligand 1 (PD-L1; also referred to as CD274), scavenger receptor A 1 (SR-A1; also referred to as CD204), and a combination thereof.

In some embodiments, the complex increases the expression level of efferocytosis receptor MerTK.

In some embodiments, the complex increases the expression level of one or more scavenger receptors selected from the group consisting of LRP-1 and SR-A1. In some instances, the surface expression level of LRP-1 is increased. In some instances, the surface expression level of SR-A1 is increased. In certain embodiments, the complex increases the expression level of two scavenger receptors selected from the group consisting of LRP-1, and SR-A1. In some instances, the surface expression levels of LRP-1 and SR-A1 are increased.

In some embodiments, the complex increases the expression level of one or more costimulatory receptors selected from the group consisting of CD14, LAIR1, and PD-L1. In other embodiments, the complex increases the expression levels of two or more costimulatory receptors selected from the group consisting of CD14, LAIR1, and PD-L1. In some instances, the surface expression level of CD14 is increased. In some instances, the surface expression level of LAIR1 is increased. In some instances, the surface expression level of PD-L1 is increased. In certain embodiments, the complex increases the expression levels of costimulatory receptors CD14 and PD-L1. In some embodiments, the complex increases the expression levels of costimulatory receptors CD14 and LAIR1. In some embodiments, the complex increases the expression levels of costimulatory receptors LAIR1 and PD-L1.

In some embodiments, complexes comprising C1q and VWF increase the expression levels of two or more receptors selected from the group consisting of MerTK, LRP-1, SR-A1, CD14, LAIR1, and PD-L1. In some embodiments, complexes comprising C1q and VWF increase the expression levels of three or more receptors selected from the group consisting of MerTK, LRP-1, SR-A1, CD14, LAIR1, and PD-L1. In some embodiments, complexes comprising C1q and VWF increase the expression levels of four or more receptors selected from the group consisting of MerTK, LRP-1, SR-A1, CD14, LAIR1, and PD-L1. In some embodiments, complexes comprising C1q and VWF increase the expression levels of five or more receptors selected from the group consisting of MerTK, LRP-1, SR-A1, CD14, LAIR1, and PD-L1. In some embodiments, complexes comprising C1q and VWF increase the expression levels of MerTK, LRP-1, SR-A1, CD14, LAIR1, and PD-L1.

Methods for determining the expression level of a phagocytosis-mediating receptor and the expression level of a costimulatory receptor by macrophages are known to those skilled in the art and assay kits are commercially available. Non-limiting examples of detection assays include immunostaining, ELISA, FACS analysis, Western blotting, and the like. For instance, useful antibodies are commercially available from R&D Systems, Biolegend, Thermo Fisher Scientific, and the like.

In some embodiments, complexes comprising C1q and VWF decrease (reduce) secretion of pro-inflammatory cytokines by macrophages. In some embodiments, the complexes mediate secretion of one or more (*e.g.*, 1, 2, 3, 4, 5, 6, 7 or 8) pro-inflammatory cytokines selected from the group consisting of IL-1α, IL-1β, IL-6, IL-10, IL-18, IL-1RA, TNF1α, and a combination thereof. In some instances, secretion by macrophages in an atherosclerosis plaque of IL-1α is decreased. In some instances, secretion by macrophages in an atherosclerosis plaque of IL-1β is decreased.

In some instances, secretion by macrophages in an atherosclerosis plaque of IL-6 is decreased. In other instances, secretion by macrophages in an atherosclerosis plaque of IL-6 is increased. In some instances, secretion by macrophages in an atherosclerosis plaque of IL-10 is decreased. In other instances, secretion by macrophages in an atherosclerosis plaque of IL-10 is increased. In some instances, secretion by macrophages in an atherosclerosis plaque of IL-18 is decreased. In other instances, secretion by macrophages in an atherosclerosis plaque of IL-18 is increased. In some instances, secretion by macrophages in an atherosclerosis plaque of IL-1RA is decreased. In other instances, secretion by macrophages in an atherosclerosis plaque of IL-1RA is increased. In some instances, secretion by macrophages in an atherosclerosis plaque of TNF1α is decreased. In other instances, secretion by macrophages in an atherosclerosis plaque of TNF1α is increased.

In some embodiments, complexes comprising C1q and VWF decrease the ratio of IL-1β/IL-1RA. In some embodiments, complexes comprising C1q and VWF decrease the ratio of IL-1α/IL-1RA.

Methods for determining the secretion of pro-inflammatory cytokines by macrophages are known to those skilled in the art and assay kits are commercially available. Non-limiting examples of detection assays include immunostaining, ELISA, FACS analysis, Western blotting, and the like. For instance, human IL-1β ELISA kits are available from Abeam (Cat. No. ab46052), Thermo Fisher Scientific (Cat. No. KHC0011), and Cisbio (Cat. No. 62HIL1BPET). Exemplary human IL-1α ELISA kits are available from Abcam (Cat. No. ab178008), Thermo Fisher Scientific (Cat. No. BMS243-2), and Cisbio (Cat. No. 62HIL1APEG).

In some embodiments, complexes comprising C1q and VWF decrease caspase-1 activation in macrophages. In some embodiments, the complexes mediate NLRP3 inflammasome assembly in macrophages. Methods for determining caspase activity are known to those skilled in the art and assay kits are commercially available. For instance, caspase 1 assay kits are available from Abcam (Cat. No. ab39412), Promega (Cat. No. G9951), and R&D Systems (Cat. No. K111-100).

Provided herein are methods of identifying a candidate agent for modulating the level of phagocytosis by macrophages. Methods are provided for screening candidate agents for the ability to modulate phagocytosis by macrophages. In some embodiments, the methods also useful for screening candidate agents for the ability to modulate macrophage-mediated inflammatory responses.

In some embodiments, the method includes treating a population of macrophages with a complex comprising recombinant VWF, recombinant complement C1q, and cholesterol crystals (CC-C1q-VWF); treating the resulting population of macrophages with a candidate agent; and in some instances, measuring the level of phagocytosis in the population of macrophages, and comparing the level of phagocytosis to the level of phagocytosis in a control population of macrophages that have not been treated with the candidate agent.

In some instances, the method includes measuring the expression level of one or more phagocytosis-mediating receptors and/or one or more costimulatory receptors in a population of macrophages, and comparing the expression level of the one or more phagocytosis-mediating receptors and/or the one or more costimulatory receptors to the expression level of the one or more phagocytosis-mediating receptors and/or the one or more costimulatory receptors in a control population of macrophages that have not been treated with the candidate agent. In other instances, the method includes measuring the expression level of one or more phagocytosis-mediating receptors in a population of macrophages, and comparing the expression level of the one or more phagocytosis-mediating receptors to the expression level of the one or more phagocytosis-mediating receptors in a control population of macrophages that have not been treated with the candidate agent. In certain instances, the method includes measuring the expression level of one or more costimulatory receptors in a population of macrophages, and comparing the expression level of the one or more costimulatory receptors to the expression level of the one or more costimulatory receptors in a control population of macrophages that have not been treated with the candidate agent. In some embodiments, the one or more phagocytosis-mediating receptors and/or the one or more costimulatory receptors are selected from the group consisting of CD14, CD86, leukocyte-associated immunoglobulin-like receptor 1 (LAIR1), lipoprotein receptor-related protein 1 (LRP-1), tyrosine protein kinase Mer (MerTK), MHC II, programmed death ligand 1 (PD-L1), scavenger receptor A 1 (SR-A1), and a combination thereof.

Methods for assaying phagocytosis are known to those skilled in the art and are commercially available. Exemplary kits for detecting and monitoring phagocytosis are available from Abeam (Cat. No. ab211156), Thermo Fisher Scientific (Cat. No. V6694), and Cayman Chemical (Cat. No. 500290).

In some instances, the method includes measuring the expression level of pro-inflammatory cytokine secretion in a population of macrophages, and comparing the expression level of pro-inflammatory cytokine secretion to the expression level of pro-inflammatory cytokine secretion in a control population of macrophages that have not been treated with the candidate agent. In some instances, the pro-inflammatory cytokine is selected from the group consisting of IL-1α, IL-1β, IL-6, IL-10, IL-18, IL-1R, TNF1α, and a combination thereof.

In some embodiments, any one of the methods herein also includes incubating recombinant VWF, recombinant complement C1q, and cholesterol crystals to form the CC-C1q-VWF complex.

### II. Recombinant von Willebrand Factor

The present invention utilizes compositions comprising recombinant von Willebrand Factor (rVWF) for treatment of subject with atherosclerosis or at risk of having atherosclerosis.

In certain embodiments, VWF proteins of the invention may comprise a construct, for example, prepared as in WO 1986/06096 published on Oct. 23, 1986 and U.S. patent application Ser. No. 07/559,509, filed on Jul. 23, 1990, in the name of Ginsburg *et al..*

The VWF useful for the present invention includes all potential forms, including the monomeric and multimeric forms. One particularly useful form of VWF are homo-multimers of at least two VWFs (*i.e.*, at least two VWF polypeptides or at least two VWF monomers). The VWF proteins may be either a biologically active derivative, or when to be used solely as a stabilizer for FVIII the VWF may be of a form not biologically active. It should also be understood that the present invention encompasses different forms of VWF to be used in combination. For example, a composition useful for the present invention may include different multimers, different derivatives and both biologically active derivatives and derivatives not biologically active.

In primary hemostasis VWF serves as a bridge between platelets and specific components of the extracellular matrix, such as collagen. The biological activity of VWF in this process can be measured by different in vitro assays (Turecek, et al., Semin. Thromb. Hemost. 28: 149-160, 2002). The ristocetin cofactor assay is based on the agglutination of fresh or formalin-fixed platelets induced by the antibiotic ristocetin in the presence of VWF.

The degree of platelet agglutination depends on the VWF concentration and can be measured by the turbidimetric method, *e.g.*, by use of an aggregometer (Weiss, et al., J. Clin. Invest. 52: 2708-2716, 1973; Macfarlane, et al., Thromb. Diath. Haemorrh. 34: 306-308, 1975). The second method is the collagen binding assay, which is based on ELISA technology (Brown, et al., Thromb. Res. 43: 303-311, 1986; Favaloro, Thromb. Haemost. 83: 127-135, 2000). A microtiter plate is coated with type I or III collagen. Then the VWF is bound to the collagen surface and subsequently detected with an enzyme-labeled polyclonal antibody. The last step is the substrate reaction, which can be photometrically monitored with an ELISA reader. As provided herein, the specific Ristocetin Cofactor activity of the VWF (VWF:RCo) of the present invention is generally described in terms of mU/µg of VWF, as measured using in vitro assays.

An advantage of the rVWF compositions of the present invention over pdVWF is that rVWF exhibits a higher specific activity than pdVWF. In some embodiments, the rVWF of the invention has a specific activity of at least about 20, 22.5, 25, 27.5, 30, 32.5, 35, 37.5, 40, 42.5, 45, 47.5, 50, 52.5, 55, 57.5, 60, 62.5, 65, 67.5, 70, 72.5, 75, 77.5, 80, 82.5, 85, 87.5, 90, 92.5, 95, 97.5, 100, 105, 110, 115, 120, 125, 130, 135, 140, 145, 150 or more mU/µg.

The rVWF of the present invention is highly multimeric comprising about 10 to about 40 subunits. In further embodiments, the multimeric rVWF produced using methods of the present invention comprise about 10-30, 12-28, 14-26, 16-24, 18-22, or 20-21 subunits. In further embodiments, the rVWF is present in multimers varying in size from dimers to multimers of over 40 subunits (>10 million Daltons). The largest multimers provide multiple binding sites that can interact with both platelet receptors and subendothelial matrix sites of injury, and are the most hemostatically active form of VWF. Application of ADAMTS13 will cleave the ultra-large rVWF multimers over time, but during production (generally through expression in cell culture), rVWF compositions of the present invention are generally not exposed to ADAMTS13 and retain their highly multimeric structure.

In one embodiment, a rVWF composition used in the methods described herein has a distribution of rVWF oligomers characterized in that 95% of the oligomers have between 6 subunits and 20 subunits. In other embodiments, the a rVWF composition has a distribution of rVWF oligomers characterized in that 95% of the oligomers have a range of subunits selected from variations 458 to 641 found in Table 2 of WO 2012/171031.

In one embodiment, a rVWF composition can be characterized according to the percentage of rVWF molecules that are present in a particular higher order rVWF multimer or larger multimer. For example, in one embodiment, at least 20% of rVWF molecules in a rVWF composition used in the methods described herein are present in an oligomeric complex of at least 10 subunits. In another embodiment, at least 20% of rVWF molecules in a rVWF composition used in the methods described herein are present in an oligomeric complex of at least 12 subunits. In yet other embodiments, a rVWF composition used in the methods provided herein has a minimal percentage (*e.g*., has at least X %) of rVWF molecules present in a particular higher-order rVWF multimer or larger multimer (*e.g*., a multimer of at least Y subunits) according to any one of variations 134 to 457 found in Table 3 to Table 5, which is herein incorporated by reference in its entirety for all purposes.

In accordance with the above, the rVWF composition administered to the subject (with or without FVIII) generally comprises a significant percentage of high molecular weight (HMW) rVWF multimers. In further embodiments, the HMW rVWF multimer composition comprises at least 10%-80% rVWF decamers or higher order multimers. In further embodiments, the composition comprises about 10-95%, 20-90%, 30-85%, 40-80%, 50-75%, or 60-70% decamers or higher order multimers. In further embodiments, the HMW rVWF multimer composition comprises at least about 10%, 20%, 30%, 40%, 50%, 60%, 70%, 80%, or 90% decamers or higher order multimers.

Assessment of the number and percentage of rVWF multimers can be conducted using methods known in the art, including without limitation methods using electrophoresis and size exclusion chromatography methods to separate VWF multimers by size, for example as discussed by Cumming, et al., (J Clin Pathol. 1993 May; 46(5): 470-473 .
Such techniques may further include immunoblotting techniques (such as Western Blot), in which the gel is immunoblotted with a radiolabeled antibody against VWF followed by chemiluminescent detection (see for example Wen, et al., (1993), J. Clin. Lab. Anal., 7: 317-323 .
Further assays for VWF include VWF:Antigen (VWF:Ag), VWF:Ristocetin Cofactor (VWF:RCof), and VWF:Collagen Binding Activity assay (VWF:CBA), which are often used for diagnosis and classification of Von Willebrand Disease. (see for example Favaloro, et al., Pathology, 1997, 29(4): 341-456 .

In further embodiments, higher order rVWF multimers of the invention are stable for about 1 to about 90 hours post-administration. In still further embodiments, the higher order rVWF multimers are stable for about 5-80, 10-70, 15-60, 20-50, 25-40, or 30-35 hours post-administration. In yet further embodiments, the higher order rVWF multimers are stable for at least 3, 6, 12, 18, 24, 36, 48, or 72 hours post-administration. In certain embodiments the stability of the rVWF multimers is assessed in vitro.

In one embodiment, higher order rVWF multimers used in the compositions and methods provided herein have a half-life of at least 12 hours post administration. In another embodiment, the higher order rVWF multimers have a half-life of at least 24 hours post administration. In yet other embodiments, the higher order rVWF multimers have a half-life selected from variations 642 to 1045 found in Table 6 of WO 2012/171031.

In specific aspects, the rVWF (plasma-free or recombinant form, including the pro-rVWF and/or purified rVWF purified in accordance with the present invention) used in accordance with the present invention is not modified with any conjugation, post-translation or covalent modifications. In particular embodiments, the rVWF of the present invention is not modified with a water soluble polymer, including without limitation, a polyethylene glycol (PEG), a polypropylene glycol, a polyoxyalkylene, a polysialic acid, hydroxyl ethyl starch, a poly-carbohydrate moiety, and the like. In some embodiments, a plasma-free VWF used in accordance with the present invention is not modified with any conjugation, post-translation or covalent modifications.

In other aspects, the rVWF (plasma-free or recombinant form, including the pro-rVWF and/or purified rVWF purified in accordance with the present invention) used in accordance with the present invention is modified through conjugation, post-translation modification, or covalent modification, including modifications of the N- or C-terminal residues as well as modifications of selected side chains, for example, at free sulfhydryl-groups, primary amines, and hydroxyl-groups. In one embodiment, a water soluble polymer is linked to the protein (directly or via a linker) by a lysine group or other primary amine. In one embodiment, the rVWF proteins of the present invention may be modified by conjugation of a water soluble polymer, including without limitation, a polyethylene glycol (PEG), a polypropylene glycol, a polyoxyalkylene, a polysialic acid, hydroxyl ethyl starch, a poly-carbohydrate moiety, and the like.

Water soluble polymers that may be used to modify the rVWF and/or FVIII include linear and branched structures. The conjugated polymers may be attached directly to the coagulation proteins of the invention, or alternatively may be attached through a linking moiety. Non-limiting examples of protein conjugation with water soluble polymers can be found in U.S. Pat. Nos. 4,640,835; 4,496,689; 4,301,144; 4,670,417; 4,791,192, and 4,179,337, as well as in Abuchowski and Davis "Enzymes as Drugs," Holcenberg and Roberts, Eds., pp. 367 383, John Wiley and Sons, New York (1981), and Hermanson G., Bioconjugate Techniques 2nd Ed., Academic Press, Inc. 2008.

Protein conjugation may be performed by a number of well-known techniques in the art, for example, see Hermanson G., Bioconjugate Techniques 2nd Ed., Academic Press, Inc. 2008. Examples include linkage through the peptide bond between a carboxyl group on one of either the coagulation protein or water-soluble polymer moiety and an amine group of the other, or an ester linkage between a carboxyl group of one and a hydroxyl group of the other. Another linkage by which a coagulation protein of the invention could be conjugated to a water-soluble polymer compound is via a Schiff base, for example, formed by a free amino group on the polymer moiety being reacted with an aldehyde group formed at the nonreducing end of the protein by periodate oxidation (Jennings and Lugowski, J. Immunol. 1981; 127:1011-8; Femandes and Gregonradis, Biochim Biophys Acta. 1997; 1341; 26-34). The generated Schiff Base can be stabilized by specific reduction with NaCNBH₃ to form a secondary amine. An alternative approach is the generation of terminal free amino groups on the polymer by reductive amination with NH₄Cl after prior oxidation. Bifunctional reagents can be used for linking two amino or two hydroxyl groups. For example, a polymer containing an amino group can be coupled to an amino group of the coagulation protein with reagents like BS3 (Bis(sulfosuccinimidyl)suberate/Pierce, Rockford, Ill.). In addition heterobifunctional cross linking reagents like Sulfo-EMCS (N-.epsilon.-Maleimidocaproyloxy) sulfosuccinimide ester/Pierce) can be used for instance to link amine and thiol groups. In other embodiments, an aldehyde reactive group, such as PEG alkoxide plus diethyl acetal of bromoacetaldehyde; PEG plus DMSO and acetic anhydride, and PEG chloride plus the phenoxide of 4-hydroxybenzaldehyde, succinimidyl active esters, activated dithiocarbonate PEG, 2,4,5-trichlorophenylcloroformate and P-nitrophenylcloroformate activated PEG, may be used in the conjugation of a coagulation protein.

In some aspects, the rVWF used in methods of the present invention has been matured in vitro with furin. In further embodiments, the furin is recombinant furin. In some embodiments, the rVWF is matured using furin and a chromatography method.

In further aspects, the rVWF used in the methods of the present invention is produced by expression in a mammalian cell culture using methods known in the art. In particular embodiments, the mammalian culture comprises CHO cells. In an exemplary embodiment, the rVWF of the invention comprises rVWF protein isolated from a CHO cell expression system. In a further embodiment, the propeptide removal is mediated in vitro through exposure of the pro-VWF to furin; in a still further embodiment, the furin used for propeptide removal is recombinant furin. In yet a further embodiment, fully glycosylated/ABO blood group glycans are absent.

In yet further embodiments, the rVWF used in methods and compositions of the present invention is produced by expression in a suitable eukaryotic host system. Examples of eukaryotic cells include, without limitation, mammalian cells, such as CHO, COS, HEK 293, BHK, SK-Hep, and HepG2; insect cells, *e.g*., SF9 cells, SF21 cells, S2 cells, and High Five cells; and yeast cells, *e.g.*, Saccharomyces or Schizosaccharomyces cells. In one embodiment, the VWF can be expressed in yeast cells, insect cells, avian cells, mammalian cells, and the like. For example, in a human cell line, a hamster cell line, or a murine cell line. In one particular embodiment, the cell line is a CHO, BHK, or HEK cell line. Typically, mammalian cells, *e.g.*, CHO cells from a continuous cell line, can be used to express the VWF of the present invention.

In certain embodiments, the nucleic acid sequence comprising a sequence coding for VWF can be a vector. The vector can be delivered by a virus or can be a plasmid. The nucleic acid sequence coding for the protein can be a specific gene or a biologically functional part thereof. In one embodiment, the protein is at least a biologically active part of VWF. A wide variety of vectors can be used for the expression of the VWF and can be selected from eukaryotic expression vectors. Examples of vectors for eukaryotic expression include: (i) for expression in yeast, vectors such as pAO, pPIC, pYES, pMET, using promoters such as AOX1, GAP, GAL1, AUG1, *etc*.; (ii) for expression in insect cells, vectors such as pMT, pAc5, pIB, pMIB, pBAC, *etc.*, using promoters such as PH, p10, MT, Ac5, OpIE2, gp64, polh, *etc*., and (iii) for expression in mammalian cells, vectors such as pSVL, pCMV, pRc/RSV, pcDNA3, pBPV, *etc*., and vectors derived from viral systems such as vaccinia virus, adeno-associated viruses, herpes viruses, retroviruses, *etc*., using promoters such as CMV, SV40, EF-1, UbC, RSV, ADV, BPV, and β-actin.

In some embodiments of the present invention, the nucleic acid sequence further comprises other sequences suitable for a controlled expression of a protein such as promoter sequences, enhancers, TATA boxes, transcription initiation sites, polylinkers, restriction sites, poly-A-sequences, protein processing sequences, selection markers, and the like which are generally known to a person of ordinary skill in the art.

In certain embodiments, the cell-culture methods of the invention may comprise the use of a microcarrier. In some embodiments, the cell-cultures of the embodiments can be performed in large bioreactors under conditions suitable for providing high volume-specific culture surface areas to achieve high cell densities and protein expression. One means for providing such growth conditions is to use microcarriers for cell-culture in stirred tank bioreactors. The concept of cell-growth on microcarriers was first described by van Wezel (van Wezel, A. L., Nature 216:64-5 (1967)) and allows for cell attachment on the surface of small solid particles suspended in the growth medium. These methods provide for high surface-to-volume ratios and thus allow for efficient nutrient utilization. Furthermore, for expression of secreted proteins in eukaryotic cell lines, the increased surface-to-volume ratio allows for higher levels of secretion and thus higher protein yields in the supernatant of the culture. Finally, these methods allow for the easy scale-up of eukaryotic expression cultures.

The cells expressing rVWF can be bound to a spherical or a porous microcarrier during cell culture growth. The microcarrier can be a microcarrier selected from the group of microcarriers based on dextran, collagen, plastic, gelatine and cellulose and others as described in Butler (In: Spier & Griffiths, Animal Cell Biotechnology 3:283-303 (1988)). It is also possible to grow the cells to a biomass on spherical microcarriers and subculture the cells when they have reached final fermenter biomass and prior to production of the expressed protein on a porous microcarrier or vice versa. Suitable spherical microcarriers can include smooth surface microcarriers, such as Cytodex^{™} 1, Cytodex^{™} 2, and Cytode^{™} 3 (GE Healthcare) and macroporous microcarriers such as Cytopore^{™} 1, Cytopore^{™} 2, Cytoline^{™} 1, and Cytoline^{™} 2 (GE Healthcare).

In certain embodiments, rVWF is expressed in cells cultured in cell culture media that produces high molecular weight rVWF. The terms "cell culture solution," "cell culture medium or media," and "cell culture supernatant" refer to aspects of cell culture processes generally well known in the art. In the context of the present invention, a cell culture solution can include cell culture media and cell culture supernatant. The cell culture media are externally added to the cell culture solution, optionally together with supplements, to provide nutrients and other components for culturing the cells expressing VWF. The cell culture supernatant refers to a cell culture solution comprising the nutrients and other components from the cell culture medium as well as products released, metabolized, and/or excreted from the cells during culture. In further embodiments, the media can be animal protein-free and chemically defined. Methods of preparing animal protein-free and chemically defined culture media are known in the art, for example in US 2008/0009040 and US 2007/0212770 .

"Protein free" and related terms refers to protein that is from a source exogenous to or other than the cells in the culture, which naturally shed proteins during growth. In another embodiment, the culture medium is polypeptide free. In another embodiment, the culture medium is serum free. In another embodiment the culture medium is animal protein free. In another embodiment the culture medium is animal component free. In another embodiment, the culture medium contains protein, *e.g*., animal protein from serum such as fetal calf serum. In another embodiment, the culture has recombinant proteins exogenously added. In another embodiment, the proteins are from a certified pathogen free animal. The term "chemically defined" as used herein shall mean, that the medium does not comprise any undefined supplements, such as, for example, extracts of animal components, organs, glands, plants, or yeast. Accordingly, each component of a chemically defined medium is accurately defined. In a preferred embodiment, the media are animal-component free and protein free.

In further embodiments, subsequent to purification from a mammalian cell culture, rFVIII is reconstituted prior to administration. In still further embodiments, the rVWF is treated with furin prior to or subsequent to reconstitution. In further embodiments, the furin is recombinant furin. In still further embodiments, the rVWF of the invention is not exposed to ADAMTS13, with the result that ultra large (*i.e.*, comprising 10 or more subunits) are present in rVWF compositions of the invention.

In specific aspects, the rVWF used in methods of the present invention is contained in a formulation containing a buffer, a sugar and/or a sugar alcohol (including without limitation trehalose and mannitol), a stabilizer (such as glycine), and a surfactant (such as polysorbate 80). In further embodiments, for formulations containing rFVIII, the formulation may further include sodium, histidine, calcium, and glutathione.

In one aspect, the formulations comprising rVWF is lyophilized prior to administration. Lyophilization is carried out using techniques common in the art and should be optimized for the composition being developed (Tang et al., Pharm Res. 21:191-200. (2004) and Chang et al., Pharm Res. 13:243-9 (1996)).

The present method also provides formulations of rVWF for use in the treatment methods provided herein. In some embodiments, the rVWF composition is used for the production of a pharmaceutical composition. In some embodiments, the rVWF can be formulated into a lyophilized formulation.

In some embodiments, the formulations comprising a VWF polypeptide of the invention are lyophilized after purification and prior to administration to a subject. Lyophilization is carried out using techniques common in the art and should be optimized for the composition being developed (Tang et al., Pharm Res. 21:191-200, (2004) and Chang et al., Pharm Res. 13:243-9 (1996)).

A lyophilization cycle is, in one aspect, composed of three steps: freezing, primary drying, and secondary drying (A. P. Mackenzie, Phil Trans R Soc London, Ser B, Biol 278:167 (1977)). In the freezing step, the solution is cooled to initiate ice formation. Furthermore, this step induces the crystallization of the bulking agent. The ice sublimes in the primary drying stage, which is conducted by reducing chamber pressure below the vapor pressure of the ice, using a vacuum and introducing heat to promote sublimation. Finally, adsorbed or bound water is removed at the secondary drying stage under reduced chamber pressure and at an elevated shelf temperature. The process produces a material known as a lyophilized cake. Thereafter the cake can be reconstituted with either sterile water or suitable diluent for injection.

The lyophilization cycle not only determines the final physical state of excipients but also affects other parameters such as reconstitution time, appearance, stability and final moisture content. The composition structure in the frozen state proceeds through several transitions (*e.g*., glass transitions, wettings, and crystallizations) that occur at specific temperatures and the structure may be used to understand and optimize the lyophilization process. The glass transition temperature (Tg and/or Tg') can provide information about the physical state of a solute and can be determined by differential scanning calorimetry (DSC). Tg and Tg' are an important parameter that must be taken into account when designing the lyophilization cycle. For example, Tg' is important for primary drying. Furthermore, in the dried state, the glass transition temperature provides information on the storage temperature of the final product.

Methods of preparing pharmaceutical formulations can include one or more of the following steps: adding a stabilizing agent as described herein to said mixture prior to lyophilizing, adding at least one agent selected from a bulking agent, an osmolarity regulating agent, and a surfactant, each of which as described herein, to said mixture prior to lyophilization. A lyophilized formulation is, in one aspect, at least comprised of one or more of a buffer, a bulking agent, and a stabilizer. In this aspect, the utility of a surfactant is evaluated and selected in cases where aggregation during the lyophilization step or during reconstitution becomes an issue. An appropriate buffering agent is included to maintain the formulation within stable zones of pH during lyophilization.

The standard reconstitution practice for lyophilized material is to add back a volume of pure water or sterile water for injection (WFI) (typically equivalent to the volume removed during lyophilization), although dilute solutions of antibacterial agents are sometimes used in the production of pharmaceuticals for parenteral administration (Chen, Drug Development and Industrial Pharmacy, 18:1311-1354 (1992)). Accordingly, methods are provided for preparation of reconstituted recombinant VWF compositions comprising the step of adding a diluent to a lyophilized recombinant VWF composition of the invention.

The lyophilized material may be reconstituted as an aqueous solution. A variety of aqueous carriers, *e.g*., sterile water for injection, water with preservatives for multi dose use, or water with appropriate amounts of surfactants (for example, an aqueous suspension that contains the active compound in admixture with excipients suitable for the manufacture of aqueous suspensions). In various aspects, such excipients are suspending agents, for example and without limitation, sodium carboxymethylcellulose, methylcellulose, hydroxypropylmethylcellulose, sodium alginate, polyvinylpyrrolidone, gum tragacanth and gum acacia; dispersing or wetting agents are a naturally-occurring phosphatide, for example and without limitation, lecithin, or condensation products of an alkylene oxide with fatty acids, for example and without limitation, polyoxyethylene stearate, or condensation products of ethylene oxide with long chain aliphatic alcohols, for example and without limitation, heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example and without limitation, polyoxyethylene sorbitan monooleate. In various aspects, the aqueous suspensions also contain one or more preservatives, for example and without limitation, ethyl, or n-propyl, p-hydroxybenzoate.

In certain embodiments, compositions of the present invention are liquid formulations for administration with the use of a syringe or other storage vessel. In further embodiments, these liquid formulations are produced from lyophilized material described herein reconstituted as an aqueous solution.

In a further aspect, the compositions of the invention further comprise one or more pharmaceutically acceptable carriers. The phrases "pharmaceutically" or "pharmacologically" acceptable refer to molecular entities and compositions that are stable, inhibit protein degradation such as aggregation and cleavage products, and in addition do not produce allergic, or other adverse reactions when administered using routes well-known in the art, as described below. "Pharmaceutically acceptable carriers" include any and all clinically useful solvents, dispersion media, coatings, antibacterial and antifungal agents, isotonic and absorption delaying agents and the like, including those agents disclosed above.

### III. Production of Recombinant VWF

The free mature recombinant von Willebrand Factor (rVWF) of the present invention can be produced recombinantly. One skilled in the art recognizes useful methods for expressing a recombinant protein in a host cell. In some instances, the method includes expressing a nucleic acid sequence encoding rVWF in a host cell such as a CHO cell and culturing the resulting host cell under certain conditions to produce rVWF, prepro-VWF, pro-VWF, and the like.

In certain embodiments, the culture of cells expressing rVWF can be maintained for at least about 7 days, or at least about 14 days, 21 days, 28 days, or at least about 5 weeks, 6 weeks, 7 weeks, or at least about 2 months, or 3, 4, 5, 6, 7, 8, 9, 10, 11, 12, 13, 14, 15, 16, 17, or 18 months or longer. The cell density at which a cell-culture is maintained at for production of a recombinant VWF protein will depend upon the culture-conditions and medium used for protein expression. One of skill in the art will readily be able to determine the optimal cell density for a cell-culture producing a rVWF. In one embodiment, the culture is maintained at a cell density of between about 0.5×10⁶ and 4×10⁷ cells/ml for an extended period of time. In other embodiments, the cell density is maintained at a concentration of between about 1.0×10⁶ and about 1.0×10⁷ cells/ml for an extended period of time. In other embodiments, the cell density is maintained at a concentration of between about 1.0×10⁶ and about 4.0×10⁶ cells/ml for an extended period of time. In other embodiments, the cell density is maintained at a concentration of between about 1.0×10⁶ and about 4.0×10⁶ cells/ml for an extended period of time. In yet other embodiments, the cell density may be maintained at a concentration between about 2.0×10⁶ and about 4.0×10⁶, or between about 1.0×10⁶ and about 2.5×10⁶, or between about 1.5×10⁶ and about 3.5×10⁶, or any other similar range, for an extended period of time. After an appropriate time in cell culture, the rVWF can be isolated from the expression system using methods known in the art.

In a specific embodiment, the cell density of the continuous cell culture for production of rVWF is maintained at a concentration of no more than 2.5×10⁶ cells/mL for an extended period. In other specific embodiments, the cell density is maintained at no more than 2.0×10⁶ cells/mL, 1.5×10⁶ cells/mL, 1.0×10⁶ cells/mL, 0.5×10⁶ cells/mL, or less. In one embodiment, the cell density is maintained at between 1.5×10⁶ cells/mL and 2.5×10⁶ cells/mL.

In one embodiment of the cell cultures described above, the cell culture solution comprises a medium supplement comprising copper. Such cell culture solutions are described, for example, in U.S. Patent No. 8,852,888 and U.S. Patent No. 9,409,971.

The polynucleotide and amino acid sequences of prepro-VWF are set out in SEQ ID NO:1 and SEQ ID NO:2, respectively, and are available at GenBank Accession Nos. NM_000552 (Homo sapiens von Willebrand factor (VWF) mRNA) and NP_000543, respectively. The amino acid sequence corresponding to the mature VWF protein is set out in SEQ ID NO: 3 (corresponding to amino acids 764-2813 of the full length prepro-VWF amino acid sequence). In some embodiments, the VWF exhibits at least 80%, at least 85%, at least 90%, at least 95%, at least 96%, at least 97%, at least 98%, at least 99%, or at least 100% identity to the sequence of SEQ ID NO:3. In some embodiments, the rVWF of the invention exhibits at least 80%, at least 85%, at least 90%, at least 95%, at least 96%, at least 97%, at least 98%, at least 99%, or at least 100% identity to the sequence of SEQ ID NO:3. *See*, for example, U.S. Patent No. 8,597,910, U.S. Patent Publication No. 2016/0129090, as well as FIG. 6.

One form of useful rVWF has at least the property of in vivo-stabilizing, e.g. binding, of at least one Factor VIII (FVIII) molecule and having optionally a glycosylation pattern which is pharmacologically acceptable. Specific examples thereof include VWF without the A2 domain thus resistant to proteolysis (Lankhof et al., Thromb. Haemost. 77: 1008-1013, 1997), and a VWF fragment from Val 449 to Asn 730 including the glycoprotein lb-binding domain and binding sites for collagen and heparin (Pietu et al., Biochem. Biophys. Res. Commun. 164: 1339-1347, 1989). The determination of the ability of a VWF to stabilize at least one FVIII molecule is, in one aspect, carried out in VWF-deficient mammals according to methods known in the state in the art.

The rVWF of the present invention can be produced by any method known in the art. One specific example is disclosed in WO86/06096 published on Oct. 23, 1986 and U.S. Patent Application No. 07/559,509, filed on Jul. 23, 1990 . Thus, methods are known in the art for (i) the production of recombinant DNA by genetic engineering, e.g. via reverse transcription of RNA and/or amplification of DNA, (ii) introducing recombinant DNA into prokaryotic or eukaryotic cells by transfection, *e.g*. via electroporation or microinjection, (iii) cultivating the transformed cells, *e.g.* in a continuous or batchwise manner, (iv) expressing VWF, *e.g*. constitutively or upon induction, and (v) isolating the VWF, *e.g*. from the culture medium or by harvesting the transformed cells, in order to (vi) obtain purified rVWF, *e.g*. via anion exchange chromatography or affinity chromatography. A recombinant VWF is, in one aspect, made in transformed host cells using recombinant DNA techniques well known in the art. For instance, sequences coding for the polypeptide could be excised from DNA using suitable restriction enzymes. Alternatively, the DNA molecule is, in another aspect, synthesized using chemical synthesis techniques, such as the phosphoramidate method. Also, in still another aspect, a combination of these techniques is used.

The invention also provides vectors encoding polypeptides of the invention in an appropriate host. The vector comprises the polynucleotide that encodes the polypeptide operatively linked to appropriate expression control sequences. Methods of effecting this operative linking, either before or after the polynucleotide is inserted into the vector, are well known. Expression control sequences include promoters, activators, enhancers, operators, ribosomal binding sites, start signals, stop signals, cap signals, polyadenylation signals, and other signals involved with the control of transcription or translation. The resulting vector having the polynucleotide therein is used to transform an appropriate host. This transformation may be performed using methods well known in the art.

Any of a large number of available and well-known host cells are used in the practice of this invention. The selection of a particular host is dependent upon a number of factors recognized by the art, including, for example, compatibility with the chosen expression vector, toxicity of the peptides encoded by the DNA molecule, rate of transformation, ease of recovery of the peptides, expression characteristics, bio-safety and costs. A balance of these factors must be struck with the understanding that not all host cells are equally effective for the expression of a particular DNA sequence. Within these general guidelines, useful microbial host cells include, without limitation, bacteria, yeast and other fungi, insects, plants, mammalian (including human) cells in culture, or other hosts known in the art.

Transformed host cells are cultured under conventional fermentation conditions so that the desired compounds are expressed. Such fermentation conditions are well known in the art. Finally, the polypeptides are purified from culture media or the host cells themselves by methods well known in the art.

Depending on the host cell utilized to express a compound of the invention, carbohydrate (oligosaccharide) groups are optionally attached to sites that are known to be glycosylation sites in proteins. Generally, O-linked oligosaccharides are attached to serine (Ser) or threonine (Thr) residues while N-linked oligosaccharides are attached to asparagine (Asn) residues when they are part of the sequence Asn-X-Ser/Thr, where X can be any amino acid except proline. X is preferably one of the 19 naturally occurring amino acids not counting proline. The structures of N-linked and O-linked oligosaccharides and the sugar residues found in each type are different. One type of sugar that is commonly found on both N-linked and O-linked oligosaccharides is N-acetylneuraminic acid (referred to as sialic acid). Sialic acid is usually the terminal residue of both N-linked and O-linked oligosaccharides and, by virtue of its negative charge, in one aspect, confers acidic properties to the glycosylated compound. Such site(s) may be incorporated in the linker of the compounds of this invention and are preferably glycosylated by a cell during recombinant production of the polypeptide compounds (*e.g.*, in mammalian cells such as CHO, BHK, COS). In other aspects, such sites are glycosylated by synthetic or semi- synthetic procedures known in the art.

In some embodiments, sialysation (also referred to as sialylation), can be performed on the column as part of the purification procedures described herein (including the anion exchange, cation exchange, size exclusion, and/or immunoaffinity methods). In some embodiments, the sialylation results in increased stability of the rVWF as compared to rVWF that has not undergone sialylation. In some embodiments, the sialylation results in increased stability of the rVWF in blood circulation (for example, after administration to a subject) as compared to rVWF that has not undergone sialylation. In some embodiments, the increased stability of sialylated rVWF results in an increase of 10%, 20%, 30%, 40%, 50%, 60%, 70%, 80%, 90%, or more as compared rVWF that has not undergone sialylation. In some embodiments, the sialylation results in increased half-life for the rVWF as compared to rVWF that has not undergone sialylation. In some embodiments, the sialylation results in increased half-life for the rVWF in blood circulation (for example, after administration to a subject) as compared to rVWF that has not undergone sialylation. In some embodiments, the increased half-life of sialylated rVWF results in an increase of 10%, 20%, 30%, 40%, 50%, 60%, 70%, 80%, 90%, or more as compared rVWF that has not undergone sialylation. In some embodiments, the increased half-life of sialylated rVWF results in rVWF that is stable for 1 hour, 2 hours, 3 hours, 4 hours, 6 hours, 12 hours, 24 hours or more in blood circulation (for example, after administration to a subject) as compared to rVWF that has not undergone sialylation.

In some embodiments, sialylation increases the number of 2,3 sialylation and/or 2,6 sialylation. In some embodiments, sialylation is increased by the addition of 2,3 sialyltransferase and/or 2,6 sialyltransferase and CMP-NANA (Cytidine-5'-monophospho-N-acetylneuraminic acid sodium salt) as an additional buffer step. In some embodiments, sialylation is increased by the addition of 2,3 sialyltransferase and CMP-NANA (Cytidine-5'-monophospho-N-acetylneuraminic acid sodium salt) as an additional buffer step. In some embodiments, 2,6 sialylation is increased by the addition of 2,6 sialyltransferase and CMP-NANA (Cytidine-5'-monophospho-N-acetylneuraminic acid sodium salt) as an additional buffer step.

In some embodiments, CMP-NANA is chemically or enzymatically modified to transfer modified sialic acid to potential free position. In some embodiments, sialylation is performed by loading rVWF onto the resin, washing with one or more buffers as described herein to deplete unwanted impurities, applying one or more buffers containing sialyltransferase and CMP-NANA at conditions that allow additional sialylation, and washing with one or more buffers to deplete excess of the sialylation reagents, and eluting with one or more buffers the enhanced rVWF (*e.g*., the rVWF with increased sialylation). In some embodiments, the sialylation process is performed as part of a cation exchange method, an anion exchange method, a size exclusion method, or an immunoaffinity purification method, as described herein.

Alternatively, the compounds are made by synthetic methods using, for example, solid phase synthesis techniques. Suitable techniques are well known in the art, and include those described in Merrifield (1973), Chem. Polypeptides, pp. 335-61 (Katsoyannis and Panayotis eds.); Merrifield (1963), J. Am. Chem. Soc. 85: 2149; Davis et al. (1985), Biochem. Intl. 10: 394-414; Stewart and Young (1969), Solid Phase Peptide Synthesis; U.S. Pat. No. 3,941,763; Finn et al. (1976), The Proteins (3rd ed.) 2: 105-253; and Erickson et al. (1976), The Proteins (3rd ed.) 2: 257-527 '. Solid phase synthesis is the preferred technique of making individual peptides since it is the most cost-effective method of making small peptides

Fragments, variants and analogs of VWF, whereby these are not part of the present invention, can be produced according to methods that are well-known in the art. Fragments of a polypeptide can be prepared using, without limitation, enzymatic cleavage (*e.g*., trypsin, chymotrypsin) and also using recombinant means to generate a polypeptide fragments having a specific amino acid sequence. Polypeptide fragments may be generated comprising a region of the protein having a particular activity, such as a multimerization domain or any other identifiable VWF domain known in the art.

Methods of making polypeptide analogs are also well-known. Amino acid sequence analogs of a polypeptide can be substitutional, insertional, addition or deletion analogs. Deletion analogs, including fragments of a polypeptide, lack one or more residues of the native protein which are not essential for function or immunogenic activity. Insertional analogs involve the addition of, *e.g.*, amino acid(s) at a non-terminal point in the polypeptide. This analog may include, for example and without limitation, insertion of an immunoreactive epitope or simply a single residue. Addition analogs, including fragments of a polypeptide, include the addition of one or more amino acids at either or both termini of a protein and include, for example, fusion proteins. Combinations of the aforementioned analogs are also contemplated.

Substitutional analogs typically exchange one amino acid of the wild-type for another at one or more sites within the protein, and may be designed to modulate one or more properties of the polypeptide without the complete loss of other functions or properties. In one aspect, substitutions are conservative substitutions. "Conservative amino acid substitution" is substitution of an amino acid with an amino acid having a side chain or a similar chemical character. Similar amino acids for making conservative substitutions include those having an acidic side chain (glutamic acid, aspartic acid); a basic side chain (arginine, lysine, histidine); a polar amide side chain (glutamine, asparagine); a hydrophobic, aliphatic side chain (leucine, isoleucine, valine, alanine, glycine); an aromatic side chain (phenylalanine, tryptophan, tyrosine); a small side chain (glycine, alanine, serine, threonine, methionine); or an aliphatic hydroxyl side chain (serine, threonine).

In one aspect, analogs are substantially homologous or substantially identical to the recombinant VWF from which they are derived. Analogs include those which retain at least some of the biological activity of the wild-type polypeptide, *e.g*. blood clotting activity.

Polypeptide variants contemplated- which are not part of the present invention - include, without imitation, polypeptides chemically modified by such techniques as ubiquitination, glycosylation, including polysialation (or polysialylation), conjugation to therapeutic or diagnostic agents, labeling, covalent polymer attachment such as pegylation (derivatization with polyethylene glycol), introduction of non-hydrolyzable bonds, and insertion or substitution by chemical synthesis of amino acids such as ornithine, which do not normally occur in human proteins. Variants retain the same or essentially the same binding properties of non-modified molecules of the invention. Such chemical modification may include direct or indirect (*e.g.*, via a linker) attachment of an agent to the VWF polypeptide. In the case of indirect attachment, it is contemplated that the linker may be hydrolyzable or non-hydrolyzable.

Preparing pegylated polypeptide analogs will in one aspect comprise the steps of (a) reacting the polypeptide with polyethylene glycol (such as a reactive ester or aldehyde derivative of PEG) under conditions whereby the binding construct polypeptide becomes attached to one or more PEG groups, and (b) obtaining the reaction product(s). In general, the optimal reaction conditions for the acylation reactions are determined based on known parameters and the desired result. For example, the larger the ratio of PEG: protein, the greater the percentage of poly-pegylated product. In some embodiments, the binding construct has a single PEG moiety at the N-terminus. Polyethylene glycol (PEG) may be attached to the blood clotting factor to, for example, provide a longer half-life in vivo. The PEG group may be of any convenient molecular weight and is linear or branched. The average molecular weight of the PEG ranges from about 2 kiloDalton ("kD") to about 100 kDa, from about 5 kDa to about 50 kDa, or from about 5 kDa to about 10 kDa. In certain aspects, the PEG groups are attached to the blood clotting factor via acylation or reductive alkylation through a natural or engineered reactive group on the PEG moiety (*e.g*., an aldehyde, amino, thiol, or ester group) to a reactive group on the blood clotting factor (*e*.*g*., an aldehyde, amino, or ester group) or by any other technique known in the art.

Methods for preparing polysialylated polypeptide are described in United States Patent Publication 20060160948, Fernandes, et al*.*, Gregoriadis; Biochim. Biophys. Acta 1341: 26-34, 1997, and Saenko et al., Haemophilia 12:42-51, 2006. Briefly, a solution of colominic acid (CA) containing 0.1 M NaIO₄ is stirred in the dark at room temperature to oxidize the CA. The activated CA solution is dialyzed against, *e.g*., 0.05 M sodium phosphate buffer, pH 7.2 in the dark and this solution was added to a rVWF solution and incubated for 18 h at room temperature in the dark under gentle shaking. Free reagents are optionally be separated from the rVWF-polysialic acid conjugate by, for example, ultrafiltration/diafiltration. Conjugation of rVWF with polysialic acid is achieved using glutaraldehyde as cross-linking reagent (Migneault et al., Biotechniques 37: 790-796, 2004).

It is also contemplated in another aspect that prepro-VWF and pro-VWF polypeptides will provide a therapeutic benefit in the formulations of the present invention. For example, US Patent No. 7,005,502 describes a pharmaceutical preparation comprising substantial amounts of pro-VWF that induces thrombin generation in vitro.

Polynucleotides encoding fragments, variants and analogs which are not part of the present invention may be readily
generated by a worker of skill to encode biologically active fragments, variants, or analogs of the naturally-occurring molecule that possess the same or similar biological activity to the naturally-occurring molecule. In various aspects, these polynucleotides are prepared using PCR techniques, digestion/ligation of DNA encoding molecule, and the like. Thus, one of skill in the art will be able to generate single base changes in the DNA strand to result in an altered codon and a missense mutation, using any method known in the art, including, but not limited to site- specific mutagenesis. As used herein, the phrase "moderately stringent hybridization conditions" means, for example, hybridization at 42°C in 50% formamide and washing at 60°C in 0.1 x SSC, 0.1% SDS. It is understood by those of skill in the art that variation in these conditions occurs based on the length and GC nucleotide base content of the sequences to be hybridized. Formulas standard in the art are appropriate for determining exact hybridization conditions. See Sambrook et al., 9.47-9.51 in Molecular Cloning, Cold Spring Harbor Laboratory Press, Cold Spring Harbor, New York (1989).

### A. VWF Multimers

Assessment of the number and percentage of rVWF multimers can be conducted using methods known in the art, including without limitation methods using electrophoresis and size exclusion chromatography methods to separate VWF multimers by size, for example as discussed by Cumming, et al., (J Clin Pathol., 1993 May; 46(5): 470-473 .
Such techniques may further include immunoblotting techniques (such as Western Blot), in which the gel is immunoblotted with a radiolabelled antibody against VWF followed by chemiluminescent detection (see, for example, Wen et al., J. Clin. Lab. Anal., 1993, 7: 317-323.
Further assays for VWF include VWF:Antigen (VWF:Ag), VWF:Ristocetin Cofactor (VWF:RCof), and VWF:Collagen Binding Activity assay (VWF:CBA), which are often used for diagnosis and classification of Von Willebrand Disease (see, for example, Favaloro, et al., Pathology, 1997, 29(4): 341-456; Sadler, JE, Annu Rev Biochem, 1998, 67:395-424; and Turecek, et al., Semin Thromb Hemost, 2010, 36:510-521.
In some embodiments, the rVWF obtained using the present methods includes any multimer pattern present in the loading sample of the rVWF. In some embodiments, the rVWF obtained using the present methods includes physiological occurring multimer patters as well as ultralarge VWF-multimer patterns.

### b. VWF Assays

In primary hemostasis VWF serves as a bridge between platelets and specific components of the extracellular matrix, such as collagen. The biological activity of VWF in this process can be measured by different in vitro assays (Turecek et al., Semin Thromb Hemost, 2010, 36: 510-521).

The VWF:Ristocetin Cofactor (VWF:RCof) assay is based on the agglutination of fresh or formalin-fixed platelets induced by the antibiotic ristocetin in the presence of VWF. The degree of platelet agglutination depends on the VWF concentration and can be measured by the turbidimetric method, *e.g.,* by use of an aggregometer (Weiss et al., J. Clin. Invest., 1973, 52: 2708-2716; Macfarlane et al., Thromb. Diath. Haemorrh., 1975, 34: 306-308). As provided herein, the specific ristocetin cofactor activity of the VWF (VWF:RCo) of the present invention is generally described in terms of mU/µg of VWF, as measured using in vitro assays.

In some embodiments, the rVWF purified according to the methods of the present invention has a specific activity of at least about 20, 22.5, 25, 27,5, 30, 32.5, 35, 37.5, 40, 42.5, 45, 47.5, 50, 52.5, 55, 57.5, 60, 62.5, 65, 67.5, 70, 72.5, 75, 77.5, 80, 82.5, 85, 87.5, 90, 92.5, 95, 97.5, 100, 105, 110, 1 15, 120, 125, 130, 135, 140, 145, 150 or more mU/µg. In some embodiments, rVWF used in the methods described herein has a specific activity of from 20 mU/µg to 150 mU /µg. In some embodiments, the rVWF has a specific activity of from 30 mU/µg to 120 mU/µg. In some embodiments, the rVWF has a specific activity from 40 mU/µg to 90 mU/µg. In some embodiments, the rVWF has a specific activity selected from variations 1 to 133 found in Table 3, below.
Table 3. Exemplary embodiments for the specific activity of rVWF found in the compositions and used in the methods provided herein.

| (mU/µg) | | (mU/µg) | | (mU/µg) | | (mU/µg) | |
|---|---|---|---|---|---|---|---|
| 20 | Var. 1 | 110 | Var. 35 | 40-150 | Var. 68 | 70-120 | Var. 101 |
| 22.5 | Var. 2 | 115 | Var. 36 | 40-140 | Var. 69 | 70-110 | Var. 102 |
| 25 | Var. 3 | 120 | Var. 37 | 40-130 | Var. 70 | 70-100 | Var. 103 |
| 27.5 | Var. 4 | 125 | Var. 38 | 40-120 | Var. 71 | 70-90 | Var. 104 |
| 30 | Var. 5 | 130 | Var. 39 | 40-110 | Var. 72 | 70-80 | Var. 105 |
| 32.5 | Var. 6 | 135 | Var. 40 | 40-100 | Var. 73 | 80-150 | Var. 106 |
| 35 | Var. 7 | 140 | Var. 41 | 40-90 | Var. 74 | 80-140 | Var. 107 |
| 37.5 | Var. 8 | 145 | Var. 42 | 40-80 | Var. 75 | 80-130 | Var. 108 |
| 40 | Var. 9 | 150 | Var. 43 | 40-70 | Var. 76 | 80-120 | Var. 109 |
| 42.5 | Var. 10 | 20-150 | Var. 44 | 40-60 | Var. 77 | 80-110 | Var. 110 |
| 45 | Var. 11 | 20-140 | Var. 45 | 40-50 | Var. 78 | 80-100 | Var. 111 |
| 47.5 | Var. 12 | 20-130 | Var. 46 | 50-150 | Var. 79 | 80-90 | Var. 112 |
| 50 | Var. 13 | 20-120 | Var. 47 | 50-140 | Var. 80 | 90-150 | Var. 113 |
| 52.5 | Var. 14 | 20-110 | Var. 48 | 50-130 | Var. 81 | 90-140 | Var. 114 |
| 55 | Var. 15 | 20-100 | Var. 49 | 50-120 | Var. 82 | 90-130 | Var. 115 |
| 57.5 | Var. 16 | 20-90 | Var. 50 | 50-110 | Var. 83 | 90-120 | Var. 116 |
| 60 | Var. 17 | 20-80 | Var. 51 | 50-100 | Var. 84 | 90-110 | Var. 117 |
| 62.5 | Var. 18 | 20-70 | Var. 52 | 50-90 | Var. 85 | 90-100 | Var. 118 |
| 65 | Var. 19 | 20-60 | Var. 53 | 50-80 | Var. 86 | 100-150 | Var. 119 |
| 67.5 | Var. 20 | 20-50 | Var. 54 | 50-70 | Var. 87 | 100-140 | Var. 120 |
| 70 | Var. 21 | 20-40 | Var. 55 | 50-60 | Var. 88 | 100-130 | Var. 121 |
| 72.5 | Var. 22 | 30-150 | Var. 56 | 60-150 | Var. 89 | 100-120 | Var. 122 |
| 75 | Var. 23 | 30-140 | Var. 57 | 60-140 | Var. 90 | 100-110 | Var. 123 |
| 77.5 | Var. 24 | 30-130 | Var. 58 | 60-130 | Var. 91 | 110-150 | Var. 124 |
| 80 | Var. 25 | 30-120 | Var. 59 | 60-120 | Var. 92 | 110-140 | Var. 125 |
| 82.5 | Var. 26 | 30-110 | Var. 60 | 60-110 | Var. 93 | 110-130 | Var. 126 |
| 85 | Var. 27 | 30-100 | Var. 61 | 60-100 | Var. 94 | 110-120 | Var. 127 |
| 87.5 | Var. 28 | 30-90 | Var. 62 | 60-90 | Var. 95 | 120-150 | Var. 128 |
| 90 | Var. 29 | 30-80 | Var. 63 | 60-80 | Var. 96 | 120-140 | Var. 129 |
| 92.5 | Var. 30 | 30-70 | Var. 64 | 60-70 | Var. 97 | 120-130 | Var. 130 |
| 95 | Var. 31 | 30-60 | Var. 65 | 70-150 | Var. 98 | 130-150 | Var. 131 |
| 97.5 | Var. 32 | 30-50 | Var. 66 | 70-140 | Var. 99 | 130-140 | Var. 132 |
| 100 | Var. 33 | 30-40 | Var. 67 | 70-130 | Var. 100 | 140-150 | Var. 133 |
| 105 | Var. 34 | | | | | | |

| | | | | | | | |
|---|---|---|---|---|---|---|---|
| Var. = Variation | | | | | | | |

The rVWF of the present invention is highly multimeric comprising about 10 to about 40 subunits. In further embodiments, the multimeric rVWF produced using methods of the present invention comprise about 10-30, 12-28, 14-26, 16-24, 18-22, or 20-21 subunits. In some embodiments, the rVWF is present in multimers varying in size from dimers to multimers of over 40 subunits (> 10 million Daltons). The largest multimers provide multiple binding sites that can interact with both platelet receptors and subendothelial matrix sites of injury, and are the most hemostatically active form of VWF. In some embodiments, the rVWF of the present invention comprises ultralarge multimers (ULMs). Generally, high and ultralarge multimers are considered to be hemostatically most effective (*see*, for example, Turecek, P., Hämostaseologie, (Vol. 37): Supplement 1, pages S15-S25 (2017)). In some embodiments, the rVWF is between 500 kDa and 20,000 kDa. In some embodiments, any desired multimer pattern can be obtained using the methods described. In some embodiments, when anion exchange and/or cation exchanger methods are employed, the pH, conductivity, and/or counterion concentration of the buffers in the one or more wash step(s) or the gradient buffers can be manipulated to obtain the desired multimer pattern. In some embodiments, then size exclusion chromatography methods are employed, the collection criteria can be employed to obtain the desired multimer pattern. In some embodiments, the described multimer pattern comprises ultralarge multimers. In some embodiments, the ultralarge multimers are at least 10,000 kDa, at least 11,000 kDa, at least 12,000 kDa, at least 13,000 kDa, at least 14,000 kDa, at least 15,000 kDa, at least 16,000 kDa, at least 17,000 kDa, at least 18,000 kDa, at least 19,000 kDa, or at least 20,000 kDa. In some embodiments, the ultralarge multimers are between about 10,000 kDa and 20,000 kDa. In some embodiments, the ultralarge multimers are between about 11,000 kDa and 20,000 kDa. In some embodiments, the ultralarge multimers are between about 12,000 kDa and 20,000 kDa. In some embodiments, the ultralarge multimers are between about 13,000 kDa and 20,000 kDa. In some embodiments, the ultralarge multimers are between about 14,000 kDa and 20,000 kDa. In some embodiments, the ultralarge multimers are between about 15,000 kDa and 20,000 kDa. In some embodiments, the ultralarge multimers are between about 16,000 kDa and 20,000 kDa. In some embodiments, the ultralarge multimers are between about 17,000 kDa and 20,000 kDa. In some embodiments, the ultralarge multimers are between about 18,000 kDa and 20,000 kDa. In some embodiments, the ultralarge multimers are between about 19,000 kDa and 20,000 kDa. In some embodiments, the rVWF obtained using the present methods includes any multimer pattern present in the loading sample of the rVWF. In some embodiments, the rVWF obtained using the present methods includes physiological occurring multimer patters as well as ultra large VWF-multimer patterns.

In some embodiments, the rVWF composition prepared by the purification method described herein has a distribution of rVWF oligomers characterized in that 95% of the oligomers have between 6 subunits and 20 subunits. In some embodiments, the rVWF composition has a distribution of rVWF oligomers characterized in that 95% of the oligomers have a range of subunits selected from variations 458 to 641 found in Table 4.

**Table 4. Exemplary embodiments for the distribution of rVWF oligomers found in the compositions and used in the methods provided herein.**

| **Subunits** | | **Subunits** | | **Subunits** | | **Subunits** | |
|---|---|---|---|---|---|---|---|
| 2-40 | Var. 458 | 6-16 | Var. 504 | 12-20 | Var. 550 | 20-28 | Var. 596 |
| 2-38 | Var. 459 | 6-14 | Var. 505 | 12-18 | Var. 551 | 20-26 | Var. 597 |
| 2-36 | Var. 460 | 6-12 | Var. 506 | 12-16 | Var. 552 | 20-24 | Var. 598 |
| 2-34 | Var. 461 | 6-10 | Var. 507 | 12-14 | Var. 553 | 20-22 | Var. 599 |
| 2-32 | Var. 462 | 6-8 | Var. 508 | 14-40 | Var. 554 | 22-40 | Var. 600 |
| 2-30 | Var. 463 | 8-40 | Var. 509 | 14-38 | Var. 555 | 22-38 | Var. 601 |
| 2-28 | Var. 464 | 8-38 | Var. 510 | 14-36 | Var. 556 | 22-36 | Var. 602 |
| 2-26 | Var. 465 | 8-36 | Var. 511 | 14-34 | Var. 557 | 22-34 | Var. 603 |
| 2-24 | Var. 466 | 8-34 | Var. 512 | 14-32 | Var. 558 | 22-32 | Var. 604 |
| 2-22 | Var. 467 | 8-32 | Var. 513 | 14-30 | Var. 559 | 22-30 | Var. 605 |
| 2-20 | Var. 468 | 8-30 | Var. 514 | 14-28 | Var. 560 | 22-28 | Var. 606 |
| 2-18 | Var. 469 | 8-28 | Var. 515 | 14-26 | Var. 561 | 22-26 | Var. 607 |
| 2-16 | Var. 470 | 8-26 | Var. 516 | 14-24 | Var. 562 | 22-24 | Var. 608 |
| 2-14 | Var. 471 | 8-24 | Var. 517 | 14-22 | Var. 563 | 24-40 | Var. 609 |
| 2-12 | Var. 472 | 8-22 | Var. 518 | 14-20 | Var. 564 | 24-38 | Var. 610 |
| 2-10 | Var. 473 | 8-20 | Var. 519 | 14-18 | Var. 565 | 24-36 | Var. 611 |
| 2-8 | Var. 474 | 8-18 | Var. 520 | 14-16 | Var. 566 | 24-34 | Var. 612 |
| 4-40 | Var. 475 | 8-16 | Var. 521 | 16-40 | Var. 567 | 24-32 | Var. 613 |
| 4-38 | Var. 476 | 8-14 | Var. 522 | 16-38 | Var. 568 | 24-30 | Var. 614 |
| 4-36 | Var. 477 | 8-12 | Var. 523 | 16-36 | Var. 569 | 24-28 | Var. 615 |
| 4-34 | Var. 478 | 8-10 | Var. 524 | 16-34 | Var. 570 | 24-26 | Var. 616 |
| 4-32 | Var. 479 | 10-40 | Var. 525 | 16-32 | Var. 571 | 26-40 | Var. 617 |
| 4-30 | Var. 480 | 10-38 | Var. 526 | 16-30 | Var. 572 | 26-38 | Var. 618 |
| 4-28 | Var. 481 | 10-36 | Var. 527 | 16-28 | Var. 573 | 26-36 | Var. 619 |
| 4-26 | Var. 482 | 10-34 | Var. 528 | 16-26 | Var. 574 | 26-34 | Var. 620 |
| 4-24 | Var. 483 | 10-32 | Var. 529 | 16-24 | Var. 575 | 26-32 | Var. 621 |
| 4-22 | Var. 484 | 10-30 | Var. 530 | 16-22 | Var. 576 | 26-30 | Var. 622 |
| 4-20 | Var. 485 | 10-28 | Var. 531 | 16-20 | Var. 577 | 26-28 | Var. 623 |
| 4-18 | Var. 486 | 10-26 | Var. 532 | 16-18 | Var. 578 | 28-40 | Var. 624 |
| 4-16 | Var. 487 | 10-24 | Var. 533 | 18-40 | Var. 579 | 28-38 | Var. 625 |
| 4-14 | Var. 488 | 10-22 | Var. 534 | 18-38 | Var. 580 | 28-36 | Var. 626 |
| 4-12 | Var. 489 | 10-20 | Var. 535 | 18-36 | Var. 581 | 28-34 | Var. 627 |
| 4-10 | Var. 490 | 10-18 | Var. 536 | 18-34 | Var. 582 | 28-32 | Var. 628 |
| 4-8 | Var. 491 | 10-16 | Var. 537 | 18-32 | Var. 583 | 28-30 | Var. 629 |
| 6-40 | Var. 492 | 10-14 | Var. 538 | 18-30 | Var. 584 | 30-40 | Var. 630 |
| 6-38 | Var. 493 | 10-12 | Var. 539 | 18-28 | Var. 585 | 30-38 | Var. 631 |
| 6-36 | Var. 494 | 12-40 | Var. 540 | 18-26 | Var. 586 | 30-36 | Var. 632 |
| 6-34 | Var. 495 | 12-38 | Var. 541 | 18-24 | Var. 587 | 30-34 | Var. 633 |
| 6-32 | Var. 496 | 12-36 | Var. 542 | 18-22 | Var. 588 | 30-32 | Var. 634 |
| 6-30 | Var. 497 | 12-34 | Var. 543 | 18-20 | Var. 589 | 32-40 | Var. 635 |
| 6-28 | Var. 498 | 12-32 | Var. 544 | 20-40 | Var. 590 | 32-38 | Var. 636 |
| 6-26 | Var. 499 | 12-30 | Var. 545 | 20-38 | Var. 591 | 32-36 | Var. 637 |
| 6-24 | Var. 500 | 12-28 | Var. 546 | 20-36 | Var. 592 | 32-34 | Var. 638 |
| 6-22 | Var. 501 | 12-26 | Var. 547 | 20-34 | Var. 593 | 34-40 | Var. 639 |
| 6-20 | Var. 502 | 12-24 | Var. 548 | 20-32 | Var. 594 | 36-38 | Var. 640 |
| 6-18 | Var. 503 | 12-22 | Var. 549 | 20-30 | Var. 595 | 38-40 | Var. 641 |

| | | | | | | | |
|---|---|---|---|---|---|---|---|
| Var. = Variation | | | | | | | |

In some embodiments, the rVWF composition prepared by the methods provided herein can be characterized according to the percentage of rVWF molecules that are present in a particular higher order rVWF multimer or larger multimer. For example, in one embodiment, at least 20% of rVWF molecules in a rVWF composition used in the methods described herein are present in an oligomeric complex of at least 10 subunits. In another embodiment, at least 20% of rVWF molecules in a rVWF composition used in the methods described herein are present in an oligomeric complex of at least 12 subunits. In yet other embodiments, a rVWF composition used in the methods provided herein has a minimal percentage (e.g., has at least X%) of rVWF molecules present in a particular higher-order rVWF multimer or larger multimer (e.g., a multimer of at least Y subunits) according to any one of variations 134 to 457 found in Table 5 to Table 7.

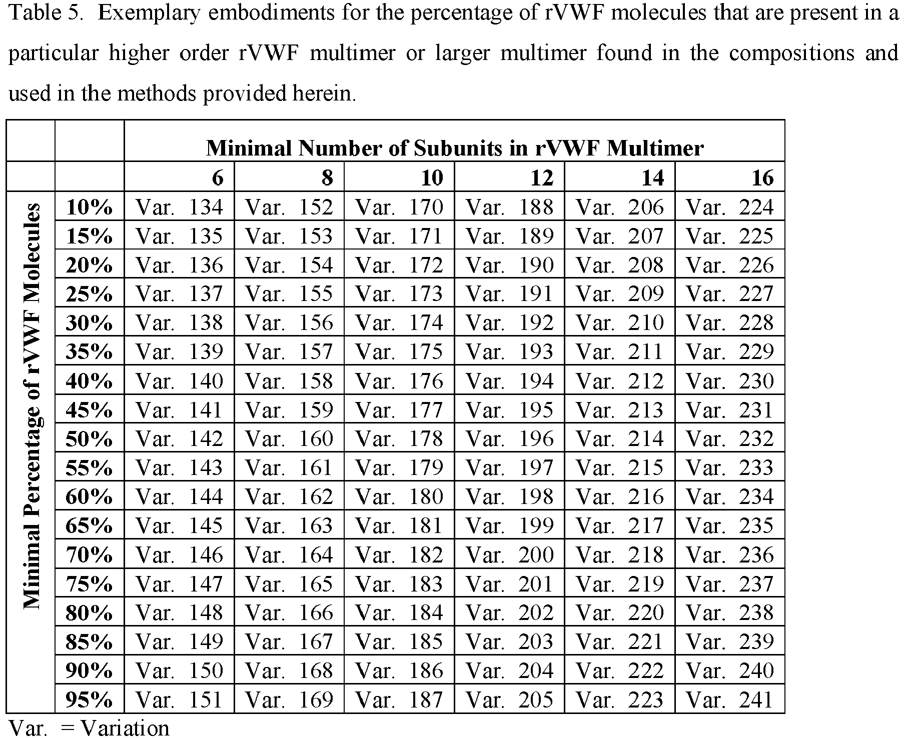

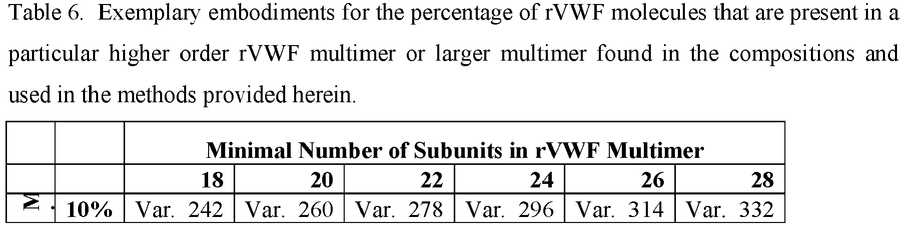

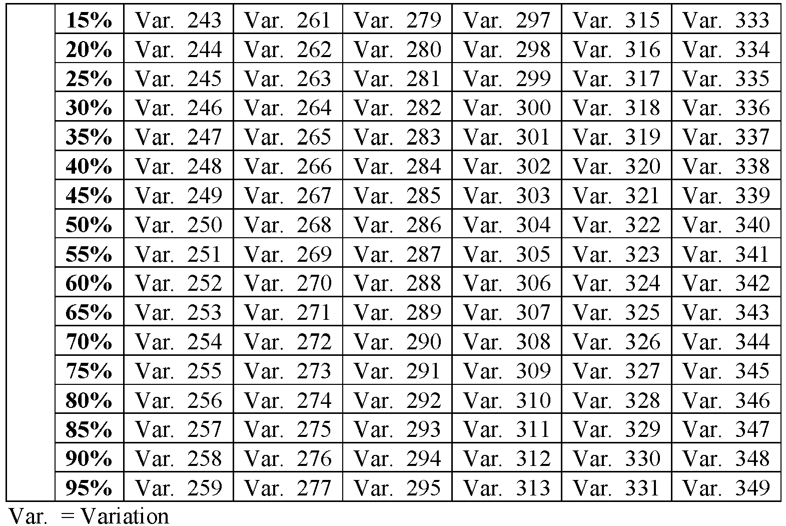

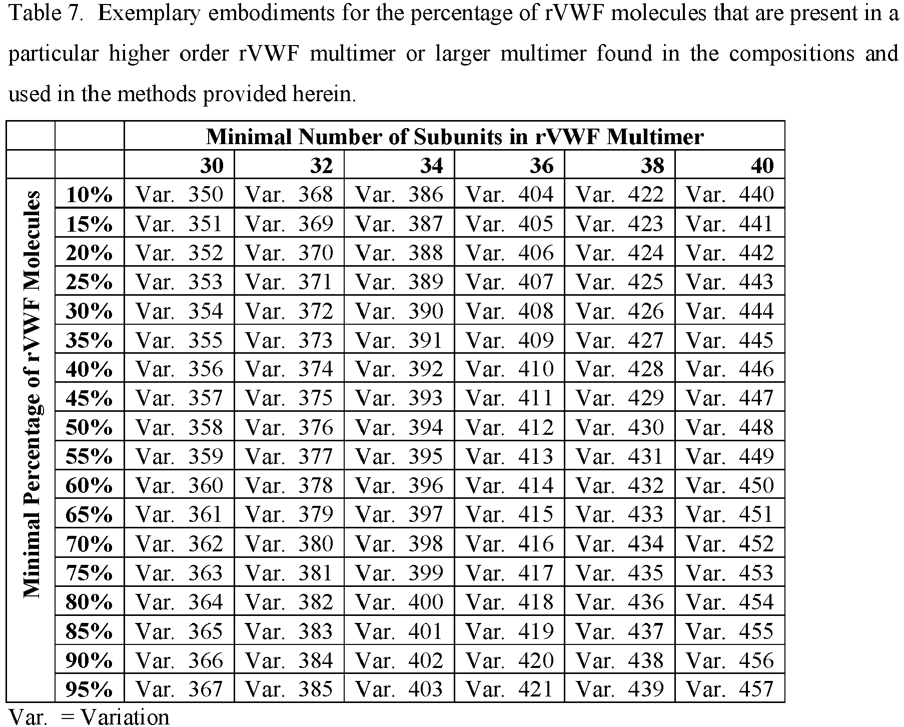

In accordance with the above, the rVWF comprises a significant percentage of high molecular weight (HMW) rVWF multimers. In further embodiments, the HMW rVWF multimer composition comprises at least 10% - 80% rVWF decamers or higher order multimers. In further embodiments, the composition comprises about 10-95%, 20-90%, 30-85%, 40-80%, 50-75%, or 60-70% decamers or higher order multimers. In further embodiments, the HMW rVWF multimer composition comprises at least about 10%, 20%, 30%, 40%, 50%, 60%, 70%, 80%, or 90% decamers or higher order multimers.

Assessment of the number and percentage of rVWF multimers can be conducted using methods known in the art, including without limitation methods using electrophoresis and size exclusion chromatography methods to separate rVWF multimers by size, for example as discussed by Cumming et al, (J Clin Pathol. 1993 May; 46(5): 470-473.
Such techniques may further include immunoblotting techniques (such as Western Blot), in which the gel is immunoblotted with a radiolabelled antibody against VWF followed by chemiluminescent detection (see for example Wen et al., (1993), J. Clin. Lab. Anal., 7: 317-323 .
Further assays for VWF include VWF:Antigen (VWF:Ag), VWF:Ristocetin Cofactor (VWF:RCof), and VWF:Collagen Binding Activity assay (VWF:CBA), which are often used for diagnosis and classification of Von Willebrand Disease. (see for example Favaloro et al., Pathology, 1997, 29(4): 341-456 .

In some embodiments, the ratio of rFVIII procoagulant activity (IU rFVIII:C) to rVWF Ristocetin cofactor activity (IU rVWF:RCo) for the rVWF prepared according to the methods of the present invention is between 3:1 and 1:5. In further embodiments, the ratio is between 2:1 and 1:4. In still further embodiments, the ratio is between 5:2 and 1:4. In further embodiments, the ratio is between 3:2 and 1:3. In still further embodiments, the ratio is about 1:1, 1:2, 1:3, 1:4, 1:5, 2:1, 2:3, 2:4, 2:5, 3:1, 3:2, 3:4, or 3:5. In further embodiments, the ratio is between 1:1 and 1:2. In yet further embodiments, the ratio is 1.1:1, 1.2:1, 1.3:1, 1.4:1, 1.5:1, 1.6:1, 1.7:1, 1.8:1, 1.9:1, or 2:1. In certain embodiments, the ratio of rFVIII procoagulant activity (IU rFVIII:C) to rVWF Ristocetin cofactor activity (IU rVWF:RCo) in a composition useful for a method described herein is selected from variations 1988 to 2140 found in Table 8.

**Table 8. Exemplary embodiments for the ratio of rFVIII procoagulant activity (IU rFVIII:C) to rVWF Ristocetin cofactor activity (IU rVWF:RCo) in compositions and used in methods provided herein.**

| **(IU rFVIII:C) to (IU rVWF:RCo)** | | **(IU rFVIII:C) to (IU rVWF:RCo)** | | **(IU rFVIII:C) to (IU rVWF:RCo)** | | **(IU rFVIII:C) to (IU rVWF:RCo)** | |
|---|---|---|---|---|---|---|---|
| 4:1 | Var. 1988 | 3:1-3:5 | Var. 2027 | 4:3-1:4 | Var. 2065 | 4:5-2:3 | Var. 2103 |
| 3:1 | Var. 1989 | 3:1-2:3 | Var. 2028 | 4:3-1:3 | Var. 2066 | 4:5-3:4 | Var. 2104 |
| 2:1 | Var. 1990 | 3:1-3:4 | Var. 2029 | 4:3-2:5 | Var. 2067 | 3:4-1:6 | Var. 2105 |
| 3:2 | Var. 1991 | 3:1-4:5 | Var. 2030 | 4:3-1:2 | Var. 2068 | 3:4-1:5 | Var. 2106 |
| 4:3 | Var. 1992 | 3:1-5:6 | Var. 2031 | 4:3-3:5 | Var. 2069 | 3:4-1:4 | Var. 2107 |
| 1:1 | Var. 1993 | 3:1-1:1 | Var. 2032 | 4:3-2:3 | Var. 2070 | 3:4-1:3 | Var. 2108 |
| 5:6 | Var. 1994 | 3:1-4:3 | Var. 2033 | 4:3-3:4 | Var. 2071 | 3:4-2:5 | Var. 2109 |
| 4:5 | Var. 1995 | 3:1-3:2 | Var. 2034 | 4:3-4:5 | Var. 2072 | 3:4-1:2 | Var. 2110 |
| 3:4 | Var. 1996 | 3:1-2:1 | Var. 2035 | 4:3-5:6 | Var. 2073 | 3:4-3:5 | Var. 2111 |
| 2:3 | Var. 1997 | 2:1-1:6 | Var. 2036 | 4:3-1:1 | Var. 2074 | 3:4-2:3 | Var. 2112 |
| 3:5 | Var. 1998 | 2:1-1:5 | Var. 2037 | 1:1-1:6 | Var. 2075 | 2:3-1:6 | Var. 2113 |
| 1:2 | Var. 1999 | 2:1-1:4 | Var. 2038 | 1:1-1:5 | Var. 2076 | 2:3-1:5 | Var. 2114 |
| 2:5 | Var. 2000 | 2:1-1:3 | Var. 2039 | 1:1-1:4 | Var. 2077 | 2:3-1:4 | Var. 2115 |
| 1:3 | Var. 2001 | 2:1-2:5 | Var. 2040 | 1:1-1:3 | Var. 2078 | 2:3-1:3 | Var. 2116 |
| 1:4 | Var. 2002 | 2:1-1:2 | Var. 2041 | 1:1-2:5 | Var. 2079 | 2:3-2:5 | Var. 2117 |
| 1:5 | Var. 2003 | 2:1-3:5 | Var. 2042 | 1:1-1:2 | Var. 2080 | 2:3-1:2 | Var. 2118 |
| 1:6 | Var. 2004 | 2:1-2:3 | Var. 2043 | 1:1-3:5 | Var. 2081 | 2:3-3:5 | Var. 2119 |
| 4:1-1:6 | Var. 2005 | 2:1-3:4 | Var. 2044 | 1:1-2:3 | Var. 2082 | 3:5-1:6 | Var. 2120 |
| 4:1-1:5 | Var. 2006 | 2:1-4:5 | Var. 2045 | 1:1-3:4 | Var. 2083 | 3:5-1:5 | Var. 2121 |
| 4:1-1:4 | Var. 2007 | 2:1-5:6 | Var. 2046 | 1:1-4:5 | Var. 2084 | 3:5-1:4 | Var. 2122 |
| 4:1-1:3 | Var. 2008 | 2:1-1:1 | Var. 2047 | 1:1-5:6 | Var. 2085 | 3:5-1:3 | Var. 2123 |
| 4:1-2:5 | Var. 2009 | 2:1-4:3 | Var. 2048 | 5:6-1:6 | Var. 2086 | 3:5-2:5 | Var. 2124 |
| 4:1-1:2 | Var. 2010 | 2:1-3:2 | Var. 2049 | 5:6-1:5 | Var. 2087 | 3:5-1:2 | Var. 2125 |
| 4:1-3:5 | Var. 2011 | 3:2-1:6 | Var. 2050 | 5:6-1:4 | Var. 2088 | 1:2-1:6 | Var. 2126 |
| 4:1-2:3 | Var. 2012 | 3:2-1:5 | Var. 2051 | 5:6-1:3 | Var. 2089 | 1:2-1:5 | Var. 2127 |
| 4:1-3:4 | Var. 2013 | 3:2-1:4 | Var. 2052 | 5:6-2:5 | Var. 2090 | 1:2-1:4 | Var. 2128 |
| 4:1-4:5 | Var. 2014 | 3:2-1:3 | Var. 2053 | 5:6-1:2 | Var. 2091 | 1:2-1:3 | Var. 2129 |
| 4:1-5:6 | Var. 2015 | 3:2-2:5 | Var. 2054 | 5:6-3:5 | Var. 2092 | 1:2-2:5 | Var. 2130 |
| 4:1-1:1 | Var. 2016 | 3:2-1:2 | Var. 2055 | 5:6-2:3 | Var. 2093 | 2:5-1:6 | Var. 2131 |
| 4:1-4:3 | Var. 2017 | 3:2-3:5 | Var. 2056 | 5:6-3:4 | Var. 2094 | 2:5-1:5 | Var. 2132 |
| 4:1-3:2 | Var. 2018 | 3:2-2:3 | Var. 2057 | 5:6-4:5 | Var. 2095 | 2:5-1:4 | Var. 2133 |
| 4:1-2:1 | Var. 2019 | 3:2-3:4 | Var. 2058 | 4:5-1:6 | Var. 2096 | 2:5-1:3 | Var. 2134 |
| 4:1-3:1 | Var. 2020 | 3:2-4:5 | Var. 2059 | 4:5-1:5 | Var. 2097 | 1:3-1:6 | Var. 2135 |
| 3:1-1:6 | Var. 2021 | 3:2-5:6 | Var. 2060 | 4:5-1:4 | Var. 2098 | 1:3-1:5 | Var. 2136 |
| 3:1-1:5 | Var. 2022 | 3:2-1:1 | Var. 2061 | 4:5-1:3 | Var. 2099 | 1:3-1:4 | Var. 2137 |
| 3:1-1:4 | Var. 2023 | 3:2-4:3 | Var. 2062 | 4:5-2:5 | Var. 2100 | 1:4-1:6 | Var. 2138 |
| 3:1-1:3 | Var. 2024 | 4:3-1:6 | Var. 2063 | 4:5-1:2 | Var. 2101 | 1:4-1:5 | Var. 2139 |
| 3:1-2:5 | Var. 2025 | 4:3-1:5 | Var. 2064 | 4:5-3:5 | Var. 2102 | 1:5-1:6 | Var. 2140 |
| 3:1-1:2 | Var. 2026 | | | | | | |

| | | | | | | | |
|---|---|---|---|---|---|---|---|
| Var. = Variation | | | | | | | |

In further embodiments, higher order rVWF multimers of the invention are stable for about 1 to about 90 hours post-administration. In still further embodiments, the higher order rVWF multimers are stable for about 5-80, 10-70, 15-60, 20-50, 25-40, or 30-35 hours post-administration. In yet further embodiments, the higher order rVWF multimers are stable for at least 3, 6, 12, 18, 24, 36, 48, or 72 hours post-administration. In certain embodiments the stability of the rVWF multimers is assessed *in vitro.*

In some embodiments, compositions and methods provided herein comprise higher order rVWF multimers having a half-life of at least 12 hours or at least 24 hours post administration. In other embodiments, the higher order rVWF multimers have a half-life selected from variations 642 to 1045 found in Table 9.

**Table 9. Exemplary embodiments for the half-life of higher order rVWF multimers found in the compositions prepared by the methods provided herein.**

| **Hours** | | **Hours** | | **Hours** | | **Hours** | |
|---|---|---|---|---|---|---|---|
| at least 1 | Var. 642 | 4-22 | Var. 743 | 14-78 | Var. 844 | 24-30 | Var. 945 |
| at least 2 | Var. 643 | 4-20 | Var. 744 | 14-72 | Var. 845 | 24-27 | Var. 946 |
| at least 3 | Var. 644 | 4-18 | Var. 745 | 14-66 | Var. 846 | 27-90 | Var. 947 |
| at least 4 | Var. 645 | 4-16 | Var. 746 | 14-60 | Var. 847 | 27-84 | Var. 948 |
| at least 5 | Var. 646 | 4-14 | Var. 747 | 14-54 | Var. 848 | 27-78 | Var. 949 |
| at least 6 | Var. 647 | 4-12 | Var. 748 | 14-48 | Var. 849 | 27-72 | Var. 950 |
| at least 7 | Var. 648 | 4-10 | Var. 749 | 14-45 | Var. 850 | 27-66 | Var. 951 |
| at least 8 | Var. 649 | 4-8 | Var. 750 | 14-42 | Var. 851 | 27-60 | Var. 952 |
| at least 9 | Var. 650 | 4-6 | Var. 751 | 14-39 | Var. 852 | 27-54 | Var. 953 |
| at least 10 | Var. 651 | 6-90 | Var. 752 | 14-36 | Var. 853 | 27-48 | Var. 954 |
| at least 11 | Var. 652 | 6-84 | Var. 753 | 14-33 | Var. 854 | 30-90 | Var. 955 |
| at least 12 | Var. 653 | 6-78 | Var. 754 | 14-30 | Var. 855 | 30-84 | Var. 956 |
| at least 14 | Var. 654 | 6-72 | Var. 755 | 14-27 | Var. 856 | 30-78 | Var. 957 |
| at least 16 | Var. 655 | 6-66 | Var. 756 | 14-24 | Var. 857 | 30-72 | Var. 958 |
| at least 18 | Var. 656 | 6-60 | Var. 757 | 14-22 | Var. 858 | 30-66 | Var. 959 |
| at least 20 | Var. 657 | 6-54 | Var. 758 | 14-20 | Var. 859 | 30-60 | Var. 960 |
| at least 22 | Var. 658 | 6-48 | Var. 759 | 14-18 | Var. 860 | 30-54 | Var. 961 |
| at least 24 | Var. 659 | 6-45 | Var. 760 | 14-16 | Var. 861 | 30-48 | Var. 962 |
| at least 27 | Var. 660 | 6-42 | Var. 761 | 16-90 | Var. 862 | 30-45 | Var. 963 |
| at least 30 | Var. 661 | 6-39 | Var. 762 | 16-84 | Var. 863 | 30-42 | Var. 964 |
| at least 33 | Var. 662 | 6-36 | Var. 763 | 16-78 | Var. 864 | 30-39 | Var. 965 |
| at least 36 | Var. 663 | 6-33 | Var. 764 | 16-72 | Var. 865 | 30-36 | Var. 966 |
| at least 39 | Var. 664 | 6-30 | Var. 765 | 16-66 | Var. 866 | 30-33 | Var. 967 |
| at least 42 | Var. 665 | 6-27 | Var. 766 | 16-60 | Var. 867 | 33-90 | Var. 968 |
| at least 45 | Var. 666 | 6-24 | Var. 767 | 16-54 | Var. 868 | 33-84 | Var. 969 |
| at least 48 | Var. 667 | 6-22 | Var. 768 | 16-48 | Var. 869 | 33-78 | Var. 970 |
| at least 54 | Var. 668 | 6-20 | Var. 769 | 16-45 | Var. 870 | 33-72 | Var. 971 |
| at least 60 | Var. 669 | 6-18 | Var. 770 | 16-42 | Var. 871 | 33-66 | Var. 972 |
| at least 66 | Var. 670 | 6-16 | Var. 771 | 16-39 | Var. 872 | 33-60 | Var. 973 |
| at least 72 | Var. 671 | 6-14 | Var. 772 | 16-36 | Var. 873 | 33-54 | Var. 974 |
| at least 78 | Var. 672 | 6-12 | Var. 773 | 16-33 | Var. 874 | 33-48 | Var. 975 |
| at least 84 | Var. 673 | 6-10 | Var. 774 | 16-30 | Var. 875 | 33-45 | Var. 976 |
| at least 90 | Var. 674 | 6-8 | Var. 775 | 16-27 | Var. 876 | 33-42 | Var. 977 |
| 2-90 | Var. 675 | 8-90 | Var. 776 | 16-24 | Var. 877 | 33-29 | Var. 978 |
| 2-84 | Var. 676 | 8-84 | Var. 777 | 16-22 | Var. 878 | 33-36 | Var. 979 |
| 2-78 | Var. 677 | 8-78 | Var. 778 | 16-20 | Var. 879 | 36-90 | Var. 980 |
| 2-72 | Var. 678 | 8-72 | Var. 779 | 16-18 | Var. 880 | 36-84 | Var. 981 |
| 2-66 | Var. 679 | 8-66 | Var. 780 | 18-90 | Var. 881 | 36-78 | Var. 982 |
| 2-60 | Var. 680 | 8-60 | Var. 781 | 18-84 | Var. 882 | 36-72 | Var. 983 |
| 2-54 | Var. 681 | 8-54 | Var. 782 | 18-78 | Var. 883 | 36-66 | Var. 984 |
| 2-48 | Var. 682 | 8-48 | Var. 783 | 18-72 | Var. 884 | 36-60 | Var. 985 |
| 2-45 | Var. 683 | 8-45 | Var. 784 | 18-66 | Var. 885 | 36-54 | Var. 986 |
| 2-42 | Var. 684 | 8-42 | Var. 785 | 18-60 | Var. 886 | 36-48 | Var. 987 |
| 2-39 | Var. 685 | 8-39 | Var. 786 | 18-54 | Var. 887 | 36-45 | Var. 988 |
| 2-36 | Var. 686 | 8-36 | Var. 787 | 18-48 | Var. 888 | 36-42 | Var. 989 |
| 2-33 | Var. 687 | 8-33 | Var. 788 | 18-45 | Var. 889 | 36-39 | Var. 990 |
| 2-30 | Var. 688 | 8-30 | Var. 789 | 18-42 | Var. 890 | 39-90 | Var. 991 |
| 2-27 | Var. 689 | 8-27 | Var. 790 | 18-39 | Var. 891 | 39-84 | Var. 992 |
| 2-24 | Var. 690 | 8-24 | Var. 791 | 18-36 | Var. 892 | 39-78 | Var. 993 |
| 2-22 | Var. 691 | 8-22 | Var. 792 | 18-33 | Var. 893 | 39-72 | Var. 994 |
| 2-20 | Var. 692 | 8-20 | Var. 793 | 18-30 | Var. 894 | 39-66 | Var. 995 |
| 2-18 | Var. 693 | 8-18 | Var. 794 | 18-27 | Var. 895 | 39-60 | Var. 996 |
| 2-16 | Var. 694 | 8-16 | Var. 795 | 18-24 | Var. 896 | 39-54 | Var. 997 |
| 2-14 | Var. 695 | 8-14 | Var. 796 | 18-22 | Var. 897 | 39-48 | Var. 998 |
| 2-12 | Var. 696 | 8-12 | Var. 797 | 18-20 | Var. 898 | 39-45 | Var. 999 |
| 2-10 | Var. 697 | 8-10 | Var. 798 | 20-90 | Var. 899 | 39-42 | Var. 1000 |
| 2-8 | Var. 698 | 10-90 | Var. 799 | 20-84 | Var. 900 | 42-90 | Var. 1001 |
| 2-6 | Var. 699 | 10-84 | Var. 800 | 20-78 | Var. 901 | 42-84 | Var. 1002 |
| 2-4 | Var. 700 | 10-78 | Var. 801 | 20-72 | Var. 902 | 42-78 | Var. 1003 |
| 3-90 | Var. 701 | 10-72 | Var. 802 | 20-66 | Var. 903 | 42-72 | Var. 1004 |
| 3-84 | Var. 702 | 10-66 | Var. 803 | 20-60 | Var. 904 | 42-66 | Var. 1005 |
| 3-78 | Var. 703 | 10-60 | Var. 804 | 20-54 | Var. 905 | 42-60 | Var. 1006 |
| 3-72 | Var. 704 | 10-54 | Var. 805 | 20-48 | Var. 906 | 42-54 | Var. 1007 |
| 3-66 | Var. 705 | 10-48 | Var. 806 | 20-45 | Var. 907 | 42-48 | Var. 1008 |
| 3-60 | Var. 706 | 10-45 | Var. 807 | 20-42 | Var. 908 | 42-45 | Var. 1009 |
| 3-54 | Var. 707 | 10-42 | Var. 808 | 20-39 | Var. 909 | 45-90 | Var. 1010 |
| 3-48 | Var. 708 | 10-39 | Var. 809 | 20-36 | Var. 910 | 45-84 | Var. 1011 |
| 3-45 | Var. 709 | 10-36 | Var. 810 | 20-33 | Var. 911 | 45-78 | Var. 1012 |
| 3-42 | Var. 710 | 10-33 | Var. 811 | 20-30 | Var. 912 | 45-72 | Var. 1013 |
| 3-39 | Var. 711 | 10-30 | Var. 812 | 20-27 | Var. 913 | 45-66 | Var. 1014 |
| 3-36 | Var. 712 | 10-27 | Var. 813 | 20-24 | Var. 914 | 45-60 | Var. 1015 |
| 3-33 | Var. 713 | 10-24 | Var. 814 | 20-22 | Var. 915 | 45-54 | Var. 1016 |
| 3-30 | Var. 714 | 10-22 | Var. 815 | 22-90 | Var. 916 | 45-48 | Var. 1017 |
| 3-27 | Var. 715 | 10-20 | Var. 816 | 22-84 | Var. 917 | 48-90 | Var. 1018 |
| 3-24 | Var. 716 | 10-18 | Var. 817 | 22-78 | Var. 918 | 48-84 | Var. 1019 |
| 3-22 | Var. 717 | 10-16 | Var. 818 | 22-72 | Var. 919 | 48-78 | Var. 1020 |
| 3-20 | Var. 718 | 10-14 | Var. 819 | 22-66 | Var. 920 | 48-72 | Var. 1021 |
| 3-18 | Var. 719 | 10-12 | Var. 820 | 22-60 | Var. 921 | 48-66 | Var. 1022 |
| 3-16 | Var. 720 | 12-90 | Var. 821 | 22-54 | Var. 922 | 48-60 | Var. 1023 |
| 3-14 | Var. 721 | 12-84 | Var. 822 | 22-48 | Var. 923 | 48-54 | Var. 1024 |
| 3-12 | Var. 722 | 12-78 | Var. 823 | 22-45 | Var. 924 | 54-90 | Var. 1025 |
| 3-10 | Var. 723 | 12-72 | Var. 824 | 22-42 | Var. 925 | 54-84 | Var. 1026 |
| 3-8 | Var. 724 | 12-66 | Var. 825 | 22-39 | Var. 926 | 54-78 | Var. 1027 |
| 3-6 | Var. 725 | 12-60 | Var. 826 | 22-36 | Var. 927 | 54-72 | Var. 1028 |
| 3-4 | Var. 726 | 12-54 | Var. 827 | 22-33 | Var. 928 | 54-66 | Var. 1029 |
| 4-90 | Var. 727 | 12-48 | Var. 828 | 22-30 | Var. 929 | 54-60 | Var. 1030 |
| 4-84 | Var. 728 | 12-45 | Var. 829 | 22-27 | Var. 930 | 60-90 | Var. 1031 |
| 4-78 | Var. 729 | 12-42 | Var. 830 | 22-24 | Var. 931 | 60-84 | Var. 1032 |
| 4-72 | Var. 730 | 12-39 | Var. 831 | 24-90 | Var. 932 | 60-78 | Var. 1033 |
| 4-66 | Var. 731 | 12-36 | Var. 832 | 24-84 | Var. 933 | 60-72 | Var. 1034 |
| 4-60 | Var. 732 | 12-33 | Var. 833 | 24-78 | Var. 934 | 60-66 | Var. 1035 |
| 4-54 | Var. 733 | 12-30 | Var. 834 | 24-72 | Var. 935 | 66-90 | Var. 1036 |
| 4-48 | Var. 734 | 12-27 | Var. 835 | 24-66 | Var. 936 | 66-84 | Var. 1037 |
| 4-45 | Var. 735 | 12-24 | Var. 836 | 24-60 | Var. 937 | 66-78 | Var. 1038 |
| 4-42 | Var. 736 | 12-22 | Var. 837 | 24-54 | Var. 938 | 66-72 | Var. 1039 |
| 4-39 | Var. 737 | 12-20 | Var. 838 | 24-48 | Var. 939 | 72-90 | Var. 1040 |
| 4-36 | Var. 738 | 12-18 | Var. 839 | 24-45 | Var. 940 | 72-84 | Var. 1041 |
| 4-33 | Var. 739 | 12-16 | Var. 840 | 24-42 | Var. 941 | 72-78 | Var. 1042 |
| 4-30 | Var. 740 | 12-14 | Var. 841 | 24-39 | Var. 942 | 78-90 | Var. 1043 |
| 4-27 | Var. 741 | 14-90 | Var. 842 | 24-36 | Var. 943 | 78-84 | Var. 1044 |
| 4-24 | Var. 742 | 14-84 | Var. 843 | 24-33 | Var. 944 | 84-90 | Var. 1045 |

| | | | | | | | |
|---|---|---|---|---|---|---|---|
| Var. = Variation | | | | | | | |

Another method for measuring the biological activity of VWF is the collagen binding assay, which is based on ELISA technology (Brown and Bosak, Thromb. Res., 1986, 43:303-311; Favaloro, Thromb. Haemost., 2000, 83 127-135). A microtiter plate is coated with type I or III collagen. Then the VWF is bound to the collagen surface and subsequently detected with an enzyme-labeled polyclonal antibody. The last step is a substrate reaction, which can be photometrically monitored with an ELISA reader.

Immunological assays of von Willebrand factors (VWF:Ag) are immunoassays that measure the concentration of the VWF protein in plasma. They give no indication as to VWF function. A number of methods exist for measuring VWF:Ag and these include both enzyme-linked immunosorbent assay (ELISA) or automated latex immunoassays (LIA.) Many laboratories now use a fully automated latex immunoassay. Historically laboratories used a variety of techniques including Laurell electroimmunoassay "Laurell Rockets" but these are rarely used in most labs today.

### IV. Kits

As an additional aspect, the invention includes kits which comprise one or more lyophilized compositions packaged in a manner which facilitates their use for administration to subjects. In one embodiment, such a kit includes pharmaceutical formulation described herein (*e.g.*, a composition comprising a therapeutic protein or peptide), packaged in a container such as a sealed bottle or vessel, with a label affixed to the container or included in the package that describes use of the compound or composition in practicing the method. In one embodiment, the pharmaceutical formulation is packaged in the container such that the amount of headspace in the container (*e.g.*, the amount of air between the liquid formulation and the top of the container) is very small. Preferably, the amount of headspace is negligible (*e.g*., almost none). In one embodiment, the kit contains a first container having a therapeutic protein or peptide composition and a second container having a physiologically acceptable reconstitution solution for the composition. In one aspect, the pharmaceutical formulation is packaged in a unit dosage form. The kit may further include a device suitable for administering the pharmaceutical formulation according to a specific route of administration. Preferably, the kit contains a label that describes use of the pharmaceutical formulations.

### V. Methods of Treating Patients Related to Forming Complexes Containing VWF and Complement C1q

One of the advantages of administering rVWF to subjects is that the higher specific activity of rVWF as compared to pdVWF allows flexibility in the amount of rVWF administered and the number of times the subject is re-dosed. As will be appreciated and as is discussed in further detail herein, the co-administered FVIII may be recombinant or plasma derived.

Single or multiple administrations of rVWF are carried out with the dose levels and pattern being selected by the treating physician. For the prevention or treatment of disease, the appropriate dosage depends on the type of disease to be treated (e.g., von Willebrand disease), the severity and course of the disease, whether drug is administered for preventive or therapeutic purposes, previous therapy, the patient's clinical history and response to the drug, and the discretion of the attending physician.

Compositions of rVWF can be contained in pharmaceutical formulations, as described herein. Such formulations can be administered orally, topically, transdermally, parenterally, by inhalation spray, vaginally, rectally, or by intracranial injection. The term parenteral as used herein includes subcutaneous injections, intravenous, intramuscular, intracisternal injection, or infusion techniques. Administration by intravenous, intradermal, intramuscular, intramammary, intraperitoneal, intrathecal, retrobulbar, intrapulmonary injection and or surgical implantation at a particular site is contemplated as well. Generally, compositions are essentially free of pyrogens, as well as other impurities that could be harmful to the recipient.

In one aspect, formulations of the invention are administered by an initial bolus followed by a continuous infusion to maintain therapeutic circulating levels of drug product. As another example, the inventive compound is administered as a one-time dose. Those of ordinary skill in the art will readily optimize effective dosages and administration regimens as determined by good medical practice and the clinical condition of the individual patient. The route of administration can be, but is not limited to, by intravenous, intraperitoneal, subcutaneous, or intramuscular administration. The frequency of dosing depends on the pharmacokinetic parameters of the agents and the route of administration. The optimal pharmaceutical formulation is determined by one skilled in the art depending upon the route of administration and desired dosage. See for example, Remington's Pharmaceutical Sciences, 18th Ed., 1990, Mack Publishing Co., Easton, Pa. 18042 pages 1435-1712 .

Such formulations influence the physical state, stability, rate of in vivo release, and rate of in vivo clearance of the administered agents. Depending on the route of administration, a suitable dose is calculated according to body weight, body surface area or organ size. Appropriate dosages may be ascertained through use of established assays for determining blood level dosages in conjunction with appropriate dose-response data. The final dosage regimen is determined by the attending physician, considering various factors which modify the action of drugs, *e.g.,* the drug's specific activity, the severity of the damage and the responsiveness of the patient, the age, condition, body weight, sex and diet of the patient, the severity of any infection, time of administration and other clinical factors. By way of example, a typical dose of a recombinant VWF of the present invention is approximately 50 IU/kg, equal to 500 µg/kg. As studies are conducted, further information will emerge regarding the appropriate dosage levels and duration of treatment for various diseases and conditions.

The practice of the present invention may employ, unless otherwise indicated, conventional techniques and descriptions of organic chemistry, polymer technology, molecular biology (including recombinant techniques), cell biology, biochemistry, and immunology, which are within the skill of the art. Such conventional techniques include polymer array synthesis, hybridization, ligation, and detection of hybridization using a label. Specific illustrations of suitable techniques can be had by reference to the example herein below. However, other equivalent conventional procedures can, of course, also be used. Such conventional techniques and descriptions can be found in standard laboratory manuals such as Genome Analysis: A Laboratory Manual Series (Vols. I-IV), Using Antibodies: A Laboratory Manual, Cells: A Laboratory Manual, PCR Primer: A Laboratory Manual, and Molecular Cloning: A Laboratory Manual (all from Cold Spring Harbor Laboratory Press), Stryer, L. (1995) Biochemistry (4th Ed.) Freeman, Highly stabilized York, Gait, "Oligonucleotide Synthesis: A Practical Approach" 1984, IRL Press, London, Nelson and Cox (2000), Lehninger, Principles of Biochemistry 3rd Ed., W. H. Freeman Pub., Highly stabilized York, N.Y. and Berg et al. (2002) Biochemistry, 5th Ed., W. H. Freeman Pub., Highly stabilized York, N.Y.

Note that as used herein and in the appended claims, the singular forms "a," "an," and "the" include plural referents unless the context clearly dictates otherwise. Thus, for example, reference to "a polymerase" refers to one agent or mixtures of such agents, and reference to "the method" includes reference to equivalent steps and methods known to those skilled in the art, and so forth. Thus, for example, reference to "an antibody" includes a plurality of such antibodies and reference to "a host cell" includes reference to one or more host cells and equivalents thereof known to those skilled in the art, and so forth. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

Unless defined otherwise, all technical and scientific terms used herein have the same meaning as commonly understood by one of ordinary skill in the art to which this invention belongs.

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges may independently be included in the smaller ranges is also encompassed within the invention, subject to any specifically excluded limit in the stated range. Where the stated range includes one or both of the limits, ranges excluding either both of those included limits are also included in the invention.

In the above description, numerous specific details are set forth to provide a more thorough understanding of the present invention. However, it will be apparent to one of skill in the art that the present invention may be practiced without one or more of these specific details. In other instances, well-known features and procedures well known to those skilled in the art have not been described in order to avoid obscuring the invention.

Although the present invention is described primarily with reference to specific embodiments, it is also envisioned that other embodiments will become apparent to those skilled in the art upon reading the present disclosure, and it is intended that such embodiments be contained within the present inventive method.

### a. Administration of rVWF for Methods of Treating Patients with Atherosclerosis

Provided herein is a method of treating atherosclerosis in a subject (*e.g.,* a subject with atherosclerosis) comprising administering to a subject a therapeutically effective amount of a composition comprising rVWF such that rVWF binds complement C1q to form C1q-rVWF complexes and the C1q-rVWF complex binds cholesterol crystals (CC), thereby forming a CC-C1q-rVWF complex and inhibiting atherosclerotic plaque progression.

In some embodiments, the rVWF described herein comprises a polypeptide selected from the group consisting of: (a) the amino acid sequence of SEQ ID NO:3; (b) a biologically active analog, fragment, or variant of (a); (c) a polypeptide encoded by the polynucleotide sequence of SEQ ID NO: 1; and (d) a biologically active analog, fragment, or variant of (c). In some embodiments, the rVWF comprises a polypeptide consisting of the amino acid sequence of SEQ ID NO:3. In some embodiments, the rVWF comprises a polypeptide consisting of a biologically active analog, fragment, or variant of the amino acid sequence of SEQ ID NO:3. In some embodiments, the rVWF comprises a polypeptide consisting of a polypeptide encoded by the polynucleotide sequence of SEQ ID NO: 1. In some embodiments, the rVWF comprises a polypeptide consisting of a polypeptide encoded by the polynucleotide sequence of SEQ ID NO: 1; and a biologically active analog, fragment, or variant of a polypeptide encoded by the polynucleotide sequence of SEQ ID NO:1.

Also provided herein is a method of decreasing inflammation of atherosclerotic plaque in a subject (*e.g.,* a subject with atherosclerosis) comprising administering to a subject a therapeutically effective amount of a composition comprising rVWF such that vWF binds complement C1q to form C1q-rVWF complexes, and the C1q-rVWF complex binds cholesterol crystals (CC), thereby forming a CC-C1q-rVWF complex and decreasing inflammation of atherosclerotic plaque.

In some embodiments, any of the rVWF compositions described herein comprise a high molecular weight vWF multimer comprising at least 20%, at least 40%, at least 60%, at least 80%, or at least 90%, or more VWF decamers or high order multimers. In some embodiments, the composition of rVWF comprises a high molecular weight vWF multimer comprising at least 20% VWF decamers or high order multimers. In some embodiments, the composition of rVWF comprises a high molecular weight vWF multimer comprising at least 40% VWF decamers or high order multimers. In some embodiments, the composition of rVWF comprises a high molecular weight vWF multimer comprising at least 60% VWF decamers or high order multimers.. In some embodiments, the composition of rVWF comprises a high molecular weight vWF multimer comprising at least 80% VWF decamers or high order multimers.

Also provided herein is a method of modulating an immune response of macrophages in a blood vessel of a subject (*e.g.,* a subject with atherosclerosis) comprising administering to a subject a therapeutically effective amount of a composition comprising rVWF such that rVWF binds complement C1q to form C1q-rVWF complexes, thereby reducing macrophage cell formation, increasing the expression level of one or more phagocytosis-mediating receptors and/or costimulatory receptors on the surface of the macrophages, decreasing phagocytosis by the macrophages, and/or decreasing pro-inflammatory cytokine secretion by the macrophages.

One of the advantages of administering rVWF to subjects with atherosclerosis is that the higher specific activity of rVWF as compared to pdVWF allows flexibility in the amount of rVWF administered and the number of times the subject is re-dosed. As will be appreciated and as is discussed in further detail herein, the co-administered FVIII may be recombinant or plasma derived.

In some embodiments, rVWF administered to the subject bind to cholesterol crystals in a C1q-dependent manner. In some embodiments, complexes comprising rVWF, CC and C1q results in increased or elevated surface expression of phagocytosis-mediated receptors by cells such as macrophages. In some instances, the phagocytosis-mediated receptors include, but are not limited, to MerTK, LRP-1 and SR-A1. In some embodiments, one or more proteins selected from MerTK, LRP-1, SR-A1, CD14, LAIR1, and PD-L1 are upregulated by such complexes comprising rVWF, CC and C1q.

In some embodiments, complexes comprising rVWF, CC and C1q results in decreased or reduced caspase-1 activation. In some embodiments, the complexes reduce IL-1 secretion (*e.g.,* IL-1β secretion). In some embodiments, the complexes reduce the IL-10/IL-1RA ratio. In some embodiments, the complexes reduce the IL-1α/IL-1RA ratio.

In some embodiments, rVWF binds to C1q and modulates the effect or activity of C1q on macrophages, such as macrophages in a subject. In certain embodiments, complexes comprising rVWF and C1q affect phagocytosis of phagocytes including macrophages. In some instances, macrophages in the subject exhibit reduced phagocytosis of complexes comprising rVWF and C1q, including complexes comprising rVWF, C1q and cholesterol crystals. In some embodiments, rVWF reduces or decreases inflammation due to atherosclerosis (*e.g.,* atherosclerotic inflammation). Complexes comprising rVWF, C1q and CC can regulate the immune response of macrophages in or around atherosclerotic plaques or lesions. In some embodiments, rVWF administered to a subject exhibit reduced foam cell formation compared to subject who has not been administered rVWF. In some embodiments, rVWF administered to a subject reduces or decelerates plaque progression in a subject having atherosclerosis.

The practice of the present invention may employ, unless otherwise indicated, conventional techniques and descriptions of organic chemistry, polymer technology, molecular biology (including recombinant techniques), cell biology, biochemistry, and immunology, which are within the skill of the art. Such conventional techniques include polymer array synthesis, hybridization, ligation, and detection of hybridization using a label. Specific illustrations of suitable techniques can be had by reference to the example herein below. However, other equivalent conventional procedures can, of course, also be used. Such conventional techniques and descriptions can be found in standard laboratory manuals such as Genome Analysis: A Laboratory Manual Series (Vols. I-IV), Using Antibodies: A Laboratory Manual, Cells: A Laboratory Manual, PCR Primer: A Laboratory Manual, and Molecular Cloning: A Laboratory Manual (all from Cold Spring Harbor Laboratory Press), Stryer, L. (1995) Biochemistry (4th Ed.) Freeman, Highly stabilized York, Gait, "Oligonucleotide Synthesis: A Practical Approach" 1984, IRL Press, London, Nelson and Cox (2000), Lehninger, Principles of Biochemistry 3rd Ed., W. H. Freeman Pub., Highly stabilized York, N.Y. and Berg et al. (2002) Biochemistry, 5th Ed., W. H. Freeman Pub., Highly stabilized York, N.Y.

Unless defined otherwise, all technical and scientific terms used herein have the same meaning as commonly understood by one of ordinary skill in the art to which this invention belongs.

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges may independently be included in the smaller ranges is also encompassed within the invention, subject to any specifically excluded limit in the stated range. Where the stated range includes one or both of the limits, ranges excluding either both of those included limits are also included in the invention.

In the above description, numerous specific details are set forth to provide a more thorough understanding of the present invention. However, it will be apparent to one of skill in the art that the present invention may be practiced without one or more of these specific details. In other instances, well-known features and procedures well known to those skilled in the art have not been described in order to avoid obscuring the invention.

Although the present invention is described primarily with reference to specific embodiments, it is also envisioned that other embodiments will become apparent to those skilled in the art upon reading the present disclosure, and it is intended that such embodiments be contained within the present inventive method.

### b. Pharmaceutical Formulations and Excipients in General

Excipients are additives that either impart or enhance the stability and delivery of a drug product (e.g., protein). Regardless of the reason for their inclusion, excipients are an integral component of a formulation and therefore need to be safe and well tolerated by patients. For protein drugs, the choice of excipients is particularly important because they can affect both efficacy and immunogenicity of the drug. Hence, protein formulations need to be developed with appropriate selection of excipients that afford suitable stability, safety, and marketability.

A lyophilized formulation is, in one aspect, at least comprised of one or more of a buffer, a bulking agent, and a stabilizer. In this aspect, the utility of a surfactant is evaluated and selected in cases where aggregation during the lyophilization step or during reconstitution becomes an issue. An appropriate buffering agent is included to maintain the formulation within stable zones of pH during lyophilization. A comparison of the excipient components contemplated for liquid and lyophilized protein formulations is provided in Table 10.

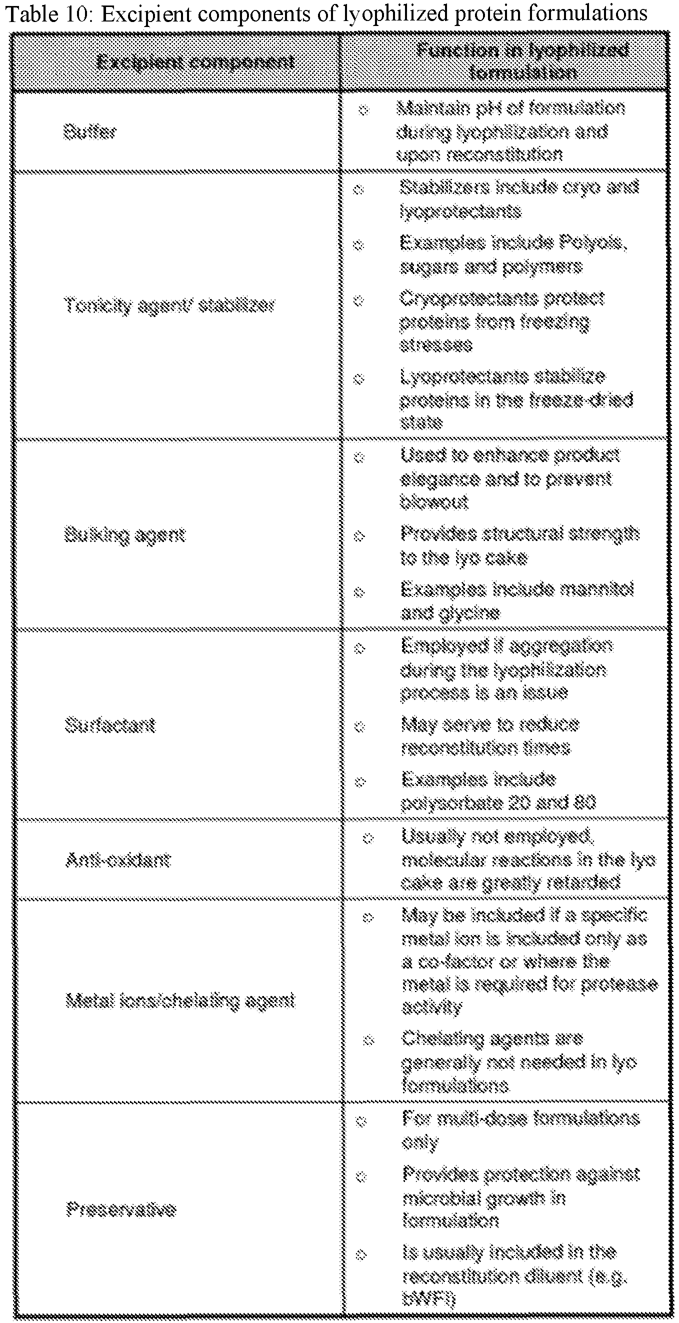

The principal challenge in developing formulations for proteins is stabilizing the product against the stresses of manufacturing, shipping and storage. The role of formulation excipients is to provide stabilization against these stresses. Excipients are also be employed to reduce viscosity of high concentration protein formulations in order to enable their delivery and enhance patient convenience. In general, excipients can be classified on the basis of the mechanisms by which they stabilize proteins against various chemical and physical stresses. Some excipients are used to alleviate the effects of a specific stress or to regulate a particular susceptibility of a specific protein. Other excipients have more general effects on the physical and covalent stabilities of proteins. The excipients described herein are organized either by their chemical type or their functional role in formulations. Brief descriptions of the modes of stabilization are provided when discussing each excipient type.

Given the teachings and guidance provided herein, those skilled in the art will know what amount or range of excipient can be included in any particular formulation to achieve a biopharmaceutical formulation of the invention that promotes retention in stability of the biopharmaceutical (*e.g.,* a protein). For example, the amount and type of a salt to be included in a biopharmaceutical formulation of the invention is selected based on the desired osmolality (*e.g.,* isotonic, hypotonic or hypertonic) of the final solution as well as the amounts and osmolality of other components to be included in the formulation.

By way of example, inclusion of about 5% sorbitol can achieve isotonicity while about 9% of a sucrose excipient is needed to achieve isotonicity. Selection of the amount or range of concentrations of one or more excipients that can be included within a biopharmaceutical formulation of the invention has been exemplified above by reference to salts, polyols and sugars. However, those skilled in the art will understand that the considerations described herein and further exemplified by reference to specific excipients are equally applicable to all types and combinations of excipients including, for example, salts, amino acids, other tonicity agents, surfactants, stabilizers, bulking agents, cryoprotectants, lyoprotectants, anti-oxidants, metal ions, chelating agents and/or preservatives.

Further, where a particular excipient is reported in molar concentration, those skilled in the art will recognize that the equivalent percent (%) w/v (*e.g.,* (grams of substance in a solution sample/mL of solution) X 100%) of solution is also contemplated.

Of course, a person having ordinary skill in the art would recognize that the concentrations of the excipients described herein share an interdependency within a particular formulation. By way of example, the concentration of a bulking agent may be lowered where, *e.g.,* there is a high protein concentration or where, *e.g.,* there is a high stabilizing agent concentration. In addition, a person having ordinary skill in the art would recognize that, in order to maintain the isotonicity of a particular formulation in which there is no bulking agent, the concentration of a stabilizing agent would be adjusted accordingly (*e.g.,* a "tonicifying" amount of stabilizer would be used). Common excipients are known in the art and can be found in Powell et al., Compendium of Excipients for Parenteral Formulations (1998), PDA J. Pharm. Sci. Technology, 52:238-311.

### c. Pharmaceutical Buffers and Buffering Agents

The stability of a pharmacologically active protein formulation is usually observed to be maximal in a narrow pH range. This pH range of optimal stability needs to be identified early during pre-formulation studies. Several approaches, such as accelerated stability studies and calorimetric screening studies, are useful in this endeavor (Remmele R.L. Jr., et al., Biochemistry, 38(16): 5241-7 (1999)). Once a formulation is finalized, the protein must be manufactured and maintained throughout its shelf-life. Hence, buffering agents are almost always employed to control pH in the formulation.

The buffer capacity of the buffering species is maximal at a pH equal to the pKa and decreases as pH increases or decreases away from this value. Ninety percent of the buffering capacity exists within one pH unit of its pKa. Buffer capacity also increases proportionally with increasing buffer concentration.

Several factors need to be considered when choosing a buffer. First and foremost, the buffer species and its concentration need to be defined based on its pKa and the desired formulation pH. Equally important is to ensure that the buffer is compatible with the protein and other formulation excipients, and does not catalyze any degradation reactions. A third important aspect to be considered is the sensation of stinging and irritation the buffer may induce upon administration. For example, citrate is known to cause stinging upon injection (Laursen T, et al., Basic Clin Pharmacol Toxicol., 98(2): 218-21 (2006)). The potential for stinging and irritation is greater for drugs that are administered via the subcutaneous (SC) or intramuscular (IM) routes, where the drug solution remains at the site for a relatively longer period of time than when administered by the IV route where the formulation gets diluted rapidly into the blood upon administration. For formulations that are administered by direct IV infusion, the total amount of buffer (and any other formulation component) needs to be monitored. One has to be particularly careful about potassium ions administered in the form of the potassium phosphate buffer, which can induce cardiovascular effects in a patient (Hollander-Rodriguez JC, et al., Am. Fam. Physician., 73(2): 283-90 (2006)).

Buffers for lyophilized formulations need additional consideration. Some buffers like sodium phosphate can crystallize out of the protein amorphous phase during freezing resulting in shifts in pH. Other common buffers such as acetate and imidazole may sublime or evaporate during the lyophilization process, thereby shifting the pH of formulation during lyophilization or after reconstitution.

The buffer system present in the compositions is selected to be physiologically compatible and to maintain a desired pH of the pharmaceutical formulation. In one embodiment, the pH of the solution is between pH 2.0 and pH 12.0. For example, the pH of the solution may be 2.0, 2.3, 2.5, 2.7, 3.0, 3.3, 3.5, 3.7, 4.0, 4.3, 4.5, 4.7, 5.0, 5.3, 5.5, 5.7, 6.0, 6.3, 6.5, 6.7, 7.0, 7.3, 7.5, 7.7, 8.0, 8.3, 8.5, 8.7, 9.0, 9.3, 9.5, 9.7, 10.0, 10.3, 10.5, 10.7, 11.0, 11.3, 11.5, 11.7, or 12.0.

The pH buffering compound may be present in any amount suitable to maintain the pH of the formulation at a predetermined level. In one embodiment, the pH buffering concentration is between 0.1 mM and 500 mM. For example, it is contemplated that the pH buffering agent is at least 0.1, 0.5, 0.7, 0.8 0.9, 1.0, 1.2, 1.5, 1.7, 2, 3, 4, 5, 6, 7, 8, 9, 10, 11, 12, 13, 14, 15, 16, 17, 18, 19, 20, 25, 30, 40, 50, 60, 70, 80, 90, 100, 200, or 500 mM.

Exemplary pH buffering agents used to buffer the formulation as set out herein include, but are not limited to organic acids, glycine, histidine, glutamate, succinate, phosphate, acetate, citrate, Tris, HEPES, and amino acids or mixtures of amino acids, including, but not limited to aspartate, histidine, and glycine. In one embodiment of the present invention, the buffering agent is citrate.

### d. Pharmaceutical Stabilizers and Bulking Agents

In one aspect of the present pharmaceutical formulations, a stabilizer (or a combination of stabilizers) is added to prevent or reduce storage-induced aggregation and chemical degradation. A hazy or turbid solution upon reconstitution indicates that the protein has precipitated or at least aggregated. The term "stabilizer" means an excipient capable of preventing aggregation or physical degradation, including chemical degradation (for example, autolysis, deamidation, oxidation, *etc.*) in an aqueous state. Stabilizers contemplated include, but are not limited to, sucrose, trehalose, mannose, maltose, lactose, glucose, raffinose, cellobiose, gentiobiose, isomaltose, arabinose, glucosamine, fructose, mannitol, sorbitol, glycine, arginine HCL, poly-hydroxy compounds, including polysaccharides such as dextran, starch, hydroxyethyl starch, cyclodextrins, N-methyl pyrollidene, cellulose and hyaluronic acid, sodium chloride, (Carpenter et al., Develop. Biol. Standard 74:225, (1991)). In the present formulations, the stabilizer is incorporated in a concentration of about 0.1, 0.5, 0.7, 0.8 0.9, 1.0, 1.2, 1.5, 1.7, 2, 3, 4, 5, 6, 7, 8, 9, 10, 11, 12, 13, 14, 15, 16, 17, 18, 19, 20, 30, 40, 50, 60, 70, 80, 90, 100, 200, 500, 700, 900, or 1000 mM. In one embodiment of the present invention, mannitol and trehalose are used as stabilizing agents.

If desired, the formulations also include appropriate amounts of bulking and osmolality regulating agents. Bulking agents include, for example and without limitation, mannitol, glycine, sucrose, polymers such as dextran, polyvinylpyrolidone, carboxymethylcellulose, lactose, sorbitol, trehalose, or xylitol. In one embodiment, the bulking agent is mannitol. The bulking agent is incorporated in a concentration of about 0.1, 0.5, 0.7, 0.8 0.9, 1.0, 1.2, 1.5, 1.7, 2, 3, 4, 5, 6, 7, 8, 9, 10, 11, 12, 13, 14, 15, 16, 17, 18, 19, 20, 30, 40, 50, 60, 70, 80, 90, 100, 200, 500, 700, 900, or 1000 mM.

### e. Pharmaceutical Surfactants

Proteins have a high propensity to interact with surfaces making them susceptible to adsorption and denaturation at air-liquid, vial-liquid, and liquid-liquid (silicone oil) interfaces. This degradation pathway has been observed to be inversely dependent on protein concentration and results in either the formation of soluble and insoluble protein aggregates or the loss of protein from solution via adsorption to surfaces. In addition to container surface adsorption, surface-induced degradation is exacerbated with physical agitation, as would be experienced during shipping and handling of the product.

Surfactants are commonly used in protein formulations to prevent surface-induced degradation. Surfactants are amphipathic molecules with the capability of out-competing proteins for interfacial positions. Hydrophobic portions of the surfactant molecules occupy interfacial positions (*e.g.,* air/liquid), while hydrophilic portions of the molecules remain oriented towards the bulk solvent. At sufficient concentrations (typically around the detergent's critical micellar concentration), a surface layer of surfactant molecules serves to prevent protein molecules from adsorbing at the interface. Thereby, surface-induced degradation is minimized. Surfactants contemplated herein include, without limitation, fatty acid esters of sorbitan polyethoxylates, *e.g.,* polysorbate 20 and polysorbate 80. The two differ only in the length of the aliphatic chain that imparts hydrophobic character to the molecules, C-12 and C-18, respectively. Accordingly, polysorbate-80 is more surface- active and has a lower critical micellar concentration than polysorbate-20.

Detergents can also affect the thermodynamic conformational stability of proteins. Here again, the effects of a given detergent excipient will be protein specific. For example, polysorbates have been shown to reduce the stability of some proteins and increase the stability of others. Detergent destabilization of proteins can be rationalized in terms of the hydrophobic tails of the detergent molecules that can engage in specific binding with partially or wholly unfolded protein states. These types of interactions could cause a shift in the conformational equilibrium towards the more expanded protein states (*e.g.* increasing the exposure of hydrophobic portions of the protein molecule in complement to binding polysorbate). Alternatively, if the protein native state exhibits some hydrophobic surfaces, detergent binding to the native state may stabilize that conformation.

Another aspect of polysorbates is that they are inherently susceptible to oxidative degradation. Often, as raw materials, they contain sufficient quantities of peroxides to cause oxidation of protein residue side-chains, especially methionine. The potential for oxidative damage arising from the addition of stabilizer emphasizes the point that the lowest effective concentrations of excipients should be used in formulations. For surfactants, the effective concentration for a given protein will depend on the mechanism of stabilization.

Surfactants are also added in appropriate amounts to prevent surface related aggregation phenomenon during freezing and drying (Chang, B, J. Pharm. Sci. 85:1325, (1996)). Thus, exemplary surfactants include, without limitation, anionic, cationic, nonionic, zwitterionic, and amphoteric surfactants including surfactants derived from naturally-occurring amino acids. Anionic surfactants include, but are not limited to, sodium lauryl sulfate, dioctyl sodium sulfo succinate and dioctyl sodium sulfonate, chenodeoxycholic acid, N-lauroylsarcosine sodium salt, lithium dodecyl sulfate, 1-octanesulfonic acid sodium salt, sodium cholate hydrate, sodium deoxycholate, and glycodeoxycholic acid sodium salt. Cationic surfactants include, but are not limited to, benzalkonium chloride or benzethonium chloride, cetylpyridinium chloride monohydrate, and hexadecyltrimethylammonium bromide. Zwitterionic surfactants include, but are not limited to, CHAPS, CHAPSO, SB3-10, and SB3-12. Non-ionic surfactants include, but are not limited to, digitonin, Triton X-100, Triton X-114, TWEEN-20, and TWEEN-80. Surfactants also include, but are not limited to lauromacrogol 400, polyoxyl 40 stearate, polyoxyethylene hydrogenated castor oil 10, 40, 50 and 60, glycerol monostearate, polysorbate 40, 60, 65 and 80, soy lecithin and other phospholipids such as dioleyl phosphatidyl choline (DOPC), dimyristoylphosphatidyl glycerol (DMPG), dimyristoylphosphatidyl choline (DMPC), and (dioleyl phosphatidyl glycerol) DOPG; sucrose fatty acid ester, methyl cellulose and carboxymethyl cellulose. Compositions comprising these surfactants, either individually or as a mixture in different ratios, are therefore further provided. In one embodiment of the present invention, the surfactant is TWEEN-80. In the present formulations, the surfactant is incorporated in a concentration of about 0.005 to about 1.0 g/L or about 0.01 to about 0.5 g/L. In formulations provided, the surfactant concentration is 0.005, 0.01, 0.02, 0.03, 0.05, 0.06, 0.07, 0.08, 0.09, 0.1, 0.2, 0.3, 0.4, 0.5, 0.6, 0.7, 0.8, 0.9 or 1.0 g/L.

### f. Pharmaceutical Salts

Salts are often added to increase the ionic strength of the formulation, which can be important for protein solubility, physical stability, and isotonicity. Salts can affect the physical stability of proteins in a variety of ways. Ions can stabilize the native state of proteins by binding to charged residues on the protein's surface. Alternatively, salts can stabilize the denatured state by binding to peptide groups along the protein backbone (-CONH-). Salts can also stabilize the protein native conformation by shielding repulsive electrostatic interactions between residues within a protein molecule. Salts in protein formulations can also shield attractive electrostatic interactions between protein molecules that can lead to protein aggregation and insolubility. In formulations provided, the salt concentration is between 0.1, 1, 10, 20, 30, 40, 50, 80, 100, 120, 150, 200, 300, and 500 mM. In some embodiments, the salt concentration is about 0.1 mM to about 500 mM or about 1 mM to about 500 mM or about 1 mM or abut 400 mM.

### g. Other Common Excipient Components: Pharmaceutical Amino Acids

Amino acids have found versatile use in protein formulations as buffers, bulking agents, stabilizers and antioxidants. Thus, in one aspect histidine and glutamic acid are employed to buffer protein formulations in the pH range of 5.5 - 6.5 and the pH range of 4.0 - 5.5 respectively. The imidazole group of histidine has a pKa = 6.0 and the carboxyl group of glutamic acid side chain has a pKa of 4.3 which makes these amino acids suitable for buffering in their respective pH ranges. Glutamic acid is particularly useful in such cases. Histidine is commonly found in marketed protein formulations, and this amino acid provides an alternative to citrate, a buffer known to sting upon injection. Interestingly, histidine has also been reported to have a stabilizing effect, with respect to aggregation when used at high concentrations in both liquid and lyophilized presentations (Chen B, et al., Pharm Res., 20(12): 1952-60 (2003)). Histidine was also observed by others to reduce the viscosity of a high protein concentration formulation. However, in the same study, the authors observed increased aggregation and discoloration in histidine containing formulations during freeze-thaw studies of the antibody in stainless steel containers. Another note of caution with histidine is that it undergoes photo-oxidation in the presence of metal ions (Tomita M, et al., Biochemistry, 8(12): 5149-60 (1969)). The use of methionine as an antioxidant in formulations appears promising; it has been observed to be effective against a number of oxidative stresses (Lam XM, et al., JPharm ScL, 86(11): 1250-5 (1997)).

In various aspects, formulations are provided which include one or more of the amino acids glycine, proline, serine, arginine and alanine have been shown to stabilize proteins by the mechanism of preferential exclusion. Glycine is also a commonly used bulking agent in lyophilized formulations. Arginine has been shown to be an effective agent in inhibiting aggregation and has been used in both liquid and lyophilized formulations. In formulations provided, the amino acid concentration is between 0.1, 1, 10, 20, 30, 40, 50, 80, 100, 120, 150, 200, 300, and 500 mM. In some embodiments, the amino acid concentration is about 0.1 mM to about 500 mM, about 1 mM to about 500 mM, about 0.1 mM to about 400 mM, 0.4 mM to about 400 mM, or about 0.4 mM to about 300 mM. In one embodiment of the present invention, the amino acid is glycine.

### h. Other Common Excipient Components: Pharmaceutical Antioxidants

Oxidation of protein residues arises from a number of different sources. Beyond the addition of specific antioxidants, the prevention of oxidative protein damage involves the careful control of a number of factors throughout the manufacturing process and storage of the product such as atmospheric oxygen, temperature, light exposure, and chemical contamination. The invention therefore contemplates the use of the pharmaceutical antioxidants including, without limitation, reducing agents, oxygen/free-radical scavengers, or chelating agents. Antioxidants in therapeutic protein formulations are, in one aspect, water-soluble and remain active throughout the product shelf -life. Reducing agents and oxygen/free-radical scavengers work by ablating active oxygen species in solution. Chelating agents such as EDTA are effective by binding trace metal contaminants that promote free-radical formation. For example, EDTA was utilized in the liquid formulation of acidic fibroblast growth factor to inhibit the metal ion catalyzed oxidation of cysteine residues.

In addition to the effectiveness of various excipients to prevent protein oxidation, the potential for the antioxidants themselves to induce other covalent or physical changes to the protein is of concern. For example, reducing agents can cause disruption of intramolecular disulfide linkages, which can lead to disulfide shuffling. In the presence of transition metal ions, ascorbic acid and EDTA have been shown to promote methionine oxidation in a number of proteins and peptides (Akers MJ, and Defelippis MR. Peptides and Proteins as Parenteral Solutions. In: Pharmaceutical Formulation Development of Peptides and Proteins. Sven Frokjaer, Lars Hovgaard, editors. Pharmaceutical Science. Taylor and Francis, UK (1999)); Fransson J.R., et al., Pharm. Sci. 86(9): 4046-1050 (1997); Yin J, et al., Pharm Res., 21(12): 2377-83 (2004)). Sodium thiosulfate has been reported to reduce the levels of light and temperature induced methionine-oxidation in rhuMab HER2; however, the formation of a thiosulfate-protein adduct was also reported in this study (Lam XM, Yang JY, et al., JPharm Sci. 86(11): 1250-5 (1997)). Selection of an appropriate antioxidant is made according to the specific stresses and sensitivities of the protein. Antioxidants contemplated in certain aspects include, without limitation, reducing agents and oxygen/free-radical scavengers, EDTA, and sodium thiosulfate.

### i. Other Common Excipient Components: Pharmaceutical Metal Ions

In general, transition metal ions are undesired in protein formulations because they can catalyze physical and chemical degradation reactions in proteins. However, specific metal ions are included in formulations when they are co-factors to proteins and in suspension formulations of proteins where they form coordination complexes (*e.g.,* zinc suspension of insulin). Recently, the use of magnesium ions (10 mM-120 mM) has been proposed to inhibit the isomerization of aspartic acid to isoaspartic acid (WO 2004039337).

Two examples where metal ions confer stability or increased activity in proteins are human deoxyribonuclease (rhDNase, Pulmozyme^{®}), and Factor VIII. In the case of rhDNase, Ca⁺² ions (up to 100 mM) increased the stability of the enzyme through a specific binding site (Chen B, et al., / Pharm Sci., 88(4): 477-82 (1999)). In fact, removal of calcium ions from the solution with EGTA caused an increase in deamidation and aggregation. However, this effect was observed only with Ca⁺² ions; other divalent cations Mg⁺², Mn⁺² and Zn⁺² were observed to destabilize rhDNase. Similar effects were observed in Factor VIII. Ca⁺² and Sr⁺² ions stabilized the protein while others like Mg⁺², Mn⁺² and Zn⁺², Cu⁺² and Fe⁺² destabilized the enzyme (Fatouros, A., et al., Int. J. Pharm., 155, 121-131 (1997). In a separate study with Factor VIII, a significant increase in aggregation rate was observed in the presence of Al⁺³ ions (Derrick TS, et al., Pharm. Sci., 93(10): 2549-57 (2004)). The authors note that other excipients like buffer salts are often contaminated with Al⁺³ ions and illustrate the need to use excipients of appropriate quality in formulated products.

### j. Other Common Excipient Components: Pharmaceutical Preservatives

Preservatives are necessary when developing multi-use parenteral formulations that involve more than one extraction from the same container. Their primary function is to inhibit microbial growth and ensure product sterility throughout the shelf- life or term of use of the drug product. Commonly used preservatives include, without limitation, benzyl alcohol, phenol and m-cresol. Although preservatives have a long history of use, the development of protein formulations that includes preservatives can be challenging. Preservatives almost always have a destabilizing effect (aggregation) on proteins, and this has become a major factor in limiting their use in multi-dose protein formulations (Roy S, et al., J Pharm ScL, 94(2): 382-96 (2005)).

To date, most protein drugs have been formulated for single-use only. However, when multi-dose formulations are possible, they have the added advantage of enabling patient convenience, and increased marketability. A good example is that of human growth hormone (hGH) where the development of preserved formulations has led to commercialization of more convenient, multi-use injection pen presentations. At least four such pen devices containing preserved formulations of hGH are currently available on the market. Norditropin^{®} (liquid, Novo Nordisk), Nutropin AQ^{®} (liquid, Genentech) & Genotropin (lyophilized - dual chamber cartridge, Pharmacia & Upjohn) contain phenol while Somatrope^{®} (Eli Lilly) is formulated with m-cresol.

Several aspects need to be considered during the formulation development of preserved dosage forms. The effective preservative concentration in the drug product must be optimized. This requires testing a given preservative in the dosage form with concentration ranges that confer anti-microbial effectiveness without compromising protein stability. For example, three preservatives were successfully screened in the development of a liquid formulation for interleukin-1 receptor (Type I), using differential scanning calorimetry (DSC). The preservatives were rank ordered based on their impact on stability at concentrations commonly used in marketed products (Remmele RL Jr., et al., Pharm Res., 15(2): 200-8 (1998)).

Development of liquid formulations containing preservatives are more challenging than lyophilized formulations. Freeze-dried products can be lyophilized without the preservative and reconstituted with a preservative containing diluent at the time of use. This shortens the time for which a preservative is in contact with the protein significantly minimizing the associated stability risks. With liquid formulations, preservative effectiveness and stability have to be maintained over the entire product shelf-life (e.g., 18-24 months). An important point to note is that preservative effectiveness has to be demonstrated in the final formulation containing the active drug and all excipient components.

Some preservatives can cause injection site reactions, which is another factor that needs consideration when choosing a preservative. In clinical trials that focused on the evaluation of preservatives and buffers in Norditropin, pain perception was observed to be lower in formulations containing phenol and benzyl alcohol as compared to a formulation containing m-cresol (Kappelgaard A.M., Horm Res., 62 Suppl 3:98-103 (2004)). Interestingly, among the commonly used preservative, benzyl alcohol possesses anesthetic properties (Minogue SC, and Sun DA., Anesth Analg., 100(3): 683-6 (2005)). In various aspects the use of preservatives provide a benefit that outweighs any side effects.

### k. Methods of Preparation of Pharmaceutical Formulations

The present invention further contemplates methods for the preparation of pharmaceutical formulations.

The present methods further comprise one or more of the following steps: adding a stabilizing agent as described herein to said mixture prior to lyophilizing, adding at least one agent selected from a bulking agent, an osmolality regulating agent, and a surfactant, each of which as described herein, to said mixture prior to lyophilization.

### l. Exemplary rVWF Formulation for Administration

In some embodiments, the present methods provide for an enhanced formulation that allows a final product with high potency (high rVWF concentration and enhanced long term stability) in order to reduce the volume for the treatment (100 IU/ml to 10,000 IU/ml). In some embodiments, the rVWF concentration in the formulation for administration is about 100 IU/ml to 10,000 IU/ml. In some embodiments, the rVWF concentration in the formulation for administration is about 500 IU/ml to 10,000 IU/ml. In some embodiments, the rVWF concentration in the formulation for administration is about 1,000 IU/ml to 10,000 IU/ml. In some embodiments, the rVWF concentration in the formulation for administration is about 2,000 IU/ml to 10,000 IU/ml. In some embodiments, the rVWF concentration in the formulation for administration is about 3,000 IU/ml to 10,000 IU/ml. In some embodiments, the rVWF concentration in the formulation for administration is about 4,000 IU/ml to 10,000 IU/ml. In some embodiments, the rVWF concentration in the formulation for administration is about 5,000 IU/ml to 10,000 IU/ml. In some embodiments, the rVWF concentration in the formulation for administration is about 6000 IU/ml to 10000 IU/ml. In some embodiments, the rVWF concentration in the formulation for administration is about 7000 IU/ml to 10000 IU/ml. In some embodiments, the rVWF concentration in the formulation for administration is about 8,000 IU/ml to 10,000 IU/ml. In some embodiments, the rVWF concentration in the formulation for administration is about 9,000 IU/ml to 10,000 IU/ml.

In some embodiments, the formulation for administration comprises one or more zwitterionic compounds, including for example, amino acids like histidine, glycine, and arginine. In some embodiments, the formulation for administration comprises a component with amphipathic characteristic having a minimum of one hydrophobic and one hydrophilic group, including for example polysorbate 80, octylpyranosid, dipeptides, and/or amphipathic peptides. In some embodiments, the formulation for administration comprises a nonreducing sugar or sugar alcohol or disaccharides, including for example, sorbitol, mannitol, sucrose, or trehalose. In some embodiments, the formulation for administration comprises a nontoxic water soluble salt, including for example, sodium chloride, that results in a physiological osmolality. In some embodiments, the formulation for administration comprises a pH in a range from 6.0 to 8.0. In some embodiments, the formulation for administration comprises a pH of about 6.0, about 6.5, about 7, about 7.5 or about 8.0. In some embodiments, the formulation for administration comprises one or more bivalent cations that stabilize rVWF, including for example, Ca²⁺, Mg²⁺, Zn²⁺, Mn²⁺ and/or combinations thereof. In some embodiments, the formulation for administration comprises about 1 mM to about 50 mM glycine, about 1 mM to about 50 mM histidine, about zero to about 300 mM sodium chloride (*e.g.,* less than 300 mM sodium), about 0.01 % to about 0.05% polysorbate 20 (or polysorbate 80), and about 0.5 % to about 20% (w/w) sucrose with a pH of about 7.0 and having a physiological osmolarity at the time point of administration.

In some embodiments, the formulation for administration can be freeze dried. In some embodiments, the formulation for administration is stable and can be stored in liquid state at about 2°C to about 8°C, as well as at about 18°C to about 25°C. In some embodiments, the formulation for administration is stable and can be stored in liquid state at about 2°C to about 8°C. In some embodiments, the formulation for administration is stable and can be stored in liquid state at about 18°C to about 25°C.

### EXAMPLES

### EXAMPLE 1: EFFECTS OF VON WILLEBRAND FACTOR (VWF) BOUND TO CLQ ON HUMAN PHAGOCYTES

### Background

Nowadays, atherosclerosis is widely accepted to be a chronic inflammatory disease (Ross 1999) involving a complex interplay between the immune and hemostatic systems driving the genesis and exacerbations of atherosclerosis. Cells and proteins of the immune system are found in all stages of atherosclerosis, including the early stage fatty streaks (sites of accumulation of lipids) with lipid-laden macrophages known as foam cells which are the consequence of a disrupted balance between macrophage uptake and efflux of cholesterol. Diverse roles of the complement system as part of the innate immune system have also become evident over the past years. In advanced stages of atherosclerosis, complement activation - in particular when involving the terminal pathway - seems to lead to an inflammatory response, contributing to disease pathology. In contrast, in early atherosclerotic lesions in the absence of, or prior to, expression of other complement components, observational and experimental studies suggest that C1q (as well as the closely related mannose-binding lectin (MBL) and early downstream components of the classical pathway) plays a protective role (Patzelt 2015).

C1q (and MBL) is an activation ligand for phagocytosis triggering enhanced phagocytic activity when bound to a particle to be ingested, or presented to the phagocyte in a multivalent manner, such as when immobilized on a surface. In the context of atherosclerosis, this function is of relevance with regard to the clearance of apoptotic cell debris as well as for the removal and metabolism of atherogenic forms of LDL (Fraser 2010, Samstad 2014). Once having bound, C1q is able to directly modulate the phagocyte activation (Fraser 2010, Benoit 2012). However, while bound Clq alone is inducing an anti-inflammatory (M2-like) macrophage phenotype, the additional presence of autoantibodies against C1q (anti-C1q), as occurring in systemic autoimmunity, can invert this character into a proinflammatory (Ml-like) phenotype (Thanei 2016). Thus, considering the well-established role of macrophages in atherosclerosis, the interplay between macrophages, C1q and molecules that secondarily bind to C1q requires further studies.

In this context it is important to note that components of the hemostatic system, that are known to be involved in atherosclerosis, have been shown to interact with complement C1q. Of these components of hemostasis, platelets as well as the von Willebrand factor (vWF) seem to be of particular importance. Activated platelets seem to have proatherosclerotic as well as modulating and tissue/vascular remodelling effects that are influenced by the presence of C1q at sites of vascular injury and inflammation. In line with these observations, platelets have been described to express several complement receptors including those for C1q (Peerschke 2001) and to be able to induce classical pathway activation with consecutive surface deposition of C1q.

With regard to vWF, this molecule is well known to be an important ligand for platelets and has been implicated in the pathogenesis of atherosclerosis as well (van Galen 2012). vWF is a large multimeric glycoprotein that has two critical functions in hemostasis, acting as a bridging molecule for normal platelet adhesion and aggregation, and serving as a carrier protein for factor VIII in the circulation. VWF is synthesized by endothelial cells (about 90% of circulating vWF) and megakaryocytes. After synthesis the vWF subunits (250kD) form dimers, which then assemble to large vWF multimers consisting of up to >40 vWF monomers that are stored in specialized organelles. Upon release into circulation these large vWF multimers mediate platelet adhesion and aggregation at sites of vascular injury, a process that is regulated by proteolysis of vWF by ADAMTS13, the vWF-cleaving protease. The larger vWF multimers have a higher thrombogenic potential due to the presence of multiple sites of interaction for platelets and subendothelial collagen.

We have shown that bound C1q allows additional binding of vWF in particular when occurring under flow conditions and this binding induces platelet rolling and adhesion. The binding of vWF to C1q could specifically be inhibited by Fab anti-C1q and C1q-derived peptides (in particular by peptide A08 that is a positively charged epitope for anti-Clq on the collagen-like region of C1q) suggesting that vWF binds to a cryptic epitope on the N-terminal part of the collagen-like region (CLR) of C1q. Binding to the CLR of C1q could be confirmed using fragments of C1q obtained by limited enzymatic digestion. Under shear stress, the C1q-vWF interaction was strongly enhanced, resembling the binding of vWF to collagen I, and could be observed as early as 10 seconds after the start of perfusion (FIG. 1A and FIG. 1B).

In addition, we could show that C1q vWF complexes induced platelet rolling and fim1 adhesion that was not observed with C1q alone. VWF bound to C1q was more efficient in platelet recruitment than equal amounts of vWF being coated alone or via an anti-vWF antibody. Furthermore, we observed vWF binding to C1q-positive apoptotic microparticles (FIG. 2A-FIG. 2E), to C1q-positive cholesterol crystals and increased vWF deposition in C1q-positive glomeruli of SLE patients compared to control nephropathy (FIG. 2A-FIG. 2E).

Interestingly, the interaction between C1q and vWF does also occur on the surface of cholesterol crystals. These observations strongly suggest a complex interplay between C1q and other factors that have been shown to be implicated in atherosclerosis, *i. e.* macrophages, platelets and vWF.

Taken together, we showed for the first time the binding of vWF to complement C1q and thus a direct interaction between starter molecules of hemostasis and the classical pathway of complement. This direct interaction is likely to contribute to the pathogenic mechanisms in complement-mediated, inflammatory diseases (Kölm et al., J Immunol, 2016, 197:3669-3679). In principal, this binding could lead to pro-, anti-inflammatory or immunomodulatory effects. In this respect, the interference of vWF-binding with the recognition of C1q by macrophages is of particular interest.

### Hypothesis

VWF bound to complement C1q modulates the immune response of macrophages.

### Aim

To elucidate the interplay between C1q and hemostasis in particular with regard to mechanisms being associated with atherosclerosis and thromboembolism. More specifically, we want to study the effect of vWF binding to C1q on the phenotype of macrophages.

### Data and Study Plan

Macrophages were obtained from PBMCs derived from healthy blood donors recruited at the Blood Donation Centre in Basel, CH. The interaction of vWF bound to C1q with phagocytes were solely studied by in vitro laboratory experiments. No application to human subjects was performed. Recombinant VWF (rVWF) was used in this study.

All three, complement C1q, macrophages and vWF, have been implicated in the pathogenesis of atherosclerosis. However, while the interplay between C1q and macrophages (Fraser 2010, Benoit 2012), between C1q and vWF as well as between vWF and macrophages (van Schooten 2008, Castro-Nunez 2012) has been described, the interplay between all three, *i.e*., vWF bound to C1q with macrophages remains to be determined. More specifically, vWF bound to C1q might affect the Clq-induced macrophage phenotype. This assumption is supported by morphological changes we observed during experiments exposing HMDMs to Clq alone or Clq-vWF complexes (FIG. 3A and FIG. 3B).

This observation was extended by staining for surface markers, cytokine release and phagocytic capacity. In addition, the interaction of peripheral blood monocytes with C1q and C1q-vWF complexes was studied under flow conditions which have been shown to be of relevance for the interaction between macrophages and vWF (Castro-Nunez 2012). Last, the interaction between Clq-vWF complexes and macrophages was investigated, and then analyses was performed using a more functional assay exposing macrophages to cholesterol crystals (coated with either C1q alone or vWF- complexed with C1q) and/or apoptotic microparticles. This is of importance since both, cholesterol crystals (Samstad 2014) as well as apoptotic cell debris have been shown to bind C1q with consecutive complement activation, and their clearance mechanisms seem to be of importance in the pathogenesis of atherosclerosis.

### References

Benoit ME, Clarke EV, Morgado EV, Fraser DA, Tenner AJ. Complement protein Clq directs macrophage polarisation and limits inflammasome activity during the uptake of apoptotic cells. J Immunol 2012; 188: 5682-5693.
Castro-NU:fiez L, Dienava-Verdoold I, Herczenik E, Mertens K, Meijer AB. Shear stress is required for the endocytic uptake of the factor VIII-von Willebrand factor complex by macrophages. J Thromb Haemost 2012; 10: 1929-1937
Fraser DA, Tenner AJ. Innate immune proteins Clq and mannan-binding lectin enhance clearance of atherogenic lipoproteins by human monocytes and macrophages. J Immunol 2010; 185: 3932-9.
Kölm et al., J Immunol, 2016, 197:3669-3679.
Patzelt J. Verschoor A, Langer HF. Platelets and the complement cascade in atherosclerosis. Front Physiol 2015; 6:49.
Peerschke EI, Ghebrehiwet B. Human blood platelet gClqR/p33. Immunol Rev 2001; 180: 56-64.
Ross R. Atherosclerosis-an inflammatory disease. N Engl J Med 1999; 340: 115-126.
Samstad EQ, Niyonzima N, Nymo S. Aune MH, Ryan L. Bakke SS, Lappegard KT, Brekke OL, Lambris JD, Damas JK, Latz E, Mollnes TE, Espevik T. Cholesterol crystals induce complement-dependent inflammasome activation and cytokine release J. Immunol 2014; 192: 2837-45.
Thanei S, Trendelenburg M. Anti-Clq Autoantibodies from Systemic Lupus Erythematosus Patients Induce a Proinflammatory Phenotype in Macrophages, J Immunol 2016; 196: 2063-74.
van Galen KP, Tuinenburg A, Smeets EM, Schutgens RE. Von Willebrand factor deficiency and atherosclerosis. Blood Rev 2012; 26: 189-96.
van Schooten CJ, Shahbazi S, GrootE, Oortwijn BD, van den Berg HM, Denis CV, Lenting PJ. Macrophages contribute to the cellular uptake of von Willebrand factor and factor VIII in vivo. Blood 2008; 112: 1704-1712.

### EXAMPLE 2: BINDING OF VON WILLEBRAND FACTOR TO COMPLEMENT C1Q DECREASES THE PHAGOCYTOSIS OF CHOLESTEROL CRYSTALS AND THE CONSECUTIVE IL-1 SECRETION IN MACROPHAGES

### Abstract

Complement C1q, an initiation molecule of the classical pathway, exerts versatile immunomodulatory functions independent of complement activation. Non-classical functions of C1q include the clearance of apoptotic cells and cholesterol crystals (CC) as well as the modulation of cytokine secretion by immune cells, such as macrophages. Moreover, C1q has been shown to mediate the binding of von Willebrand factor (vWF), initiation molecule of primary hemostasis. However, the consequences of this C1q-vWF interaction on the phagocytosis of CC by macrophages remained elusive. Therefore, we used CC-C1q-vWF complexes to study the immunological effects on human monocyte-derived macrophages (HMDMs). HMDMs were investigated by analyzing surface receptor expression, phagocytosis of CC complexes, cytokine secretion and caspase-1 activity. We found that vWF only bound to CC in a C1q-dependent manner. Exposure of macrophages to CC-C1q-vWF complexes resulted in an upregulated expression of phagocytosis-mediating receptors MerTK, LRP-1 and SR-A1 and costimulatory receptors CD14, LAIR1 and PD-L1 when compared to CC-C1q without vWF, but late and early phagocytosis of CC-C1q complexes was hampered in the presence of vWF. In addition, this resulted in a diminished caspase-1 activation and consecutive reduction in pro-inflammatory IL-1β cytokine secretion, IL-1β /IL- 1RA ratio and IL-1α/IL-1RA ratio. In conclusion, our results demonstrate that vWF binding to C1q was found to substantially modulate the effects of C1q on HMDMs. In this way, the C1q-vWF interaction might be beneficial in retarding foam cell formation and dampening inflammation (Donat et al., Frontiers in Immunology, 10 (2019) 1-11, Article 2712).

The complement system is a highly effective part of the innate immune system. The multiple functions of complement include defense against bacterial infections, bridging innate and adaptive immunity and the clearance of immune complexes and components of inflammation (Walport, 2001). The complement system can be activated through three distinct pathways: the classical, the lectin and the alternative pathways. All three pathways converge in a shared terminal response resulting in the formation of C5a and C3a as potent inflammatory effector molecules and C5b-9 as membrane attack complex. However, each pathway is initiated through different characteristic recognition molecules (Morgan and Harris, 2015). The initiation of the classical pathway is triggered by C1q through sensing of bound antibodies as well as pathogen- and damage-associated molecular patterns (PAMPs/DAMPs). In addition, more recent research has shown a number of functions for C1q that are independent of downstream complement activation (Nayak *et al*., 2010). On the one hand, opsonization with C1q enhances the clearance of diverse structures, namely immune complexes (Bobak *et al*., 1987) and apoptotic cells (Bohlson *et al*., 2007) as well as atherogenic lipoproteins (Fraser and Tenner, 2010) and cholesterol crystals (CC) (Samstad *et al*., 2014) by phagocytes. On the other hand, anti-inflammatory properties for C1q have been well described. For example, bound C1q decreases the release of pro-inflammatory cytokines and increases the production of anti-inflammatory mediators by phagocytes (Fraser *et al*., 2006; Fraser *et al*., 2009). Additionally, the presence of C1q on apoptotic cells skews macrophage polarization towards an anti-inflammatory phenotype (Benoit *et al*., 2012).

Apart from C1q's extensively studied involvement in immunity, a complex cross-talk between complement and coagulation is becoming ever more evident (Markiewski *et al*., 2007).

Complement components have been found to induce hemostasis and *vice versa* coagulation factors can trigger complement activation, therefore combining two powerful plasma cascades. Within the hemostatic cascade, von Willebrand factor (vWF) acts as an important starter molecule by mediating platelet adhesion and aggregation. Immune cells, such as macrophages, are competent to take up and clear vWF through scavenger receptors (van Schooten *et al*., 2008; Wohner *et al*., 2018). Moreover, vWF has been shown to interact with complement factor H (Feng *et al*., 2013; Turner and Moake, 2013) and therefore can modulate the activation of complement via the alternative pathway (Feng *et al*., 2015). Furthermore, a direct interaction between vWF and C1q was found by our group, demonstrating that C1q, bound to surfaces such as apoptotic cells and CC, acts as a binding partner for vWF (Kolm *et al*., 2016).

CC can be found as a characteristic feature in the intima of atherosclerotic arteries from early lesions to late plaque (Franklin *et al*., 2016) and are widely used as an *in vitro* model of atherosclerosis (Corr *et al*., 2016; Zimmer *et al*., 2016; Bakke *et al*., 2017). Formation of CC occurs upon fatty streak development by an increased uptake and exhausted efflux of cholesterol by lipid-laden macrophages known as foam cells. In *in vitro* and *in vivo* models of atherosclerosis, CC have been shown to be implicated in the activation of the NOD [nucleotide oligomerization domain], LRR [leucine-rich repeat]-, and PYD [pyrin domain]-containing protein 3 (NLRP3) inflammasome and downstream cytokine secretion, consequently triggering local and systemic inflammation (Abela, 2010; Duewell *et al*., 2010; Rajamaki *et al*., 2010). While the role of CC and macrophages in atherosclerosis appears unambiguous, C1q can play a dual role. On the one hand, C1q bound to oxidized low-density lipoproteins (LDL) or CC has been shown to activate the classical pathway, and in this context driving the progression of atherosclerosis in animal models (Schmiedt *et al*., 1998; Buono *et al*., 2002). On the other hand, C1q has also been described to be protective in early atherosclerosis *in vivo* (Bhatia *et al*., 2007; Lewis *et al*., 2009) and to increase cholesterol efflux transporter expression *in vitro* (Fraser and Tenner, 2010), hence revealing atheroprotective properties. Similarly, the role of vWF in atherosclerosis is still a matter of debate. Although various studies suggest that vWF deficiency provides protection from atherosclerosis in animals, in humans, an unequivocal protective effect of vWF deficiency on atherosclerosis has not been demonstrated so far (van Galen *et al*., 2012).

Taken together, CC, macrophages, C1q and vWF have all been implicated in atherosclerosis. Nevertheless, the consequences of the interaction between C1q and vWF, especially on phagocytes, remain to be determined. In order to better understand this interaction, the aim of our study was to investigate the immunological effect of complexes consisting of cholesterol crystals, C1q and von Willebrand factor (CC-C1q-vWF complexes) by studying receptor expression, phagocytosis and cytokine secretion of macrophages.

### Material and Methods

### Preparation of CC

Cholesterol (suitable for cell culture, Sigma Aldrich, St. Louis, MO, USA) was dissolved in 95% ethanol at 60°C (12.5g/l), sterile filtered and allowed to crystallize at room temperature (RT) for 7 days (d). Excess amount of liquid was removed from the suspension, followed by drying for 5 d. Finally, CC were grinded and stored at -20°C until further use.

### Preparation of CC, CC-C1q and CC-C1q-vWF Complexes for Characterization of C1q and vWF Binding

CC were suspended in PBS (Life Technology, Carlsbad, CA, USA) and sonicated. For generation of CC-C1q complexes, 50 µg/ml purified C1q (Complement Technology, Tyler, Tx, USA) was added and incubated for 1 hour (hr) at RT on a shaker. For generation of CC- C1q-vWF complexes, 10 µg/ml recombinant vWF (provided by Shire, Lexington, MA, USA; characterization described in Turecek et al., Himostaseologie, 2009, 29 (suppl. 1): S32-38 was added to PBS-washed CC-C1q complexes, vortexed rigorously and further incubated for 1 hr at RT on a shaker. CC complexes were further incubated with monoclonal mouse anti- C1q (clone 32A6 (Bigler *et al*., 2009)) or polyclonal rabbit anti-vWF (Abcam, Cambridge, UK) for 1 hr at RT on a shaker. Secondary antibody staining was performed with donkey anti-mouse IgG-AlexaFluor (AF)555 (Life Technology) and goat anti-rabbit IgG-AF647 (Abcam) in PBS/1% BSA (Sigma Aldrich)/0.5 M NaCl for 30 min at 4°C in the dark. For flow cytometry, data were acquired using a BD Accuri 6 (BD Biosciences, San Jose, CA, USA) and analyzed with FlowJo10. For confocal microcopy, CC were spun onto cytoslides (Shandon, Pittsburg, PA, USA) by a Cytospin centrifuge (Thermo Fisher Scientific, Waltham, MA, USA) and analyzed using Nikon A1R Nala (Nikon, Tokyo, Japan) and NIS software. For imaging flow cytometry, analyses were carried out using ImageStreamX Mark II and IDEAS software (both EMD Millipore, Billerica, MA, USA).

### Cell Culture

Peripheral blood mononuclear cells were isolated from fresh buffy coats (Blood Transfusion Centre of the University Hospital Basel, Basel, Switzerland) by Ficoll gradient centrifugation using Lymphoprep (Stemcell Technologies, Vancouver, Canada). Monocytes were obtained by CD14+ magnetic-activated cell sorting beads (Miltenyi, Bergisch Gladbach, Germany) according to the manufacturer's instructions (yielding an average purity of 95-98% CD14+ monocytes determined by flow cytometry). Monocytes were differentiated into human monocyte-derived macrophages (HMDMs), cultured in DMEM supplemented with 1% penicillin/streptomycin (DMEM+), 10% fetal calf serum (FCS) (all from Life Technology) and 50 ng/ml GM-CSF (Immunotools, Frisoythe, Germany) at a cell concentration of 0.5×10⁵ cells/ml in 6-well plates (BD Biosciences, Franklin Lakes, NJ, USA) and maintained in 5% CO2 at 37 °C for 7 days.

### Treatment with CC Complexes

After 7 d, HMDMs were washed with prewarmed DMEM+, optionally stimulated with 100 ng/ml lipopolysaccharide (LPS) (E. coli O127:B8, Sigma Aldrich) and treated with 0.5 mg/ml CC, CC-C1q or CC-C1q-vWF complexes for indicated time points. Complexes were prepared as described above, washed with PBS and resuspended in DMEM+ before adding to cells.

### Surface Receptor Expression

HMDMs were stimulated with LPS and treated with CC, CC-C1q or CC-C1q-vWF complexes as described above. After 18 hr, HMDMs were washed with PBS and incubated with PBS/10 mM EDTA (AppliChem, Darmstadt, Germany) for 30 min at 4°C. Cells were collected in FACS buffer (PBS/0,1% FCS/1 mM EDTA) and resuspended at a cell concentration of 5×10⁵ cells/100 µl and incubated with 2 µg/ml of human IgG for 45 min at 4°C to block unspecific binding of antibodies to Fcy receptors. Staining was performed for 30 min at 4°C in the dark 160 in PBS using the following antibodies: anti-MHC II-FITC (Immunotools), anti-tyrosine-protein-kinase Mer (MerTK)-PE (R&D Systems, Minneapolis, MN, USA), anti-programmed death ligand 1 (PD-L1/CD274)-APC and anti-CD14-PeCy7 (both from Biolegend, San Diego, CA, USA) (antibody panel 1) or anti-CD86-FITC (Biolegend), anti- lipoprotein receptor-related protein 1 (LRP-1/CD91)-PE (Thermo Fisher Scientific, Waltham, MA, USA), anti-leukocyte-associated immunoglobulin-like receptor 1 (LAIR1/CD305)-AF647 and anti-scavenger receptor A 1 (SR-A1/CD204)-PeCy7 (both from Biolegend) (antibody panel 2).

HMDMs were washed and resuspended in FACS buffer. Data were acquired using a BD LSRFortessa (BD Biosciences) and analyzed with FlowJo10. Gating was performed on SSC/CD14+ cells (antibody panel 1) or SSC/CD91+ cells (antibody panel 2), respectively, and geometric mean fluorescence intensity (gMFI) was calculated.

### Phagocytosis Assay

*Assessment of granularity of HMDMs.* HMDMs were kept untreated or treated with CC, CC- C1q or CC-C1q-vWF complexes as described above. After 18 hr, HMDMs were harvested with PBS/10 mM EDTA and resuspended at a cell concentration of 5×10⁵ cells/100 µl in FACS buffer. HMDMs were stained with anti-CD11c-APC (Biolegend) for 30 min at 4°C in the dark. Data were acquired using a BD LSRFortessa (BD Biosciences) and analyzed with FlowJo10. For the quantification of phagocytosis the percentage of CD11c+ cells with high cell granularity, indicated by a shift into the side scatter (SSC) ^{high} gate (gate set according to shift in SSC from CD11c+ untreated to CC treated cells), was determined.

*Assessment of phagocytosedpHrodo-dyed CC complexes.* HMDMs were harvested with PBS/10mM EDTA and resuspended in phagocytosis buffer (DMEM+/12.5 mM HEPES (Sigma Aldrich)/5 mM MgCl₂) at a density of 5×10⁵ cells/100 µl. For pHrodo-dyed CC complexes, 1 mg/ml CC were suspended in 0.1M NaHCO₃ buffer (pH 8.3) and incubated with 10 µg/ml pHrodo Red Ester (Thermo Fisher Scientific) for 1 hr in the dark before the addition of C1q or C1q-vWF as described above. After a final wash, pHrodo-dyed CC complexes were added to HMDMs at a concentration of 0.5 mg/ml and incubated at 37°C for 30 min. Unphagocytosed CC complexes were washed away and HMDMs were stained with anti- CD11c-FITC (BioRad) for 30 min at 4°C in the dark and resuspended in FACS buffer. Data were acquired using a Beckman Coulter CytoFLEX (Beckman Coulter, Brea, CA, USA) and analyzed with FlowJo10. For the quantification of phagocytosis, the percentage of CD11c+ cells with a shift into the pHrodo Red Ester+ gate (gate set according to shift in pHrodo Red Ester from CD11c+ untreated to CC treated cells) was determined.

### Quantification of Secreted Cytokine Levels

HMDMs were stimulated with LPS and treated with CC, CC-C1q or CC-C1q-vWF complexes as described above. After 18 hr, supernatants were collected, centrifuged to remove cellular debris and CC and stored at -80°C until measurements. Analyses of cytokine secretion was carried out in duplicates with ELISA kits according to the manufacturer's instructions. IL-1β, IL-1α, IL-6 and IL-10 were measured using Biolegend ELISA kits, IL-18 and IL-1RA using Abcam ELISA kits and TNFα using a BD Bioscience ELISA kit.

### Caspase-1 Activity Assay

HMDMs were kept untreated or treated with CC, CC-C1q or CC-C1q-vWF complexes as described above. After 18 hr, HMDMs were harvested with PBS/10 mM EDTA and resuspended at a cell concentration of 5×10⁵ cells/ml. Cells were incubated for 1 hr with fluorochrome-labeled inhibitors of caspases (FLICA) probes for caspase-1 detection according to the manufacturer's instruction (FAM FLICA Caspase-1 Assay Kit, Immunochemistry Technology, Bloomington, MN, USA). HMDMs were stained with anti-CD11c-APC (Biolegend) for 30 min at 4°C in the dark. Data were acquired using a BD LSRFortessa (BD Biosciences) and analyzed with FlowJo10. Quantification of caspase-1 activity was determined by the percentage of CD11c+ cells in the FLICA+ gate.

### Statistical Analysis

Data are expressed as median ± interquartile range (IQR), if not stated otherwise. Wilcoxon matched pairs signed rank test was used to compare two groups of paired data. When more than 2 groups were compared, repeated measures (RM) ANOVA followed by a Tukey posttest was used as indicated. Data were analyzed with a statistical package program (GraphPad Prism 7, La Jolla, CA, USA). Ap-value <0.05 was considered statistically significant.

### Results

### vWF Binds to CC in a Clq-Dependent Manner

Whereas C1q is described as a classical opsonin for a variety of DAMPs (Benoit *et al*., 2012), this complement molecule has been also shown to adhere to oxidized LDL (Fraser and Tenner, 2010). In addition, Samstad et. al demonstrated C1q binding on CC after incubation with human plasma (Samstad *et al*., 2014). Therefore, we first analyzed, whether surface-bound C1q on CC secondarily enables the binding of vWF. Hence, we characterized the binding of vWF to C1q immobilized on CC by flow cytometry (FIG. 4A-FIG. 4C), confocal microscopy (FIG. 4D) and imaging flow cytometry (FIG. 4E). C1q deposition on the surface of CC is shown in FIG. 4A. The incubation of CC with vWF in the absence of C1q showed no vWF deposition on the CC surface (orange histogram in FIG. 4B, FIG. 4C). Only in the presence of surface-bound C1q, vWF is enabled to bind (green histogram in FIG. 4B, FIG. 4C). The gMFI for vWF binding in the presence of C1q was 67-fold higher compared to CC without C1q (mean gMFI ± SD of C1q+vWF: gMFI 134,000 ± 44,000 vs. vWF: gMFI 2,000 ± 500, *p*=0.0087). In a next step, we analyzed the localization of vWF binding to CC-C1q complex. Using confocal microscopy, C1q and vWF could be visualized on the surface of CC. C1q and vWF stainings co-localized (FIG. 4D). Finally, we used imaging flow cytometry to analyze a larger CC population. Again, we observed a similar merged staining pattern for C1q and vWF on the surface of CC (FIG. 4E).

Taken together, the results demonstrate that bound C1q mediates the binding of vWF to CC and vWF alone is not able to bind to the surface of CC.

### CC-C1q-vWF Complexes Upregulate the Surface Receptor Expression of HMDMs

Macrophages have high plasticity, enabling these cells to change their phenotype according to the environmental stimuli (Sica and Mantovani, 2012). In this context, C1q has been shown to elicit an upregulated expression of MerTK receptor, which is involved in the process of dead cell removal, termed efferocytosis (Galvan *et al*., 2012). Moreover, a polarization of macrophages towards an anti-inflammatory state has been described for C1q (Thanei and Trendelenburg, 2016). Therefore, we aimed to investigate the phenotype of HMDMs in our *in vitro* model. For this purpose, HMDMs were kept untreated or treated with CC, CC-C1q or CC-C1q-vWF complexes for 18 hr. To mimic the inflammatory milieu present in, *e.g.,* atherosclerotic plaques (Ross, 1999), HMDMs were simultaneously exposed to 100 ng/ml LPS for 18 hr. The phenotype was studied by analyzing the expression of surface CD14, CD86, LAIR1, LRP-1, MerTK, MHC II, PD-L1 and SR-A1 (Fig. 2A-H). HMDMs stimulated with CC-C1q-vWF complexes significantly upregulated the expression of CD14 (*p*=0.0312), LAIR1 (p=0.0312), LRP-1 (*p*=0.0312), MerTK (*p*=0.0312), PD-L1 (*p*=0.0312) and SR-A1 (*p*=0.0312) as compared to CC-C1q complexes. In four out of six donors, CD86 expression was upregulated, while MHC II expression was downregulated in five out of six donors. Neither the median receptor expression of CD86 nor of MHC II was significantly affected. Also, CC treatment did not induce any significant changes in surface receptor expression as compared to untreated HMDMs (data not shown).

Our results demonstrate that CC-C1q-vWF complexes uniquely affect the expression of surface receptors by an upregulation of efferocytosis receptor MerTK, scavenger receptors LAIR1, LRP-1 and SR-A1 as well as costimulatory receptors, namely CD14 and PD-L1.

### Phagocytosis of CC-C1q-vWF Complexes by HMDMs is Hampered

Since C1q is involved in the processes of efferocytosis (Taylor *et al*., 2000) as well as phagocytosis (Fraser *et al*., 2009) and as the additional presence of vWF upregulates efferocytosis and scavenger receptors (FIG. 5A-FIG. 5H), we next investigated the role of C1q-vWF binding in the uptake of CC complexes by HMDMs (FIG. 6A-FIG. 6D). Therefore, HMDMs were kept untreated or treated with CC, CC-C1q or CC-C1q-vWF complexes for 18 hr. The phagocytosis of CC leads to an increase in cell granularity, which can be determined by a shift in SSC using flow cytometry. Analyzed as control, untreated CD11c+ HMDMs do not express a SSC^{high} population. When HMDMs were treated with CC, CC-C1q or CC-C1-vWF complexes, the cells exhibit a SSC^{high} population (FIG. 6A). HMDMs show a significant decrease in cells positive for phagocytosis after the treatment with CC-C1q-vWF complexes compared to CC-C1q complexes (median phagocytosis (IQR) in six independent donors of CC-Clq-vWF: 13.65% (5.83-16.35%) vs. CC-Clq: 24.05% (22.55-34.60%), *p*=0.0312) (FIG. 6B). To exploit the effect on early phagocytosis, we incubated CC with the pH-dependent pHrodo Red dye (FIG. 6C and FIG. 6D). Analyzed as control, unstimulated CD11c+ HMDMs only exhibited a dim fluorescent signal for pHrodo Red. Consequently, fluorescent signal for pHrodo Red increased strongly when HMDMs were treated with pHrodo-dyed CC complexes for 30 min, due to the fusion of phagocytoses CC with the acidic lysosome. For the early phagocytosis, HMDMs had phagocytosed significantly less CC-C1q-vWF complexes than CC-C1q complexes (median phagocytosis (IQR) in six independent donors of CC-Clq-vWF: 54.55% (40.05-60.03%) vs. CC-Clq: 62,40% (49.05-68.78%), *p*=0.0312) (FIG. 6D).

In summary, late as well as early phagocytosis of CC-C1q-vWF complexes is reduced by HMDMs as compared to CC-C1q complexes.

### CC-C1q-vWF Complexes Reduce IL-1 Cytokine Secretion of HMDMs

CC have been repetitively described as capable inducers of IL-1β secretion in human monocytes and macrophages (Duewell *et al*., 2010). On the contrary, C1q has been shown to dampen the pro-inflammatory cytokine secretion for the same cell types (Thanei and Trendelenburg, 2016). Consequently, we next examined the effect of CC-C1q-vWF complexes on the cytokine profile of HMDMs. For this purpose, HMDMs kept untreated or treated with CC, CC-C1q or CC-C1q-vWF complexes were stimulated with 100 ng/ml LPS for 18 hr and supernatants were analyzed for the secretion of IL-1β, IL-1α, IL-1RA, IL-18, IL-6, IL-10 and TNFα cytokine levels (FIG. 7A-FIG. 7I). The CC treatment induced a strong IL-1β and IL-1α secretion by HMDMs and a moderate increase in IL-18 secretion as compared to untreated HMDMs. A robust decrease in pro-inflammatory cytokines for IL-1β and IL-1α was observed with CC-C1q complexes, and a decreasing trend for IL-6 and TNFα secretion. The additional presence of vWF on CC-C1q complexes resulted in a significantly enhanced the reduction of IL-1β secretion (*p*=0.0078), IL-1β/IL-1RA ratio (*p*=0.0078) and IL-1α/IL-1RA ratio (*p*=0.0234). All other cytokines did not significantly change by vWF bound to CC-C1q complexes. Differences in cytokine secretion of HMDMs according to the treatment added were not due to differences in cell death as assessed by quantification of early and late apoptosis or necrosis (data not shown).

Taken together, our data show that IL-1β cytokine secretion and IL-1β/IL-1RA and IL-1α/IL-1RA ratios by HMDMs after exposure to CC-C1q complexes are diminished further in the presence of vWF. This reduction appears to be IL-1 specific.

### CC-C1q-vWF Complexes Suppress Caspase-1 Activity of HMDMs

It is well known that IL-1 maturation, cleavage and secretion is regulated on a transcriptional as well as posttranscriptional level. While a priming signal through pattern recognition receptors is required for pro-IL-1β transcription, the maturation is dependent on the formation of the NLRP3 inflammasome and consecutive caspase-1 activation (Broz and Dixit, 2016). Therefore, we aimed to examine whether the observed change in IL-1 cytokine secretion was the resulting effect of a preceding NLRP3 inflammasome assembly. To address this point, HMDMs were kept untreated or treated with CC, CC-C1q or CC-C1q-vWF complexes for 18 hr and the effect on caspase-1 activation was quantified with FLICA probes. Upon CC treatment, HMDMs showed a marked increase in FLICA signal, demonstrating caspase-1 activity. While the presence of C1q on CC exhibited only a delicate reduction in caspase-1 activity the additional presence of vWF significantly suppressed caspase-1 activity in HMDMs (median FLICA+ cells (IQR) in six independent donors of CC-C1q: 11.54% (7.29-28.38%) vs. CC-Clq-vWF: 9.37% (5.92-22.73%), *p*=0.0312)) (FIG. 8B).

Overall, our data show that HMDMs treated with CC-C1q-vWF complexes exhibit decreased caspase-1 activity that impacts on NLRP3 inflammasome dependent IL-1β secretion.

### Discussion

The cross-talk between the complement and the hemostatic systems is extensive and can provide synergistic benefits for the human body (Rayes *et al*., 2014; Nissila *et al*., 2018). Yet, the role of many interplays is still unknown. In particular, even though an interplay between bound complement C1q and von Willebrand factor has been demonstrated previously (Kolm *et al*., 2016), it's impact on the immune system remained to be elucidated. In our study, we now illustrate that cholesterol crystals (CC) provide another physiological surface that allows a C1q-vWF interaction. Moreover, we found that the binding of vWF to C1q is capable of modulating the immune response of macrophages by an upregulated expression of phagocytosis-mediating receptors and costimulatory receptors, hampered phagocytosis and enhanced suppression of pro-inflammatory cytokine secretion.

Deposition of CC is described as a hallmark of atherosclerotic plaques. After recognition as DAMPs and ingestion by phagocytes, CC trigger ROS formation and lysosomal leakage with consecutive NLRP3 inflammasome assembly, caspase-1 generation and IL-1β secretion. (Rajamaki *et al*., 2010). IL-1β secretion leads to further recruitment of phagocytes by an amplification loop in a concerted action with other pro-inflammatory cytokines and chemokines (Libby, 2017). Phagocytes, in particular macrophages, also are responsible for the essential function of recycling LDL and cholesterol in the periphery, but can develop into lipid-laden macrophage-derived foam cells during the course of the disease when their recycling capacity is overwhelmed. In a first step, those foam cells can become apoptotic due to various stimuli, such as prolonged endoplasmic reticulum stress. In a second step, apoptotic cells that are insufficiently cleared (as occurring in advanced lesions due to defective efferocytosis), lead to cellular necrosis, in turn contributing to the formation of the necrotic core (Tabas, 2010). Consequently, enhanced ingestion of LDL and CC fuels foam cell development, which is described to be detrimental in later stages of atherosclerosis (Maguire *et al*., 2019). Hence, the conclusion that CC induce arterial inflammation and destabilization of atherosclerotic plaques (Janoudi *et al*., 2016) seems to be plausible. Complement C1q can be considered as a double-edged sword in the context of atherosclerosis. Previous studies showed that the clearance of oxidized LDL and modified LDL is enhanced by binding of C1q (Fraser and Tenner, 2010), but simultaneously leads to a polarization of macrophages towards an anti-inflammatory phenotype by a reduction in pro-inflammatory cytokine secretion (Ho *et al*., 2016). In addition, C1q induces mRNA transcription of cholesterol efflux transporters (Fraser and Tenner, 2010). In contrast to these atheroprotective traits, C1q was demonstrated to be present on CC from human plasma (Pilely *et al*., 2017) and found to be complexed to ApoE in human arteries (Yin *et al*., 2019), where it enables complement activation and thus is contributing to atheroprogression (Niculescu *et al*., 1985; Torzewski *et al*., 1998).

With regard to vWF, previous studies with vWF-deficient animals (van Galen) and on ADAMTS 13-deficient patients (Bettoni *et al*., 2017), which cannot cleave vWF multimers, suggest an athero- and thromboprogressive effect of vWF. Therefore, one could hypothesize disadvantageous consequences for the additional presence of vWF on CC-C1q complexes on macrophages. However, the interaction of vWF with complement factor H dampens the pro-inflammatory effect of complement *in vitro* (Rayes *et al*., 2014) and the impact of the subsequent binding of vWF to C1q on the immune response remained unexplored.

Previously, C1q and vWF have been regarded as separately acting molecules. Here, we identified not only a complex formation of C1q bound to the surface of CC with the subsequently binding vWF. The treatment of HMDMs with CC-C1q-vWF complexes results in an upregulated expression of surface receptors of efferocytosis (MerTK), scavenger receptors (LRP-1 and SR-A1) and costimulatory receptors (CD14, LAIR1 and PD-L1) compared to CC-C1q complexes alone. Studies investigating the role of the phagocytosis-mediating receptors MerTK and LRP-1 indicate atheroprotective features (Cai *et al*., 2017; Mueller *et al*., 2018) whereas the role of SR-A1 in cardiovascular disease is still controversial (reviewed by Ben and colleagues (Ben *et al*., 2015)). Additionally, LAIR1 was described to have beneficial effects on foam cell formation (Yi *et al*., 2018). Therefore, we next sought to determine the effect on the phagocytic capacity of HMDMs. Interestingly, the presence of vWF on CC-C1q complexes strongly diminished the late as well as early phagocytosis of CC by HMDMs, hereby reversing the effect of C1q alone. A possible explanation for this unexpected finding could be that the upregulated expression of phagocytosis-mediating receptors is representing a reinforcing feedback loop that is triggered in order to compensate for the decreased ingestion of CC-C1q-vWF complexes. Last, our data illustrate a significant decrease in IL-1 cytokine secretion by HMDMs when treated with CC-C1q-vWF complexes compared to CC-C1q complexes without vWF. The clinical significance of IL-1 in cardiovascular disease was demonstrated by the anti-IL-1beta antibody Canakinumab Anti-inflammatory Thrombosis Outcome Study (CANTOS) (Ridker *et al*., 2017). Thus, a reduction in phagocytosis and inflammation could retard plaque progression (Libby, 2002; Moore *et al*., 2013).

One limitation of our study is the *in vitro* character that is not fully reflecting the *in vivo* situation in humans that is likely to be more complex. Nevertheless, C1q's role in human atherosclerosis is supported by studies that have shown C1q expression in atherosclerotic carotid arteries of patients (Vlaicu *et al*., 1985; Cao *et al*., 2003; Peerschke *et al*., 2004) and hence underlining the relevance of our results. Second, *in vivo,* shear stress is necessary to unfold the full functional potential of vWF (Springer, 2014). In our study however, permanent shear stress was not applied, since the physiological occurrence of shear stress would rather reflect the situation during plaque rupture resulting from continuous blood flow but not the one inside the plaque itself.

Last, the mechanism by which the C1q-vWF interaction, in form of CC-C1q-vWF complexes, exerts its effect on HMDMs might be due to different modes of action. The first one could be the at least partial steric shielding of the C1q molecule by vWF, hereby weakening the effects of C1q (*e.g.,* FIG. 6A-FIG. 6D) Another mode of action could be an independent effect of vWF (*e.g.,* FIG. 5A-5H and FIG. 7A-FIG. 7I). Future studies will have to explore the potential modes of action responsible for the overall impact of CC-C1q-vWF complexes. In addition, since the mutual interactions between complement and hemostatic systems *in vivo* are likely to be more complex, our *in vitro* model will have to be developed further in order to approach a physiological setting. Recently, Gravastrand, *et al*., have described CC to induce complement-dependent activation of hemostasis (Gravastrand *et al*., 2019). Hence, the implementation of downstream complement components, such as C4 and C3, into our system and its effect on HMDMs in the additional presence of platelets warrants more work in the future.

In conclusion, with this study, we have provided new insights into an emerging cross-talk between complement C1q and hemostasis-initiating vWF. Our findings revealed that binding of vWF to C1q on CC regulates the immune response of human-monocyte derived macrophages. We showed that CC-C1q-vWF complexes provoke a hampered phagocytosis together with an accompanied reduction in IL-1 cytokine secretion by macrophages that could prove favorable for decelerating plaque progression.

### References

Abela, G.S. (2010). Cholesterol crystals piercing the arterial plaque and intima trigger local and systemic inflammation. J Clin Lipidol 4(3), 156-164. doi: 10.1016/j.jacl.2010.03.003.
Bakke, S.S., Aune, M.H., Niyonzima, N., Pilely, K., Ryan, L., Skjelland, M., et al. (2017). Cyclodextrin Reduces Cholesterol Crystal-Induced Inflammation by Modulating Complement Activation. J Immunol 199(8), 2910-2920. doi: 10.4049/jimmunol.1700302.
Ben, J., Zhu, X., Zhang, H., and Chen, Q. (2015). Class A1 scavenger receptors in cardiovascular diseases. Br JPharmacol 172(23), 5523-5530. doi: 10.1111/bph. 13105.
Benoit, M.E., Clarke, E.V., Morgado, P., Fraser, D.A., and Tenner, A.J. (2012). Complement protein C1q directs macrophage polarization and limits inflammasome activity during the uptake of apoptotic cells. JImmunol 188(11), 5682-5693. doi: 10.4049/ jimmunol.1103760.
Bettoni, S., Galbusera, M., Gastoldi, S., Donadelli, R., Tentori, C., Sparta, G., et al. (2017). Interaction between Multimeric von Willebrand Factor and Complement: A Fresh Look to the Pathophysiology of Microvascular Thrombosis. J Immunol 199(3), 1021¬1040. doi: 10.4049/jimmunol.1601121.
Bhatia, V.K., Yun, S., Leung, V., Grimsditch, D.C., Benson, G.M., Botto, M.B., et al. (2007). Complement C1q reduces early atherosclerosis in low-density lipoprotein receptor-deficient mice. Am J Pathol 170(1), 416-426. doi: 10.2353/ ajpath.2007.060406.
Bigler, C., Schaller, M., Perahud, I., Osthoff, M., and Trendelenburg, M. (2009). Autoantibodies against complement C1q specifically target C1q bound on early apoptotic cells. i 183(5), 3512-3521. doi: 10.4049/jimmunol.0803573.
Bobak, D.A., Gaither, T.A., Frank, M.M., and Tenner, A.J. (1987). Modulation of FcR function by complement: subcomponent C1q enhances the phagocytosis of IgG-opsonized targets by human monocytes and culture-derived macrophages. J Immunol 138(4), 1150-1156.
Bohlson, S.S., Fraser, D.A., and Tenner, A.J. (2007). Complement proteins C1q and MBL are pattern recognition molecules that signal immediate and long-term protective immune functions. Mol Immunol 44(1-3), 33-43. doi: 10.1016/ j.molimm.2006.06.021.
Broz, P., and Dixit, V.M. (2016). Inflammasomes: mechanism of assembly, regulation and signalling. Nat Rev Immunol 16(7), 407-420. doi: 10.1038/nri.2016.58.
Buono, C., Come, C.E., Witztum, J.L., Maguire, G.F., Connelly, P.W., Carroll, M., et al. (2002). Influence of C3 deficiency on atherosclerosis. Circulation 105(25), 3025¬3031.
Cai, B., Thorp, E.B., Doran, A.C., Sansbury, B.E., Daemen, M.J., Dorweiler, B., et al. (2017). MerTK receptor cleavage promotes plaque necrosis and defective resolution in atherosclerosis. J Clin Invest 127(2), 564-568. doi: 10.1172/jci90520.
Cao, W., Bobryshev, Y.V., Lord, R.S., Oakley, R.E., Lee, S.H., and Lu, J. (2003). Dendritic cells in the arterial wall express C1q: potential significance in atherogenesis. Cardiovasc Res 60(1), 175-186.
Corr, E.M., Cunningham, C.C., and Dunne, A. (2016). Cholesterol crystals activate Syk and PI3 kinase in human macrophages and dendritic cells. Atherosclerosis 251, 197-205. doi: 10.1016/j.atherosclerosis.2016.06.035.
D uewell, P., Kono, H., Rayner, K.J., Sirois, C.M., Vladimer, G., Bauernfeind, F.G., et al. (2010). NLRP3 inflammasomes are required for atherogenesis and activated by cholesterol crystals. Nature 464(7293), 1357-1361. doi: 10.1038/nature08938.
Feng, S., Liang, X., Cruz, M.A., Vu, H., Zhou, Z., Pemmaraju, N., et al. (2013). The interaction between factor H and Von Willebrand factor. PLoS One 8(8), e73715. doi: 10.1371/joumal.pone.0073715.
Feng, S., Liang, X., Kroll, M.H., Chung, D.W., and Afshar-Kharghan, V. (2015). von Willebrand factor is a cofactor in complement regulation. Blood 125(6), 1034-1037. doi: 10.1182/blood-2014-06-585430.
Franklin, B.S., Mangan, M.S., and Latz, E. (2016). Crystal Formation in Inflammation. Annu Rev Immunol 34, 173-202. doi: 10.1146/annurev-immunol-041015-055539.
Fraser, D.A., Bohlson, S.S., Jasinskiene, N., Rawal, N., Palmarini, G., Ruiz, S., et al. (2006). C1q and MBL, components of the innate immune system, influence monocyte cytokine expression. JLeukoc Biol 80(1), 107-116. doi: 10.1189/jlb.1105683.
Fraser, D.A., Laust, A.K., Nelson, E.L., and Tenner, A.J. (2009). C1q differentially modulates phagocytosis and cytokine responses during ingestion of apoptotic cells by human monocytes, macrophages, and dendritic cells. J Immunol 183(10), 6175-6185. doi: 10.4049/jimmunol.0902232.
Fraser, D.A., and Tenner, A.J. (2010). Innate immune proteins C1q and mannan-binding lectin enhance clearance of atherogenic lipoproteins by human monocytes and macrophages. J Immunol 185(7), 3932-3939. doi: 10.4049/jimmunol.1002080.
Galvan, M.D., Foreman, D.B., Zeng, E., Tan, J.C., and Bohlson, S.S. (2012). Complement component C1q regulates macrophage expression of Mer tyrosine kinase to promote clearance of apoptotic cells. J Immunol 188(8), 3716-3723. doi: 10.4049/jimmunol.1102920.
Gravastrand, C.S., Steinkjer, B., Halvorsen, B., Landsem, A., Skjelland, M., Jacobsen, E.A., et al. (2019). Cholesterol Crystals Induce Coagulation Activation through Complement-Dependent Expression of Monocytic Tissue Factor. J Immunol doi: 10.4049/jimmunol.1900503.
Ho, M.M., Manughian-Peter, A., Spivia, W.R., Taylor, A., and Fraser, D.A. (2016). Macrophage molecular signaling and inflammatory responses during ingestion of atherogenic lipoproteins are modulated by complement protein C1q. Atherosclerosis 253, 38-46. doi: 10.1016/j.atherosclerosis.2016.08.019.
Janoudi, A., Shamoun, F.E., Kalavakunta, J.K., and Abela, G.S. (2016). Cholesterol crystal induced arterial inflammation and destabilization of atherosclerotic plaque. Eur Heart J 37(25), 1959-1967. doi: 10.1093/eurheartj/ehv653.
Kolm, R., Schaller, M., Roumenina, L.T., Niemiec, I., Kremer Hovinga, J.A., Khanicheh, E., et al. (2016). Von Willebrand Factor Interacts with Surface-Bound C1q and Induces Platelet Rolling. J Immunol 197(9), 3669-3679. doi: 10.4049/jimmunol.1501876.
Lewis, M.J., Malik, T.H., Ehrenstein, M.R., Boyle, J.J., Botto, M., and Haskard, D.O. (2009). Immunoglobulin M is required for protection against atherosclerosis in low-density lipoprotein receptor-deficient mice. Circulation 120(5), 417-426. doi: 10.1161/circulationaha. 109.868158.
Libby, P. (2002). Inflammation in atherosclerosis. Nature 420(6917), 868-874. doi: 10.1038/nature01323.
Libby, P. (2017). Interleukin-1 Beta as a Target for Atherosclerosis Therapy: Biological Basis of CANTOS and Beyond. J Am Coll Cardiol 70(18), 2278-2289. doi: 10.1016/j.jacc.2017.09.028.
Maguire, E.M., Pearce, S.W.A., and Xiao, Q. (2019). Foam cell formation: A new target for fighting atherosclerosis and cardiovascular disease. Vascul Pharmacol 112, 54-71. doi: 10.1016/j.vph.2018.08.002.
Markiewski, M.M., Nilsson, B., Ekdahl, K.N., Mollnes, T.E., and Lambris, J.D. (2007). Complement and coagulation: strangers or partners in crime? Trends Immunol 28(4), 184-192. doi: 10.1016/j.it.2007.02.006.
Moore, K., Sheedy, F., and Fisher, E. (2013). Macrophages in atherosclerosis: a dynamic balance. Nat Rev Immunol 13(10), 709-721. doi: 10.1038/nri3520.
Morgan, B.P., and Harris, C.L. (2015). Complement, a target for therapy in inflammatory and degenerative diseases. Nat Rev Drug Discov 14(12), 857-877. doi: 10.1038/nrd4657.
Mueller, P.A., Zhu, L., Tavori, H., Huynh, K., Giunzioni, I., Stafford, J.M., et al. (2018). Deletion of Macrophage Low-Density Lipoprotein Receptor-Related Protein 1 (LRP1) Accelerates Atherosclerosis Regression and Increases C-C Chemokine Receptor Type 7 (CCR7) Expression in Plaque Macrophages. Circulation 138(17), 1850-1863. doi: 10.1161/ circulationaha. 117.031702.
Nayak, A., Ferluga, J., Tsolaki, A.G., and Kishore, U. (2010). The non-classical functions of the classical complement pathway recognition subcomponent C1q. Immunol Lett 131(2), 139-150. doi: 10.1016/j.imlet.2010.03.012.
Niculescu, F., Rus, H., Cristea, A., and Vlaicu, R. (1985). Localization of the terminal C5b-9 complement complex in the human aortic atherosclerotic wall. Immunol Lett 10(2), 109-114.
Nissila, E., Hakala, P., Leskinen, K., Roig, A., Syed, S., Van Kessel, K.P.M., et al. (2018). Complement Factor H and Apolipoprotein E Participate in Regulation of Inflammation in THP-1 Macrophages. Front Immunol 9, 2701. doi: 10.3389/fimmu.2018.02701.
Peerschke, E.I., Minta, J.O., Zhou, S.Z., Bini, A., Gotlieb, A., Colman, R.W., et al. (2004). Expression of gC1q-R/p33 and its major ligands in human atherosclerotic lesions. Mol Immunol 41(8), 759-766. doi: 10.1016/j.molimm.2004.04.020.
Pilely, K., Fumagalli, S., Rosbjerg, A., Genster, N., Skjoedt, M.-O., Perego, C., et al. (2017). C-Reactive Protein Binds to Cholesterol Crystals and Co-Localizes with the Terminal Complement Complex in Human Atherosclerotic Plaques. Frontiers in Immunology 8(1040). doi: 10.3389/fimmu.2017.01040.
Rajamaki, K., Lappalainen, J., Oorni, K., Valimaki, E., Matikainen, S., Kovanen, P.T., et al. (2010). Cholesterol crystals activate the NLRP3 inflammasome in human macrophages: a novel link between cholesterol metabolism and inflammation. PLoS One 5(7), e11765. doi: 10.1371/journal.pone.0011765.
Rayes, J., Roumenina, L.T., Dimitrov, J.D., Repesse, Y., Ing, M., Christophe, O., et al. (2014). The interaction between factor H and VWF increases factor H cofactor activity and regulates VWF prothrombotic status. Blood 123(1), 121-125. doi: 10.1182/blood-2013-04-495853.
Ridker, P.M., Everett, B.M., Thuren, T., MacFadyen, J.G., Chang, W.H., Ballantyne, C., et al. (2017). Antiinflammatory Therapy with Canakinumab for Atherosclerotic Disease. N Engl J Med 377(12), 1119-1131. doi: 10.1056/NEJMoa1707914.
Ross, R. (1999). Atherosclerosis--an inflammatory disease. N Engl J Med 340(2), 115-126. doi: 10.1056/nejm199901143400207.
Samstad, E.O., Niyonzima, N., Nymo, S., Aune, M.H., Ryan, L., Bakke, S.S., et al. (2014). Cholesterol crystals induce complement-dependent inflammasome activation and cytokine release. J Immunol 192(6), 2837-2845. doi: 10.4049/jimmunol.1302484.
Schmiedt, W., Kinscherf, R., Deigner, H.P., Kamencic, H., Nauen, O., Kilo, J., et al. (1998). Complement C6 deficiency protects against diet-induced atherosclerosis in rabbits. Arterioscler Thromb Vasc Biol 18(11), 1790-1795.
Sica, A., and Mantovani, A. (2012). Macrophage plasticity and polarization: in vivo veritas. J Clin Invest 122(3), 787-795. doi: 10.1172/jci59643.
Springer, T.A. (2014). von Willebrand factor, Jedi knight of the bloodstream. Blood 124(9), 1412-1425. doi: 10.1182/blood-2014-05-378638.
Tabas, I. (2010). Macrophage death and defective inflammation resolution in atherosclerosis. Nat Rev Immunol 10(1), 36-46. doi: 10.1038/nri2675.
Taylor, P.R., Carugati, A., Fadok, V.A., Cook, H.T., Andrews, M., Carroll, M.C., et al. (2000). A hierarchical role for classical pathway complement proteins in the clearance of apoptotic cells in vivo. J Exp Med 192(3), 359-366.
Thanei, S., and Trendelenburg, M. (2016). Anti-C1q Autoantibodies from Systemic Lupus Erythematosus Patients Induce a Proinflammatory Phenotype in Macrophages. J Immunol 196(5), 2063-2074. doi: 10.4049/jimmunol. 1501659.
Torzewski, M., Klouche, M., Hock, J., Messner, M., Dorweiler, B., Torzewski, J., et al. (1998). Immunohistochemical demonstration of enzymatically modified human LDL and its colocalization with the terminal complement complex in the early atherosclerotic lesion. Arterioscler Thromb Vasc Biol 18(3), 369-378.
Turner, N.A., and Moake, J. (2013). Assembly and activation of alternative complement components on endothelial cell-anchored ultra-large von Willebrand factor links complement and hemostasis-thrombosis. PLoS One 8(3), e59372. doi: 10. 1371/j ournal.pone. 0059372.
van Galen, K.P., Tuinenburg, A., Smeets, E.M., and Schutgens, R.E. (2012). Von Willebrand factor deficiency and atherosclerosis. Blood Rev 26(5), 189-196. doi: 10.1016/j.blre.2012.05.002.
van Schooten, C.J., Shahbazi, S., Groot, E., Oortwijn, B.D., van den Berg, H.M., Denis, C.V., et al. (2008). Macrophages contribute to the cellular uptake of von Willebrand factor and factor VIII in vivo. Blood 112(5), 1704-1712. doi: 10.1182/blood-2008-01-133181.
Vlaicu, R., Rus, H.G., Niculescu, F., and Cristea, A. (1985). Immunoglobulins and complement components in human aortic atherosclerotic intima. Atherosclerosis 55(1), 35-50.
Walport, M.J. (2001). Complement. First of two parts. N Engl J Med 344(14), 1058-1066. doi: 10.1056/nejm200104053441406.
Wohner, N., Muczynski, V., Mohamadi, A., Legendre, P., Proulle, V., Ayme, G., et al. (2018). Macrophage scavenger receptor SR-AI contributes to the clearance of von Willebrand factor. Haematologica 103(4), 728-737. doi: 10.3324/haematol.2017.175216.
Yi, X., Zhang, J., Zhuang, R., Wang, S., Cheng, S., Zhang, D., et al. (2018). Silencing LAIR-1 in human THP-1 macrophage increases foam cell formation by modulating PPARgamma and M2 polarization. Cytokine 111, 194-205. doi: 10.1016/j.cyto.2018.08.028.
Yin, C., Ackermann, S., Ma, Z., Mohanta, S.K., Zhang, C., Li, Y., et al. (2019). ApoE attenuates unresolvable inflammation by complex formation with activated C1q. Nat Med 25(3), 496-506. doi: 10.1038/s41591-018-0336-8.
Zimmer, S., Grebe, A., Bakke, S.S., Bode, N., Halvorsen, B., Ulas, T., et al. (2016). Cyclodextrin promotes atherosclerosis regression via macrophage reprogramming. Sci Transl Med 8(333), 333ra350. doi: 10.1126/scitranslmed.aad6100.

The examples set forth above are provided to give those of ordinary skill in the art a complete disclosure and description of how to make and use the embodiments of the compositions, systems and methods of the invention, and are not intended to limit the scope of what the inventors regard as their invention. Modifications of the above-described modes for carrying out the invention that are obvious to persons of skill in the art are intended to be within the scope of the following claims. All patents and publications mentioned in the specification are indicative of the levels of skill of those skilled in the art to which the invention pertains.

## Claims

1. A composition comprising Von Willebrand Factor (VWF) for use in a method of treating atherosclerosis in a subject comprising administering to a subject a therapeutically effective amount of said composition such that VWF binds complement C1q to form C1q-VWF complexes and the C1q-VWF complex binds cholesterol crystals (CC), thereby forming a CC-C1q-VWF complex and inhibiting atherosclerotic plaque progression,
wherein the VWF is recombinant VWF (rVWF).

2. The composition for the use of claim 1 wherein inflammation of atherosclerotic plaque in a subject is decreased comprising administering to a subject a therapeutically effective amount of said composition such that said rVWF binds complement C1q to form C1q-VWF complexes, and the C1q-VWF complex binds cholesterol crystals (CC), thereby forming a CC-C1q-VWF complex and decreasing inflammation of atherosclerotic plaque.

3. The composition for the use of claim 1 wherein an immune response of macrophages in a blood vessel of a subject is modulated comprising administering to a subject a therapeutically effective amount of said composition such that said rVWF binds complement C1q to form C1q-VWF complexes, thereby reducing macrophage cell formation, increasing the expression level of one or more phagocytosis-mediating receptors and/or costimulatory receptors on the surface of the macrophages, decreasing phagocytosis by the macrophages, and/or decreasing pro-inflammatory cytokine secretion by the macrophages.

4. The composition for the use of any one of the preceding claims, wherein the rVWF comprises a polypeptide selected from the group consisting of:
(a) the amino acid sequence of SEQ ID NO:3;
(b) a polypeptide encoded by the polynucleotide sequence of SEQ ID NO: 1.

5. The composition for the use of any one of the preceding claims,
wherein the composition of rVWF comprises a high molecular weight VWF multimer comprising at least 20% VWF decamers or high order multimers, preferably at least 40% VWF decamer or high order multimers, and more preferably at least 60% VWF decamers or high order multimers,
wherein the rVWF is matured in vitro by treatment with Furin, and/or
wherein the rVWF has a higher specific activity compared to plasma-derived VWF.

6. The composition for the use of claim 3, wherein the C1q-VWF complex binds cholesterol crystals (CC), thereby forming a CC-C1q-VWF complex.

7. The composition for the use of any one of claims 3-6, wherein the one or more phagocytosis-mediating receptors and/or costimulatory receptors are selected from the group consisting of CD14, CD86, leukocyte-associated immunoglobulin-like receptor 1 (LAIR1), lipoprotein receptor-related protein 1 (LRP-1), tyrosine protein kinase Mer (MerTk), MHC II, programmed death ligand 1 (PD-L1), scavenger receptor A 1 (SR-A1), and a combination thereof, and/or wherein the pro-inflammatory cytokine is selected from the group consisting of IL-1α, IL-1β, IL-6, IL-10, IL-18, IL-1R, TNF1α, and a combination thereof.

8. The composition for the use of any one of the preceding claims, further comprising determining the level of C1q-VWF complex in a sample taken from the subject after administrating the compositions comprising VWF, preferably
wherein determining the level of C1q-VWF complex comprises detecting the C1q-VWF complex on the surface of a cell in the sample.

9. An *in vitro* method of identifying a candidate agent for modulating phagocytosis of macrophages comprising:
(a) incubating rVWF, complement C1q, and cholesterol crystals to form a complex comprising rVWF, complement C1q, and cholesterol crystals (CC-C1q-VWF complex);
(b) treating a population of macrophages with the CC-C1q-VWF complex;
(c) treating the population of macrophages with a candidate agent;
(d) measuring the level of phagocytosis in the population of macrophages; and
(e) comparing the level of phagocytosis to the level of phagocytosis of a population of macrophages that have not been treated with the candidate agent, preferably
wherein the measuring of the level of phagocytosis comprises detecting the percentage of CD11c-positive cells in the population of macrophages.

10. An *in vitro* method of identifying a candidate agent for modulating expression of phagocytosis-mediating receptors and costimulatory receptors by macrophages comprising:
(a) incubating rVWF, complement C1q, and cholesterol crystals to form a complex comprising rVWF, complement C1q, and cholesterol crystals (CC-C1q-VWF complex);
(b) treating a population of macrophages with the CC-C1q-VWF complex;
(c) treating the population of macrophages with a candidate agent;
(d) measuring the level of expression of phagocytosis-mediating receptors and costimulatory receptors in the population of macrophages; and
(e) comparing the level of expression to the level of expression of phagocytosis-mediating receptors and costimulatory receptors in a population of macrophages that have not been treated with the candidate agent, preferably
wherein the phagocytosis-mediating receptors and costimulatory receptors are selected from the group consisting of CD14, CD86, leukocyte-associated immunoglobulin-like receptor 1 (LAIR1), lipoprotein receptor-related protein 1 (LRP-1), tyrosine protein kinase Mer (MerTk), MHC II, programmed death ligand 1 (PD-L1), scavenger receptor A 1 (SR-A1), and a combination thereof.

11. An *in vitro* method of identifying a candidate agent for modulating pro-inflammatory cytokine secretion by macrophages comprising:
(a) incubating rVWF, complement C1q, and cholesterol crystals to form a complex comprising rVWF, complement C1q, and cholesterol crystals (CC-C1q-VWF complex);
(b) treating a population of macrophages with the CC-C1q-VWF complex;
(c) treating the population of macrophages with a candidate agent;
(d) measuring the level of pro-inflammatory cytokine secretion in the population of macrophages; and
(e) comparing the level of pro-inflammatory cytokine secretion to the level of pro-inflammatory cytokine secretion in a population of macrophages that have not been treated with the candidate agent, preferably
wherein the pro-inflammatory cytokine is selected from the group consisting of IL-1α, IL-1β, IL-6, IL-10, IL-18, IL-1R, TNF1α, and a combination thereof.

12. The *in vitro* method of any one of claims 9-11, wherein the rVWF comprises a polypeptide selected from the group consisting of:
(a) the amino acid sequence of SEQ ID NO:3;
(b) a biologically active analog, fragment, or variant of (a);
(c) a polypeptide encoded by the polynucleotide sequence of SEQ ID NO:1; and
(d) a biologically active analog, fragment, or variant of (c), and/or
wherein the rVWF comprises a high molecular weight VWF vWF multimer comprising at least 20% VWF decamers or high order multimers, preferably
wherein the high molecular weight VWF vWF multimer comprises at least 40% VWF decamers or high order multimers.

13. The *in vitro* method of claim 12, wherein the high molecular weight VWF multimer comprises at least 60% VWF decamers or high order multimers.

14. The *in vitro* method of any one of claims 9 - 13, wherein the rVWF is matured in vitro by treatment with Furin, and/or
wherein the rVWF has a higher specific activity compared to plasma-derived VWF.

15. The *in vitro* method of any one of claims 9 - 14, wherein the macrophages are human monocyte-derived macrophages.

## Patentansprüche

1. Zusammensetzung, umfassend Von-Willebrand-Faktor (VWF) zur Verwendung in einem Verfahren zum Behandeln von Atherosklerose bei einem Subjekt, umfassend Verabreichen einer therapeutisch wirksamen Menge dieser Zusammensetzung an ein Subjekt, sodass VWF Komplement C1q bindet, um Clq-VWF-Komplexe zu bilden, und der C1q-VWF-Komplex Cholesterinkristalle (CC) bindet, wodurch ein CC-C1q-VWF-Komplex gebildet wird und die atherosklerotische Plaque-Progression inhibiert wird,
wobei der VWF rekombinanter VWF (rVWF) ist.

2. Zusammensetzung zur Verwendung nach Anspruch 1, wobei Entzündungen von atherosklerotischen Plaques bei einem Subjekt verringert werden, umfassend Verabreichen einer therapeutisch wirksamen Menge der Zusammensetzung an ein Subjekt, sodass der rVWF Komplement C1q bindet, um Clq-VWF-Komplexe zu bilden, und der C1q-VWF-Komplex Cholesterinkristalle (CC) bindet, wodurch ein CC-C1q-VWF-Komplex gebildet wird und Entzündungen von atherosklerotischen Plaques zurückgehen.

3. Zusammensetzung zur Verwendung nach Anspruch 1, wobei eine Immunantwort von Makrophagen in einem Blutgefäß eines Subjekts moduliert wird, umfassend Verabreichen einer therapeutisch wirksamen Menge der Zusammensetzung an ein Subjekt, sodass der rVWF Komplement C1q bindet, um Clq-VWF-Komplexe zu bilden, wodurch die Makrophagenzellbildung verringert wird, das Expressionsniveau eines oder mehrerer phagocytosevermittelnder Rezeptoren und/oder kostimulatorischer Rezeptoren auf der Oberfläche der Makrophagen erhöht wird, Phagocytose durch die Makrophagen verringert wird und/oder pro-inflammatorische Zytokinsekretion durch die Makrophagen verringert wird.

4. Zusammensetzung zur Verwendung nach einem der vorhergehenden Ansprüche, wobei der rVWF ein Polypeptid umfasst, das ausgewählt ist aus der Gruppe bestehend aus:
(a) der Aminosäuresequenz von SEQ ID NO:3;
(b) einem Polypeptid, das von der Polynukleotidsequenz von SEQ ID NO: 1 codiert wird.

5. Zusammensetzung zur Verwendung nach einem der vorhergehenden Ansprüche,
wobei die Zusammensetzung von rVWF ein hochmolekulares VWF-Multimer umfasst, das mindestens 20 % VWF-Decamere oder Multimere höherer Ordnung, vorzugsweise mindestens 40 % VWF-Decamere oder Multimere höherer Ordnung und mehr bevorzugt mindestens 60 % VWF-Decamere oder Multimere höherer Ordnung umfasst,
wobei der rVWF in vitro durch Behandlung mit Furin gereift ist, und/oder
wobei der rVWF eine höhere spezifische Aktivität im Vergleich zu aus Plasma stammendem VWF aufweist.

6. Zusammensetzung zur Verwendung nach Anspruch 3, wobei der C1q-VWF-Komplex Cholesterinkristalle (CC) bindet, wodurch ein CC-C1q-VWF-Komplex gebildet wird.

7. Zusammensetzung zur Verwendung nach einem der Ansprüche 3-6, wobei der eine oder die mehreren phagocytosevermittelnden Rezeptoren und/oder kostimulatorischen Rezeptoren aus der Gruppe bestehend aus CD14, CD86, leukozytenassoziiertem immunoglobulinähnlichem Rezeptor 1 (LAIR1), Lipoprotein Receptor-related Protein 1 (LRP-1), Tyrosinproteinkinase Mer (MerTk), MHC II, Programmed Death Ligand 1 (PD-L1), Scavenger-Rezeptor A 1 (SR-A1) und einer Kombination davon ausgewählt sind, und/oder wobei das proinflammatorische Zytokin aus der Gruppe bestehend aus IL-1α, IL-1β, IL-6, IL-10, IL-18, IL-1R, TNF1α und einer Kombination davon ausgewählt ist.

8. Zusammensetzung zur Verwendung nach einem der vorhergehenden Ansprüche, ferner umfassend Bestimmen des Spiegels von C1q-VWF-Komplex in einer Probe, die von dem Subjekt nach Verabreichen der Zusammensetzungen, die VWF enthalten, entnommen wurde,
wobei Bestimmen des Spiegels von C1q-VWF-Komplex vorzugsweise Erfassen des C1q-VWF-Komplexes auf der Oberfläche einer Zelle in der Probe umfasst.

9. In-vitro-Verfahren zum Identifizieren eines Kandidatenwirkstoffs zum Modulieren der Phagozytose von Makrophagen, umfassend:
(a) Inkubieren von rVWF, Komplement C1q und Cholesterinkristallen zum Bilden eines Komplexes, der rVWF, Komplement C1q und Cholesterinkristalle (CC-C1q-VWF-Komplex) umfasst;
(b) Behandeln einer Population von Makrophagen mit dem CC-C1q-VWF-Komplex;
(c) Behandeln der Population von Makrophagen mit einem Kandidatenwirkstoff;
(d) Messen des Phagozytosestadiums in der Population von Makrophagen; und
(e) Vergleichen des Phagozytosestadiums mit dem Phagozytosestadium einer Population von Makrophagen, die nicht mit dem Kandidatenwirkstoff behandelt wurden,
wobei das Messen des Phagozytosestadiums vorzugsweise Erfassen des prozentualen Anteils an CD11c-positiven Zellen in der Population von Makrophagen umfasst.

10. In-vitro-Verfahren zum Identifizieren eines Kandidatenwirkstoffs zum Modulieren der Expression von phagozytosevermittelnden Rezeptoren und kostimulatorischen Rezeptoren durch Makrophagen, umfassend:
(a) Inkubieren von rVWF, Komplement C1q und Cholesterinkristallen zum Bilden eines Komplexes, der rVWF, Komplement C1q und Cholesterinkristalle (CC-C1q-VWF-Komplex) umfasst;
(b) Behandeln einer Population von Makrophagen mit dem CC-C1q-VWF-Komplex;
(c) Behandeln der Population von Makrophagen mit einem Kandidatenwirkstoff;
(d) Messen des Expressionsniveaus von phagozytosevermittelnden Rezeptoren und kostimulatorischen Rezeptoren in der Population von Makrophagen; und
(e) Vergleichen des Expressionsniveaus mit dem Expressionsniveau von phagozytosevermittelnden Rezeptoren und kostimulatorischen Rezeptoren in einer Population von Makrophagen, die nicht mit dem Kandidatenwirkstoff behandelt wurden,
wobei die phagozytosevermittelnden Rezeptoren und kostimulatorischen Rezeptoren vorzugsweise aus der Gruppe bestehend aus CD14, CD86, leukozytenassoziiertem immunoglobulinähnlichem Rezeptor 1 (LAIR1), Lipoprotein Receptor-related Protein 1 (LRP-1), Tyrosinproteinkinase Mer (MerTk), MHC II, Programmed Death Ligand 1 (PD-L1), Scavenger-Rezeptor A 1 (SR-A1) und einer Kombination davon ausgewählt sind.

11. In-vitro-Verfahren zum Identifizieren eines Kandidatenwirkstoffs zum Modulieren der Sekretion proinflammatorischer Zytokine durch Makrophagen, umfassend:
(a) Inkubieren von rVWF, Komplement C1q und Cholesterinkristallen zum Bilden eines Komplexes, der rVWF, Komplement C1q und Cholesterinkristalle (CC-C1q-VWF-Komplex) umfasst;
(b) Behandeln einer Population von Makrophagen mit dem CC-C1q-VWF-Komplex;
(c) Behandeln der Population von Makrophagen mit einem Kandidatenwirkstoff;
(d) Messen des Niveaus der Sekretion proinflammatorischer Zytokine in der Population von Makrophagen; und
(e) Vergleichen des Niveaus der Sekretion proinflammatorischer Zytokine mit dem Niveau der Sekretion proinflammatorischer Zytokine einer Population von Makrophagen, die nicht mit dem Kandidatenwirkstoff behandelt wurden,
wobei das proinflammatorische Zytokin vorzugsweise aus der Gruppe bestehend aus IL-1α, IL-1β, IL-6, IL-10, IL-18, IL-1R, TNF1α und einer Kombination davon ausgewählt ist.

12. In-vitro-Verfahren nach einem der Ansprüche 9-11, wobei der rVWF ein Polypeptid umfasst, das ausgewählt ist aus der Gruppe bestehend aus:
(a) der Aminosäuresequenz von SEQ ID NO:3;
(b) einem biologisch aktiven Analogon, Fragment oder einer biologisch aktiven Variante von (a);
(c) einem Polypeptid, das von der Polynukleotidsequenz von SEQ ID NO: 1 codiert wird; und
(d) einem biologisch aktiven Analogon, Fragment oder einer biologisch Variante von (c) und/oder
wobei der rVWF ein hochmolekulares VWF-Multimer umfasst, das mindestens 20 % VWF-Decamere oder Multimere höherer Ordnung umfasst,
wobei das hochmolekulare VWF-Multimere vorzugsweise mindestens 40 % VWF-Decamere oder Multimere höherer Ordnung umfasst.

13. In-vitro-Verfahren nach Anspruch 12, wobei das hochmolekulare VWF-Multimer mindestens 60 % VWF-Decamere oder Multimere höherer Ordnung umfasst.

14. In-vitro-Verfahren nach einem der Ansprüche 9-13, wobei der rVWF in-vitro durch Behandlung mit Furin gereift ist, und/oder
wobei der rVWF eine höhere spezifische Aktivität im Vergleich zu aus Plasma stammendem VWF aufweist.

15. *In-vitro*-Verfahren nach einem der Ansprüche 9-14, wobei die Makrophagen menschliche, von Monozyten stammende Makrophagen sind.

## Revendications

1. Composition comprenant le facteur de Von Willebrand (VWF) destinée à être utilisée dans un procédé de traitement de l'athérosclérose chez un sujet, comprenant l'administration à un sujet d'une quantité thérapeutiquement efficace de ladite composition de sorte que le VWF se lie au complément C1q pour former des complexes C1q-VWF et le complexe C1q-VWF se lie aux cristaux de cholestérol (CC), formant ainsi un complexe CC-C1q-VWF et inhibant la progression de plaques athérosclérotiques,
dans laquelle le VWF est du VWF recombinant (rVWF).

2. Composition destinée à être utilisée selon la revendication 1 dans laquelle l'inflammation de plaques athérosclérotiques chez un sujet est diminuée, comprenant l'administration à un sujet d'une quantité thérapeutiquement efficace de ladite composition de sorte que ledit rVWF se lie au complément C1q pour former des complexes C1q-VWF, et le complexe C1q-VWF se lie aux cristaux de cholestérol (CC), formant ainsi un complexe CC-C1q-VWF et diminuant l'inflammation de plaques athérosclérotiques.

3. Composition destinée à être utilisée selon la revendication 1, dans laquelle une réponse immunitaire des macrophages dans un vaisseau sanguin d'un sujet est modulée, comprenant l'administration à un sujet d'une quantité thérapeutiquement efficace de ladite composition de sorte que ledit rVWF se lie au complément C1q pour former des complexes C1q-VWF, réduisant ainsi la formation de cellules macrophages, augmentant le taux d'expression d'un ou de plusieurs récepteurs médiateurs de la phagocytose et/ou récepteurs costimulateurs sur la surface des macrophages, diminuant la phagocytose par les macrophages, et/ou diminuant la sécrétion de cytokines pro-inflammatoires par les macrophages.

4. Composition destinée à être utilisée selon l'une quelconque des revendications précédentes, dans laquelle le rVWF comprend un polypeptide choisi dans le groupe constitué par :
(a) les séquences d'acides aminés de SEQ ID NO:3 ;
(b) un polypeptide codé par la séquence polynucléotidique de SEQ ID NO:1.

5. Composition destinée à être utilisée selon l'une quelconque des revendications précédentes,
dans laquelle la composition de rVWF comprend un multimère de VWF de haut poids moléculaire comprenant au moins 20 % de décamères de VWF ou de multimères d'ordre élevé, de préférence au moins 40 % de décamères de VWF ou de multimères d'ordre élevé, et de manière davantage préférée au moins 60 % de décamères de VWF ou de multimères d'ordre élevé,
dans laquelle le rVWF est mûri in vitro par traitement à la furine, et/ou
dans laquelle le rVWF présente une activité spécifique supérieure à celle du VWF dérivé du plasma.

6. Composition destinée à être utilisée selon la revendication 3, dans laquelle le complexe C1q-VWF se lie aux cristaux de cholestérol (CC), formant ainsi un complexe CC-C1q-VWF.

7. Composition destinée à être utilisée selon l'une quelconque des revendications 3 à 6, dans laquelle le ou les récepteurs médiateurs de phagocytose et/ou récepteurs costimulateurs sont choisis dans le groupe constitué par le CD14, le CD86, le récepteur 1 de type immunoglobuline associé aux leucocytes (LAIR1), la protéine 1 associée au récepteur des lipoprotéines (LRP-1), la protéine de type récepteur à activité tyrosine kinase Mer (MerTk), le CMH II, le ligand de mort programmé 1 (PD-L1), le récepteur éboueur A 1 (SR-A1), et une combinaison de ceux-ci, et/ou dans laquelle la cytokine pro-inflammatoire est choisie parmi le groupe est constitué par IL-1α, IL-1β, IL-6, IL-10, IL-18, IL-1R, TNF1α, et une combinaison de ceux-ci.

8. Composition destinée à être utilisée selon l'une quelconque des revendications précédentes, comprenant en outre la détermination du taux de complexe C1q-VWF dans un échantillon prélevé chez le sujet après administration des compositions comprenant du VWF, de préférence
dans laquelle la détermination du taux de complexe C1q-VWF comprend la détection du complexe C1q-VWF sur la surface d'une cellule dans l'échantillon.

9. Procédé *in vitro* d'identification d'un agent candidat de modulation de la phagocytose des macrophages comprenant :
(a) l'incubation du rVWF, du complément C1q et des cristaux de cholestérol pour former un complexe comprenant le rVWF, le complément C1q et des cristaux de cholestérol (complexe CC-C1q-VWF) ;
(b) le traitement d'une population de macrophages avec le complexe CC-C1q-VWF ;
(c) le traitement de la population de macrophages avec un agent candidat ;
(d) la mesure du taux de phagocytose dans la population de macrophages ; et
(e) la comparaison du taux de phagocytose par rapport au taux de phagocytose d'une population de macrophages qui n'ont pas été traités avec l'agent candidat, de préférence
dans lequel la mesure du taux de phagocytose comprend la détection du pourcentage de cellules CD11c positives dans la population de macrophages.

10. Procédé *in vitro* d'identification d'un agent candidat pour moduler l'expression des récepteurs médiateurs de la phagocytose et des récepteurs costimulateurs par les macrophages comprenant :
(a) l'incubation du rVWF, du complément C1q et des cristaux de cholestérol pour former un complexe comprenant le rVWF, le complément C1q et des cristaux de cholestérol (complexe CC-C1q-VWF) ;
(b) le traitement d'une population de macrophages avec le complexe CC-C1q-VWF ;
(c) le traitement de la population de macrophages avec un agent candidat ;
(d) la mesure du taux d'expression des récepteurs médiateurs de la phagocytose et des récepteurs costimulateurs dans la population de macrophages ; et
(e) la comparaison du taux d'expression par rapport au taux d'expression des récepteurs médiateurs de la phagocytose et des récepteurs costimulateurs dans une population de macrophages qui n'ont pas été traités avec l'agent candidat, de préférence
dans lequel les récepteurs médiateurs de phagocytose et les récepteurs costimulateurs sont choisis dans le groupe constitué par le CD14, le CD86, le récepteur 1 de type immunoglobuline associé aux leucocytes (LAIR1), la protéine 1 associée au récepteur des lipoprotéines (LRP-1), la protéine à type de récepteur à activité tyrosine kinase Mer (MerTk), le CMH II, le ligand de mort programmé 1 (PD-L1), le récepteur éboueur A 1 (SR-A1), et une combinaison de ceux-ci.

11. Procédé *in vitro* d'identification d'un agent candidat pour moduler la sécrétion de cytokines pro-inflammatoires par les macrophages comprenant :
(a) l'incubation du rVWF, du complément C1q et des cristaux de cholestérol pour former un complexe comprenant le rVWF, le complément C1q et des cristaux de cholestérol (complexe CC-C1q-VWF) ;
(b) le traitement d'une population de macrophages avec le complexe CC-C1q-VWF ;
(c) le traitement de la population de macrophages avec un agent candidat ;
(d) la mesure du taux de sécrétion de cytokines pro-inflammatoires dans la population de macrophages ; et
(e) la comparaison du taux de sécrétion de cytokines pro-inflammatoires par rapport au taux de sécrétion de cytokines pro-inflammatoires dans une population de macrophages qui n'ont pas été traités avec l'agent candidat, de préférence
dans lequel la cytokine pro-inflammatoire est choisie dans le groupe constitué par IL-1α, IL-1β, IL-6, IL-10, IL-18, IL-1R, TNF1α, et une combinaison de ceux-ci.

12. Procédé *in vitro* selon l'une quelconque des revendications 9 à 11, dans lequel le rVWF comprend un polypeptide choisi parmi le groupe constitué par :
(a) les séquences d'acides aminés de SEQ ID NO:3 ;
(b) un analogue, fragment ou variant biologiquement actif de (a) ;
(c) un polypeptide codé par la séquence polynucléotidique de SEQ ID NO:1 ; et
(d) un analogue, fragment ou variant biologiquement actif de (c), et/ou
dans lequel le rVWF comprend un multimère de VWF de haut poids moléculaire comprenant au moins 20 % de décamères de VWF ou de multimères d'ordre élevé, de préférence
dans lequel le multimère de VWF de haut poids moléculaire comprend au moins 40 % de décamères de VWF ou de multimères d'ordre élevé.

13. Procédé *in vitro* selon la revendication 12, dans lequel le multimère de VWF de haut poids moléculaire comprend au moins 60 % de décamères de VWF ou de multimères d'ordre élevé.

14. Procédé *in vitro* selon l'une quelconque des revendications 9 à 13, dans lequel le rVWF est mûri in vitro par traitement à la furine, et/ou
dans lequel le rVWF présente une activité spécifique supérieure à celle du VWF dérivé du plasma.

15. Procédé *in vitro* selon l'une quelconque des revendications 9 à 14, dans lequel les macrophages sont des macrophages humains dérivés des monocytes.
